# EUROPEAN PATENT SPECIFICATION

(11) **EP 1 715 856 B1**
(45) Date of publication and mention of the grant of the patent: **11.07.2012**
(21) Application number: 04815693.9
(22) Date of filing: 23.12.2004
(51) Int. Cl.: A61K 31/135, A61K 9/00, A61K 9/10, A61K 9/16, A61K 9/28, A61K 9/48

(54) **ATOMOXETINE FORMULATIONS**
ATOMOXETIN-FORMULIERUNGEN
PREPARATIONS D'ATOMOXETINE

(30) Priority: 31.12.2003 US 533517 P
(43) Date of publication of application: 02.11.2006
(73) Proprietor: Actavis Group PTC ehf., 220 Hafnarfirdi (IS)
(72) Inventor: BOEHM, Garth, Westfield, NJ 07090 (US); DUNDON, Josephine, Fanwood, NJ 07023 (US)
(74) Representative: Lawrence, Malcolm Graham
(86) International application number: PCT/US2004/043678
(87) International publication number: WO 2005/065673

(56) References cited:
- WO-A-03/013492
- CABALLERO J ET AL: "ATOMOXETINE HYDROCHLORIDE FOR THE TREATMENT OF ATTENTION-DEFICIT/HYPERACTIVITY DISORDER" CLINICAL THERAPEUTICS, EXCERPTA MEDICA, PRINCETON, NJ, US, vol. 25, no. 12, December 2003 (2003-12), pages 3065-3083, XP008035477 ISSN: 0149-2918
- MATTIUZ EDWARD L ET AL: "Disposition and metabolic fate of atomoxetine hydrochloride: Pharmacokinetics, metabolism, and excretion in the Fischer 344 rat and beagle dog." DRUG METABOLISM AND DISPOSITION, vol. 31, no. 1, January 2003 (2003-01), pages 88-97, XP002325691 ISSN: 0090-9556

## Description

### BACKGROUND

Atomoxetine and its salts, particularly atomoxetine hydrochloride, have been employed as a pharmaceutically active agents in the treatment of attention deficit disorder (ADD) also known as attention deficit hyperactivity disorder (ADHD).

Atomoxetine is the R(-) isomer of tomoxetine; the chemical name for atomoxetine is (-)-*N*-methyl-3-phenyl-3-(*o*-tolyloxy)-propyl amine. Atomoxetine acts as selective norepinephrine inhibitor, and is believed to exert its clinical effects primarily via selective inhibition of the presynaptic norepinephrine transporter.

Atomoxetine is currently formulated as atomoxetine hydrochloride capsules containing 5 mg, 10 mg, 18 mg, 25 mg, 40 mg, and 60 mg of atomoxetine. Atomoxetine is administered as a single daily dose or as evenly divided twice daily doses for morning and afternoon/early evening administration. Currently marketed formulations of atomoxetine have certain properties that are not ideal in all situations. For example, the twice daily morning/early evening administration schedule is beneficial for many ADD patients because it provides high plasma levels of atomoxetine to the patient at times of the day when symptoms are likely to be the most problematic. However, it requires two administrations of atomoxetine. For juvenile patients, the second administration generally needs to be performed at school. A once daily "pulsed" dosage form of atomoxetine providing high plasma levels of atomoxetine immediately after administration, and again about 6 to 8 hours after administration is highly desirable. Single dosage forms that provide particular plasma profiles of atomoxetine are thus desirable.

Additionally, because the dosage forms of atomoxetine are quite small it is possible to formulate this active agent for less frequent administration, providing dosage forms for administration every second day, or for biweekly administration. Improved control of atomoxetine pharmacokinetic properties may be achieved with alternative formulations.

WO 03/013492 A discloses atomoxetine in combination with CYP2D6 inhibitors for the treatment of neurological disorders.

The present invention addresses these and other needs for improved atomoxetine dosage forms, particularly controlled-release, pulsed-release, and sustained-release dosage forms.

### SUMMARY OF THE INVENTION

In a first aspect, an atomoxetine solid dosage formulation comprises a matrix, wherein the matrix comprises a pharmaceutically effective amount of atomoxetine and a wax material.

A method of making the wax formulation comprising hot melting a waxy material to form a melt, granulating atomoxetine with the melt to form a granulate; milling the granulate; and compressing granulate to form a matrix is also provided.

A press-coat dosage form comprises a core composition comprising atomoxetine or a pharmcetically acceptable salt thereof, a waxy material; and a coating composition comprising hydrophilic polymer, wherein the coating composition is press-coated onto the core composition.

In another aspect, the invention provides a press-coat dosage form comprising a core composition comprising atomoxetine or a pharmcetically acceptable salt thereof and carnauba wax; and a coating composition comprising atomoxetine or a pharmcetically acceptable salt thereof and hydroxypropylmethyl cellulose (HPMC), wherein the coating composition is press-coated onto the core.

A method for preparing a press-coat dosage form comprising providing a core composition comprising atomoxetine or a salt thereof and a waxy material, providing a coating composition comprising the atomoxetine or a salt thereof and a hydrophilic polymer, and press-coating the coating composition onto the core composition to provide the press-coat dosage form is also provided.

Described herein are sprinkle dosage forms. Described is also a dosage form of atomoxetine comprising an easily openable capsule enclosing a plurality of micropellets, where each of the micropellets comprises a seed coated with a first coating mixture of atomoxetine and a suitable binder such as polyvinylpyrrolidone, HPMC, HPC, PVA, or any other suitable binder, and coated thereon with a second coating mixture of about 90% to about 70% by weight of a non-hydrophilic polymer and about 10% to about 30% by weight of a hydrophilic polymer.

Also described herein are taste-masked liquid dosage forms and fast-dissolve dosage forms. In one instance the disclosure provides a taste-masked liquid dosage form, comprising: atomoxetine particles; and polymer encapsulating the particles, wherein the polymer has quaternary ammonium groups on the polymer backbone; and a liquid suspending medium for suspending the encapsulated particles, wherein the liquid suspending medium comprises a water-based medium adjusted to a predetermined pH at which atomoxetine remains substantially insoluble.

Described herein are chewable taste-masked dosage forms. A chewable taste-masked dosage form, comprising: a microcapsule of about 10 microns to about 1.5 mm in diameter having a core comprising, atomoxetine or a pharmcetically acceptable salt thereof, and a polymer mixture coating having sufficient elasticity to withstand chewing. The polymeric mixture coating comprises about 50% by weight of a polymer that forms a polymeric film at temperatures of at least about 30 °C; and about 50% by weight of a low temperature film forming copolymer that forms a polymeric film at temperatures less than about 25 °C; and the polymeric mixture coating is adapted to release atomoxetine in the stomach.

Controlled-release dosage forms of atomoxetine may be characterized by certain plasma level profiles of atomoxetine. For example, an oral dosage form comprises atomoxetine or a pharmcetically acceptable salt thereof in controlled-release form which provides a maximum concentration of atomoxetine (C*ₘₐₓ*) and a concentration of atomoxetine at about 48 hours after administration (C*₄₈*), wherein the ratio of C*ₘₐₓ* to C*₄₈* is less than about 4:1.

In another aspect, an oral dosage form comprises atomoxetine or a phanncetically acceptable salt thereof in controlled-release form, which, at steady-state, provides a maximum atomoxetine concentration (C*ₘₐₓ*), an atomoxetine plasma concentration at about 12 hours after administration (C*₁₂*), and an atomoxetine plasma concentration at about 24 hours after administration (C*₂₄*). The average atomoxetine plasma concentration produced by this dosage form between C*ₘₐₓ* and C*₁₂* is substantially equal to the average atomoxetine plasma concentration between C*₁₂* and C*₂₄*.

Pulsed-release formulations are also described. An oral dosage form comprising atomoxetine or a pharmaceutically acceptable salt thereof in sustained-release form, which, at steady-state, provides a first maximum atomoxetine plasma concentration (C*ₘₐₓ₁*) between 0 hours and about 12 hours after administration, and a second maximum atomoxetine plasma concentration (C*ₘₐₓ₂*) between about 12 hours and about 24 hours after administration, wherein the ratio of C*ₘₐₓ₁* and C*ₘₐₓ₂* is between about 1:4 and about 4:1.

An oral dosage form comprising atomoxetine or a pharmaceutically acceptable salt thereof in pulsed-release form, which, at steady-state, provides a first maximum atomoxetine plasma concentration (C*ₘₐₓ₁*) between 0 hours and about 3 hours after administration, and a second maximum atomoxetine plasma concentration (C*ₘₐₓ₂*) between about 5 hours and about 9 hours after administration, wherein the ratio of C*ₘₐₓ₁* and C*ₘₐₓ₂* is between about 1:4 and about 4:1.

Described herein are sustained-release oral dosage forms. The disclosure provides a sustained-release oral dosage form of atomoxetine comprising a first subunit and a second subunit, wherein the first subunit comprises atomoxetine and a first release-retarding material and the second subunit comprises atomoxetine and a second release-retarding material. The first and second release-retarding material can be the same or different. The dosage form, at steady-state, provides a maximum plasma concentration of the atomoxetine (C*ₘₐₓ*) and an plasma concentration of atomoxetine at about 24 hours after administration (C*₂₄*), wherein the ratio of C*ₘₐₓ* to C*₂₄* is less than about 4:1.

Described herein is a semi-delayed-release atomoxetine dosage form that provides a moderate atomoextine plasma concentration upon AM administration and a larger atomoxetine plasma concentration in the afternoon or evening. Thus, the disclosure provides a pulsed-release dosage form as described above, wherein the ratio of Cₘₐₓ₁ to Cₘₐₓ₂ is greater than 1: 1.5 and less than about 1:4.

Described herein are controlled-release dosage forms that may be characterized by values for the Area Under the Curve (AUC) of the plasma level of atomoxetine versus time after administration. A controlled-release dosage form of atomoxetine or a salt thereof provides an AUC between 0 and 24 hours after administration that is more than 80 percent and less than 120 percent of the AUC provided by an equivalent weight of STRATTERA between 0 and 24 hours after administration.

Described herein are methods of treating attention deficit disorder, comprising orally administering to a human on a once-daily basis an oral sustained-release dosage form comprising atomoxetine or a pharmcetically acceptable salt thereof which, at steady-state, provides a maximum atomoxetine plasma concentration (C*ₘₐₓ*) and an atomoxetine plasma concentration at about 24 hours after administration (C*₂₄*), wherein the ratio of C*ₘₐₓ* to C*₂₄* is less than about 4: 1.

Described herein are controlled-release wax dosage forms, press-coat dosage forms, sprinkle dosage forms, and taste masked dosage forms of atomoxetine or a pharmcetically acceptable salt thereof and at least one excipient having the dissolution profiles described above. Also described herein are wax dosage forms, press-coat dosage forms, sprinkle dosage forms, and taste masked dosage forms that produce certain plasma levels of atomoxetine upon administration. For example the disclosure provides such dosage forms characterized by the plasma concentration of atomoxetine described above.

Also described herein are wax dosage forms, press-coat dosage forms, sprinkle dosage forms, and taste masked dosage forms as sustained-release oral dosage forms comprising a first subunit and a second subunit. The first subunit comprises atomoxetine and a first release-retarding material and the second subunit comprises atomoxetine and a second release-retarding material, wherein the first and second release-retarding material can be the same or different, and wherein the dosage form, at steady-state, provides a maximum atomoxetine plasma concentration (C*ₘₐₓ*) and an atomoxetine plasma concentration at about 24 hours after administration (C*₂₄*), wherein the ratio of C*ₘₐₓ* to C*₂₄* is less than about 4:1.

Further described herein are wax dosage forms, press-coat dosage forms, sprinkle dosage forms, and taste masked dosage forms as controlled-release oral dosage forms which provides the AUC values described above.

Described herein are combination dosage forms, which contain atomoxetine together with at least one other active agent. For example, the disclosure provides an oral dosage form comprising atomoxetine or a pharmaceutically acceptable salt thereof together with at least one active agent that is a stimulant, such as methylphenidate, dextroamphetamine, amphetamine, or pemoline; a tricyclic antidepressant such as desipramine, imipramine, or nortryptiline; bupropion; an alpha-adrenergic antagonist such as clonidine or guanfacine; a mood stabilizer such as lithium, valproate, carbamazepine; or a selective serotonin reuptake inhibitor such as paroxetine, sertaline, or fluvoxamine.

The disclosure also relates to the following preferred features. Preferred feature 1 provides, a solid dosage formulation comprising a matrix, wherein the matrix comprises a pharmaceutically effective amount of atomoxetine or a pharmaceutically acceptable salt thereof; and a wax material. Preferred feature 2 provides the solid dosage formulation of Preferred feature 1, wherein the matrix comprises a pharmaceutically effective amount of atomoxetine hydrochloride. Preferred feature 3 provides the solid dosage formulation of Preferred feature 2, wherein the wax material includes carnauba wax, glyceryl behenate, castor wax, or any combination thereof. Preferred feature 4 provides the solid dosage form of any one of Preferred features 1 to 3, wherein the matrix is coated with a coating composition. Preferred feature 5 provides the solid dosage form of Preferred feature 4, wherein the coating composition is a functional coating composition. Preferred feature 6 provides the solid dosage form of Preferred feature 5, wherein the functional coating composition comprises a non-water permeable component; and a water-soluble component. Preferred feature 7 provides the solid dosage form of Preferred feature 4, wherein the coating composition is a non-functional coating composition. Preferred feature 8 provides the solid dosage form of Preferred feature 7, wherein the non-functional coating composition comprises a water-soluble component in the substantial absence of a non-water-permeable component. Preferred feature 9 provides the solid dosage form of Preferred feature 8, wherein the non-functional coating composition comprises pharmaceutically acceptable dyes, pigments, or mixtures thereof preferred feature 10 provides the solid dosage form of Preferred feature 1 or Preferred feature 2, wherein the matrix further comprises a processing aid. Preferred feature 11 provides the solid dosage form of Preferred feature 1, wherein the matrix further comprises an additional active agent.

Preferred feature 12 provides a method of making a solid dosage form comprising a matrix, the method comprising: hot melting a waxy material to form a melt, granulating atomoxetine hydrochloride with the melt to form a granulate; milling the granulate; and compressing granulate to form a matrix. Preferred feature 13 provides the method according to Preferred feature 12, further comprising blending the granulate with a processing aid; prior to compressing the granulate to form a matrix. Preferred feature 13 provides the method according to Preferred feature 13, further comprising coating the matrix with a functional and/or a non-functional coating. Preferred feature 15 provides the solid dosage form resulting from the method of Preferred feature 13.

Preferred feature 16 provides the solid dosage form of Preferred feature 2, having a size that is substantially smaller than the size of a same strength dosage form of STRATTERA. Preferred feature 17 provides a tablet comprising the solid dosage form of Preferred feature 1 or Preferred feature 2. Preferred feature 18 provides the tablet of Preferred feature 17, further comprising a functional or non-functional coating. Preferred feature 19 provides a capsule comprising the solid dosage form of Preferred feature 1 or Preferred feature 2.

Preferred feature 20 provides a press-coat dosage form comprising a core composition comprising an active agent, which active agent is atomoxetine or a pharmaceutically acceptable salt thereof, a waxy material; and a coating composition comprising a hydrophilic polymer, wherein the coating composition is press-coated onto the core composition. Preferred feature 21 provides the press-coat dosage form of Preferred feature 20, wherein the active agent is atomoxetine hydrochloride. Preferred feature 22 provides the press-coat dosage form of Preferred feature 21, wherein the coating composition further comprises the active agent. Preferred feature 23 provides the press-coat dosage form of Preferred feature 22, wherein the active agent comprising the coating composition is atomoxetine hydrochloride. Preferred feature 24 provides the press-coat dosage form of Preferred feature 22, wherein the ratio of the active agent in the core composition to the active agent in the coating composition is about 1:99 to about 99:1. Preferred feature 25 provides the press-coat dosage form of Preferred feature 22, wherein the ratio of the active agent in the core composition to the active agent in the coating composition greater than about 5:3. Preferred feature 26 provides the press-coat dosage form of any one of Preferred features 20 to 25, wherein the waxy material is carnauba wax, tribehenin, fatty alcohols, lauryl alcohol, myristyl alcohol, stearyl alcohol, palmityl alcohol, fatty acids, lauric acid, myristic acid, stearic acid, palmitic acid, polyethylenes, castor wax, C₁₆₋₃₀ fatty acid triglycerides, beeswax, or a mixture of any combination thereof. Preferred feature 27 provides the press-coat dosage form of any one of Preferred features 20 to 26, wherein the hydrophilic polymer comprises a hydrophilic cellulose polymer. Preferred feature 28 provides the press-coat dosage form of Preferred feature 27, comprising a core composition comprising an active agent which is atomoxetine hydrochloride and wherein the hydrophilic cellulose polymer is hydroxypropylmethyl cellulose (HPMC). Preferred feature 29 provides the press-coat dosage form of Preferred feature 20, comprising the core composition comprising an active agent which is atomoxetine or atomoxetine hydrochloride, carnauba wax; and the coating composition comprising the active agent and hydroxypropylmethyl cellulose, wherein the coating composition is press-coated onto the core. Preferred feature 30 provides the press-coat dosage form of Preferred feature 29, comprising the core composition comprising atomoxetine hydrochloride and carnauba wax, the coating composition comprising atomoxetine hydrochloride and hydroxypropylmethyl cellulose, wherein the coating composition is press-coated onto the core, and comprising an additional coating composition comprising atomoxetine hydrochloride. Preferred feature 31 provides the press-coat dosage form of Preferred feature 30, wherein the additional coating composition is an immediate-release coating composition. Preferred feature 32 provides the press-coat dosage form of any one of Preferred features 29 to 31, wherein the active agent in the core composition and the active agent in the coating composition are present in an amount to provide a substantially zero order release profile. Preferred feature 33 provides the press-coat dosage form of any one of Preferred features 29 to 31, wherein the active agent in the core composition and the active agent in the coating composition are present in an amount to provide a substantially first order release profile. Preferred feature 34 provides the press-coat dosage form of any one of Preferred features 29 to 31, wherein the active agent in the core composition and the active agent in the coating composition are present in an amount to provide a substantially second order release profile. Preferred feature 35 provides the press-coat dosage form of any one of Preferred features 20 to 34, wherein the press-coat dosage form is a tablet.

Preferred feature 36 provides a method for preparing a press-coat dosage form, the method comprising providing a core composition comprising atomoxetine or a pharmaceutically acceptable salt thereof and a waxy material, providing a coating composition comprising atomoxetine or a pharmaceutically acceptable salt thereof and a hydrophilic polymer, and press-coating the coating composition onto the core composition to provide the press-coat dosage form.

Preferred feature 37 provides a dosage form of atomoxetine comprising an easily openable capsule enclosing a plurality of micropellets, where each of the micropellets comprises a seed coated with a first coating mixture of atomoxetine and polyvinylpyrrolidone and coated thereon with a second coating mixture of about 70% to about 90% by weight of a non-hydrophilic polymer and about 10% to about 30% by weight of a hydrophilic polymer. Preferred feature 38 provides the dosage form of Preferred feature 37, wherein the non-hydrophilic polymer is ethyl cellulose. Preferred feature 39 provides the dosage form of Preferred feature 37 or 38, wherein the hydrophilic polymer is hydroxypropyl methyl cellulose. Preferred feature 40 provides the dosage form of Preferred feature 37, wherein the dosage form is formulated as a sustained-release dosage form. Preferred feature 41 provides the dosage form of Preferred feature 37, wherein the weight of the second coating mixture is about 5-10% of the weight of the micropellets before the second coating is applied. Preferred feature 42 provides the dosage form of Preferred feature 37, wherein the second coating mixture comprises about 3 parts ethylcellulose to 1 about part hydroxypropylcellulose. Preferred feature 43 provides the dosage form of Preferred feature 37, wherein the polyvinylpyrrolidone has a molecular weight of about 30,000 to about 50,000. Preferred feature 44 provides the dosage form of Preferred feature 43 wherein the polyvinylpyrrolidone has a molecular weight of about 40,000. Preferred feature 45 provides the dosage form of Preferred feature 37 wherein the seed is sugar having a mesh size of 60/80. Preferred feature 46 provides the dosage form of Preferred feature 37 further wherein the micropellets have a mean diameter of about 0.5 to about 0.7 mm.

Preferred feature 47 provides a taste-masked liquid dosage form, comprising particles of an active agent, wherein the active agent is atomoxetine or a pharmaceutically acceptable salt thereof; and a polymer encapsulating the particles, wherein the polymer has quaternary ammonium groups on the polymer backbone; and a liquid suspending medium for suspending the encapsulated particles, wherein the liquid suspending medium comprises a water-based medium adjusted to a predetermined pH at which the active agent remains substantially insoluble. Preferred feature 48 provides the taste-masked liquid dosage form of Preferred feature 47, wherein the liquid dosage form is a fast-dissolve form. Preferred feature 49 provides the taste-masked liquid dosage form of Preferred feature 47, wherein the active agent is atomoxetine hydrochloride. Preferred feature 50 provides the liquid dosage form of Preferred feature 47, wherein the polymer is a copolymer of acrylic and methacrylic acid esters with quaternary ammonium groups, or a copolymer of methyl methacrylate and triethylammonium methacrylate. Preferred feature 51 provides the liquid dosage form of any one of Preferred features 47 to 50, wherein the ratio of polymer to active agent is about 0.01:1 to about 10:1. Preferred feature 52 provides the liquid dosage form of Preferred feature 47, wherein the active agent is in the form of ion-exchange complex or a cyclodextrin complex, or as a mixture with a wax, a lipid, a dissolution inhibitor, a taste-masking agent, a taste-suppressing agent, a carrier, an excipient, a filler, or a combination comprising at least one of the foregoing forms. Preferred feature 53 provides the liquid dosage form of Preferred feature 47, further comprising an additional polymer, wherein the additional polymer is a cellulose ether, a cellulose ester, or a polymer that dissolves at acidic or alkaline pH. Preferred feature 54 provides the liquid dosage form of Preferred feature 47, wherein the suspending medium further comprises a buffering agent. Preferred feature 55 provides the liquid dosage form of Preferred feature 54, wherein the buffering agent has a buffer strength of 0.1 to 1 moles/liter. Preferred feature 56 provides the liquid dosage form of Preferred feature 47, further comprising a stabilizer, wherein the stabilizer is methyl cellulose, sodium alginate, xanthan gum, (poly)vinyl alcohol, microcrystalline cellulose, colloidal silicas, bentonite clay, or a combination of any of the foregoing stabilizers. Preferred feature 57 provides the liquid dosage form of Preferred feature 47, wherein the particle size is about 0.1 to about 500 micrometers.

Preferred feature 58 provides a chewable taste-masked dosage form, comprising a microcapsule of about 10 microns to about 1.5 mm in diameter having a core comprising a pharmaceutically active agent, which is atomoxetine or a pharmaceutically acceptable salt thereof, and a polymer mixture coating having sufficient elasticity to withstand chewing; the polymeric mixture coating comprising about 50% by weight of a polymer that forms a polymeric film at temperatures of at least about 30 °C; and about 50% by weight of a low temperature film forming copolymer that forms a polymeric film at temperatures less than about 25 °C; the polymeric mixture coating being adapted to release the pharmaceutically active agent in the stomach. Preferred feature 59 provides the taste-masked dosage form of Preferred feature 58, wherein the polymer that forms a polymeric film at temperatures of at least about 30 °C is an ethyl cellulose. Preferred feature 60 provides the taste-masked dosage form of Preferred feature 58, wherein the dosage form is a sustained-release dosage form. Preferred feature 61 provides the taste-masked dosage form of Preferred feature 58, wherein the low temperature film forming copolymer is a methacrylic acid ester copolymer or a styrene acrylate copolymer. Preferred feature 62 provides the taste-masked dosage form of Preferred feature 61, wherein the low temperature film forming copolymer comprises a polymethacrylic acid ester copolymer having a mean molecular weight of about 800,000. Preferred feature 63 provides the taste-masked dosage form of Preferred feature 58, wherein the core further comprises a diluent. Preferred feature 64 provides the taste-masked dosage form of Preferred feature 58, wherein said polymer coating further comprises a plasticizer. Preferred feature 65 provides the taste-masked dosage form of preferred feature 64, wherein the plasticizer is polyethylene glycol, triacetin, vinylpyrrolidone, diethyl phthallate, dibutylsebacate, or a citric acid ester. Preferred feature 66 provides the taste-masked dosage form of Preferred feature 58, wherein the active agent is atomoxetine hydrochloride. Preferred feature 67 provides the taste-masked dosage form of Preferred feature 58, wherein the polymeric mixture coating comprises about 70% by weight ethyl cellulose polymer aqueous dispersion and about 30% by weight of the low temperature film forming copolymer, the polymeric mixture coating being adapted to release the active agent in the stomach. Preferred feature 68 provides the taste-masked dosage form of Preferred feature 58, formulated for rapid release of the active agent in the upper intestinal tract. Preferred feature 69 provides the taste-masked dosage form of Preferred feature 68, wherein the polymeric mixture coating comprises about 50% by weight ethyl cellulose polymer aqueous dispersion and about 50% by weight low temperature film forming polymer, wherein the polymeric mixture coating is adapted to release the active agent in the upper intestinal tract. Preferred feature 70 provides the taste-masked dosage form of Preferred feature 68, wherein the low temperature film forming copolymer comprises a polymethacrylic acid ester copolymer having a mean molecular weight of about 800,000. Preferred feature 71 provides the taste-masked dosage form of Preferred feature 68, wherein the core further comprises a diluent. Preferred feature 72 provides the taste-masked dosage form of Preferred feature 68, wherein the polymeric mixture coating further comprises a plasticizer. Preferred feature 73 provides the taste-masked dosage form of Preferred feature 72, wherein the plasticizer is polyethylene glycol, triacetin, vinylpyrrolidone, diethyl phthallate, dibutylsebacate, or a citric acid ester.

Preferred feature 74 provides the dosage form of any one of Preferred features 1, 20, or 37, wherein the dosage form provides an AUC between 0 and 24 hours after administration that is more than 80 percent and less than 120 percent of the AUC provided by an equivalent weight of STRATTERA between 0 and 24 hours after administration. Preferred feature 75 provides an oral dosage form comprising atomoxetine or a pharmaceutically acceptable salt thereof in controlled-release form which provides a maximum atomoxetine plasma concentration (C*ₘₐₓ*) and an atomoxetine plasma concentration at about 48 hours after administration to a patient (C*₄₈*), wherein the ratio of C*ₘₐₓ* to C*₄₈* is less than about 4:1. Preferred feature 76 provides an oral dosage form comprising atomoxetine or a pharmaceutically acceptable salt thereof in controlled-release form which provides a maximum atomextine plasma concentration (C*ₘₐₓ*) and an atomoxetine plasma concentration at about 24 hours after administration to a patient (C*₂₄*), wherein the ratio of C*ₘₐₓ* to C*₂₄* is less than about 4:1. Preferred feature 77 provides the oral dosage form of Preferred feature 75 or Preferred feature 76, wherein the patient is a human patient. Preferred feature 78 provides the oral dosage form of Preferred feature 77, wherein the human patient is an extensive metabolizer.

Preferred feature 79 provides the oral dosage form of any one of Preferred features 1, 20, or 37, which when administered to a human patient provides a maximum atomoxetine plasma concentration (C*ₘₐₓ*) and an atomoxetine plasma concentration at about 48 hours after administration (C*₄₈*), wherein the ratio of C*ₘₐₓ* to C*₄₈* is less than about 4:1. Preferred feature 80 provides the oral dosage form of any one of Preferred features 1, 20, or 37, which when administered to a human patient provides a maximum atomoxetine plasma concentration (C*ₘₐₓ*) and an atomoxetine plasma concentration at about 24 hours after administration (C*₂₄*), wherein the ratio of C*ₘₐₓ* to C*₂₄* is less than about 4:1. Preferred feature 81 provides the oral dosage form of any one of Preferred features 75 to 80, comprising atomoxetine hydrochloride. Preferred feature 82 provides the oral dosage form of any one of Preferred features 75 to 81, wherein the ratio is achieved at steady-state.

Preferred feature 83 provides an oral dosage form comprising atomoxetine or a pharmaceutically acceptable salt thereof in controlled-release form, which when administered to a human patient provides, at steady-state, a maximum atomoxetine plasma concentration (C*ₘₐₓ*), an atomoxetine plasma concentration at about 12 hours after administration (C*₁₂*), and an atomoxetine plasma concentration at about 24 hours after administration (C*₂₄*), wherein the average atomoxetine plasma concentration between C*ₘₐₓ* and C*₁₂* is substantially equal to the average atomoxetine plasma concentration between C*₁₂* and C*₂₄*. Preferred feature 84 provides the oral dosage form of Preferred feature 83, which when administered to a juvenile human patient, provides, at steady-state, a maximum atomoxetine plasma concentration (C*ₘₐₓ*), an atomoxetine plasma concentration at about 12 hours after administration (C*₁₂*), and an atomoxetine plasma concentration at about 24 hours after administration (C*₂₄*), wherein the average atomoxetine plasma concentration between C*ₘₐₓ* and C*₁₂* is substantially equal to the average atomoxetine plasma concentration between C*₁₂* and C*₂₄*.

Preferred feature 85 provides the oral dosage form of any one of Preferred features 1, 20, or 37, which when administered to a human patient provides a maximum atomoxetine plasma concentration (C*ₘₐₓ*), an atomoxetine plasma concentration at about 12 hours after administration (C*₁₂*), and an atomoxetine plasma concentration at about 24 hours after administration (C*₂₄*), wherein the average atomoxetine plasma concentration between C*ₘₐₓ* and C*₁₂* is substantially equal to the average atomoxetine plasma concentration between *C₁₂* and C*₂₄*. Preferred feature 86 provides the dosage form of any one of Preferred features 83 to 85, comprising atomoxetine hydrochloride. Preferred feature 87 provides the oral dosage form of Preferred feature 85 or 86, which provides a C*ₘₐₓ* at between 5.5 and 12 hours after administration. Preferred feature 88 provides the oral dosage form of Preferred feature 85 or 86, which provides a C*ₘₐₓ* at between 2 and 3.5 hours after administration.

Preferred feature 89 provides an oral dosage form comprising atomoxetine or a pharmaceutically acceptable salt thereof in sustained-release form, which, at steady-state, provides a first maximum atomoxetine plasma concentration (C*ₘₐₓ₁*) between 0 hours and about 12 hours after administration, and a second maximum atomoxetine plasma concentration (C*ₘₐₓ₂*) between about 12 hours and about 24 hours after administration, wherein the ratio of C*ₘₐₓ₁* and C*ₘₐₓ₂* is between about 1:4 and about 4:1.

Preferred feature 90 provides an oral dosage form comprising atomoxetine or a pharmaceutically acceptable salt thereof in pulsed-release form, which, at steady-state, provides a first maximum atomoxetine plasma concentration (C*ₘₐₓ₁*) between 0 hours and about 3 hours after administration, and a second maximum atomoxetine plasma concentration (C*ₘₐₓ₂*) between about 5 hours and about 9 hours after administration, wherein the ratio of *Cₘₐₓ₁* and C*ₘₐₓ₂*.is between about 1:4 and about 4:1. Preferred feature 91 provides the oral dosage form of Preferred feature 89 or 90, comprising atomoxetine hydrochloride. Preferred feature 92 provides the oral dosage form of Preferred feature 89, which, at steady-state, provides a first maximum atomoxetine plasma concentration (C*ₘₐₓ₁*) between 0 hours and about 12 hours after administration, a second maximum atomoxetine plasma concentration (C*ₘₐₓ₂*) between about 12 hours and about 24 hours after administration, and an atomoxetine plasma concentration at about 24 hours after administration (C*₂₄*), wherein the average atomoxetine plasma concentration between about C*ₘₐₓ₁* and about *Cₘₐₓ₂* is substantially equal to the average atomoxetine plasma concentration between about C*ₘₐₓ₂* and about C*₂₄*. Preferred feature 93 provides the oral dosage form of Preferred feature 90, which, at steady-state, provides a first maximum atomoxetine plasma concentration (C*ₘₐₓ₁*) and a first minimum atomoxetine plasma concentration (C*ₘᵢₙ₁*) between 0 hours and about 5 hours after administration, a second maximum atomoxetine plasma concentration (C*ₘₐₓ₂*) between about 5 hours and about 9 hours after administration, and an atomoxetine plasma concentration at about 24 hours after administration (C*₂₄*), wherein the ratio of C*ₘₐₓ₁* to C*ₘᵢₙ₁* is less than about 4:1 or the ratio of C*ₘₐₓ₂* to C*₂₄* is less than about 4:1. Preferred feature 94 provides the oral dosage form of Preferred feature 89, which, at steady-state, provides a first maximum atomoxetine plasma concentration (C*ₘₐₓ₁*) and a first minimum atomoxetine plasma concentration (C*ₘᵢₙ₁*) between 0 hours and about 12 hours after administration, a second maximum atomoxetine plasma concentration (C*ₘₐₓ₂*), between 12 and 24 hours after administration, and an atomoxetine plasma concentration at about 24 hours after administration (C*₂₄*), wherein the ratio of *Cₘₐₓ₁* to C*ₘᵢₙ₁* is less than about 4:1 or the ratio of *Cₘₐₓ₂* to C*₂₄* is less than about 4:1. Preferred feature 95 provides the oral dosage form of Preferred feature 94, wherein C*ₘₐₓ₂* occurs about 12 to about 14 hours after administration. Preferred feature 96 provides the oral dosage form of Preferred feature 93, wherein C*ₘₐₓ₂* occurs about 6 to about 8 hours after administration. Preferred feature 97 provides the oral dosage form of any one of Preferred features 93 to 96, wherein, at steady-state, the difference between the ratio of C*ₘₐₓ₁* to *Cₘᵢₙ₁* and the ratio of *Cₘₐₓ₂* to C₂₄ is less than about 30%. Preferred feature 98 provides the oral dosage form of Preferred feature 97, wherein the difference between the ratio of C*ₘₐₓ₁* to C*ₘᵢₙ₁* and the ratio of *Cₘₐₓ₂* to C₂₄ is less than about 20%.

Preferred feature 99 provides a sustained-release oral dosage form comprising a first subunit and a second subunit, wherein the first subunit comprises atomoxetine and a first release-retarding material and the second subunit comprises atomoxetine and a second release-retarding material, wherein the first and second release-retarding material can be the same or different, and wherein the dosage form, at steady-state, provides a maximum atomoxetine plasma concentration (C*ₘₐₓ*) and an atomoxetine plasma concentration at about 24 hours after administration (C*₂₄*), wherein the ratio of C*ₘₐₓ* to C*₂₄* is less than about 4:1. Preferred feature 100 provides the oral dosage form of Preferred feature 89 or 90, wherein the ratio of *Cₘₐₓ₁* to C*ₘₐₓ₂* is greater than 1:1.5 and less than about 1:4. Preferred feature 101 provides the oral dosage form of Preferred feature 100, wherein the ratio of C*ₘₐₓ₁* to *Cₘₐₓ₂* is greater than 1:3.

Preferred feature 102 provides a method of treating attention deficit disorder, the method comprising orally administering to a human on a once-daily basis an oral sustained-release dosage form comprising atomoxetine or a pharmaceutically acceptable salt thereof which, at steady-state, provides a maximum atomoxetine plasma concentration (C*ₘₐₓ*) and an atomoxetine plasma concentration at about 24 hours after administration (C*₂₄*), wherein the ratio of C*ₘₐₓ* to C*₂₄* is less than about 4:1.

Preferred feature 103 provides the dosage form of any one of Preferred features 1, 20, or 37, which provides an AUC between 0 and 24 hours after administration that is more than 80 percent and less than 120 percent of the AUC provided by an equivalent weight of STRATTERA between 0 and 24 hours after administration.

Preferred feature 104 provides a dosage form of comprising atomoxetine which provides an AUC between 0 and 24 hours after administration that is more than 80 percent and less than 120 percent of the AUC provided by 2 times the equivalent weight of STRATTERA between 0 and 24 hours after administration.

Preferred feature 105 provides an oral dosage form comprising atomoxetine or a pharmaceutically acceptable salt thereof in sustained-release form, which, at steady-state, provides a first AUC (AUC₁) between 0 and about 12 hours and a second AUC (AUC₂) between about 12 hours and about 24 hours, wherein difference between AUC₂ and AUC₁ is less than about 50%. Preferred feature 106 provides the oral dosage form of Preferred feature 105, wherein AUC₁ and AUC₂ are about equal.

Preferred feature 107 provides an oral dosage form comprising atomoxetine or a pharmaceutically acceptable salt thereof together with at least one active agent selected from methylphenidate, dextroamphetamine, amphetamine, pemoline, desipramine, imipramine, nortryptiline, bupropion, clonidine, guanfacine, lithium, valproate, carbamazepine, paroxetine, sertaline, and fluvoxamine.

Preferred feature 108 provides a dosage form according to any one of Preferred features 1, 20, 37, 47, 58, 75, 82, 88, or 89, comprising at least one additional active agent selected from methylphenidate, dextroamphetamine, amphetamine, pemoline, desipramine, imipramine, nortryptiline, bupropion, clonidine, guanfacine, lithium, valproate, carbamazepine, paroxetine, sertaline, and fluvoxamine.

### DETAILED DESCRIPTION OF THE INVENTION

Described herein are improved formulations comprising atomoxetine such as, for example, controlled-release formulations, including, but not limited to wax formulations, press-coated formulations, easily administrable formulations, osmotic pump technology formulations, and combination formulations.

One type of formulation described herein is a controlled-release formulation. Controlled-release formulations, such as longer acting formulations that can be administered once daily or even less frequently, are particularly desirable for atomoxetine. Controlled-release formulations may provide many inherent therapeutic benefits that are not achieved with corresponding short acting, immediate-release preparations. For some attention deficit disorder patients blood levels of an ADD medicament must be maintained at a therapeutically effective level to provide symptomatic relief. Unless conventional rapid acting drug therapy is carefully administered at frequent intervals to maintain effective steady-state blood levels of atomoxetine, peaks and valleys in the blood level of atomoxetine occur because of the rapid absorption, systemic excretion of the compound and through metabolic inactivation, thereby producing special problems in maintaining efficacy. Additionally patient compliance, which can be problematic among juvenile patients, may be improved with dosage formulations that can be administered less frequently. The inconvenience of administering medicines during the school day can also be alleviated with sustained-release atomoxetine formulations.

Controlled-release formulations of atomoxetine may be formulated using osmotic pump technology. This technology uses osmotic pressure to deliver atomoxetine at a controlled rate. Osmotic pump, dosage formulations include a semi-permeable membrane surrounding a core that contains at least two components, one component comprising atomoxetine, the other comprising an osmotic push layer, such as an osmotically active polymer. Some time after the dosage form is swallowed water enters the membrane causing the push layer to swell, releasing atomoxetine at a controlled rate through a laser-drilled hole in the membrane. Osmotic pump technology thus may be useful in certain atomoxetine formulations.

Sustained-release formulations of atomoxetine can be administered once daily or even less frequently. Sustained-release formulations can be based on matrix technology. In this technology atomoxetine is embedded in an excipient that makes a non-disintegrating core called a matrix. Diffusion of atomoxetine occurs through the core.

A preferred sustained-release formulation is one that does not comprise a gelling agent. Formulations with gelling agents do not extend release of the drug beyond about five hours due to attrition of the gel when fully hydrated in the gastrointestinal tract. In addition, exhibit different release characteristics when administered with food.

Semi-delayed-release dosage forms, which are a type of pulsed- release dosage form, of atomoxetine are provided. Such dosage forms provide a moderate dosage immediately after administration and a larger dosage some hours after administration. Such semi-delayed-release dosage forms are particularly useful for providing a moderate atomoxetine plasma concentration upon AM administration and a larger atomoxetine plasma concentration in the afternoon or evening.

In other circumstances it may be desirable to precisely control the plasma levels of atomoxetine for a number of hours after administration. Pulsed-release formulations, containing some combination of immediate-release, sustained-release, and delayed-release formulations in the same dosage form can be used in place of multiple immediate and sustained-release dosages in such situations. Other types of pulse release formulations, which are tailored to provide a particular plasma level profile are useful in other types of clinical situations.

Enteric coated formulations, which protect the stomach against the irritant effects of atomoxetine, are also desirable. Such formulations can be coated with a composition that is non-toxic and includes a pharmaceutically acceptable enteric polymer which is predominantly soluble in the intestinal fluid, but substantially insoluble in the gastric juices.

Another issue is that the current tablet formulations may be inadequate for juvenile patients who require dosage forms that are easy to swallow. Easily administered formulations, such as chewable tablets, sprinkle forms, liquid formulations, taste-masked formulations, and fast dissolve tablets are thus desirable.

Patients suffering from attention deficit disorder often take multiple medications to effectively control their symptoms. Combinations, which contain atomoxetine and also contain one or more other active agents typically prescribed for patients suffering from attention deficit disorder, are convenient for administration and also improve patient compliance. For example, attention deficit disorder patients frequently receive stimulants, tricyclic anti-depressants, mood stabilizers, alpha-adrenergic agonists, or selective serotonin reuptake inhibitors. Thus formulations which incorporate both atomoxetine and one or more of these other active agents in a single dosage forms are desirable.

### CHEMICAL DESCRIPTION AND TERMINOLOGY

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising", "having", "including", and "containing" are to be construed as open-ended terms (i.e., meaning "including, but not limited to") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

The term "active agent" is meant to include solvates (including hydrates) of the free compound or salt, crystalline and non-crystalline forms, as well as various polymorphs. Unless otherwise specified, the term "active agent" is used herein to indicate atomoxetine or a salt thereof. For example, an active agent can include all optical isomers of the compound and all pharmaceutically acceptable salts thereof either alone or in combination.

Unless otherwise specified, or clearly indicated by the text, "atomoxetine" includes both the free base of atomoxetine, (-)-*N*-methyl-3-phenyl-3-(o-tolyloxy)-propyl amine, and all pharmaceutically acceptable salts of this compound. The preferred atomoxetine salt is atomoxetine hydrochloride. The term "atomoxetine or its salts" indicates the pharmaceutically acceptable salts of atomoxetine.

Certain formulations described herein may be "coated". The coating can be a suitable coating, such as, a functional or a non-functional coating, or multiple functional and/or non-functional coatings. By "functional coating" is meant to include a coating that modifies the release properties of the total formulation, for example, a sustained-release coating. By "non-functional coating" is meant to include a coating that is not a functional coating, for example, a cosmetic coating. A non-functional coating can have some impact on atomoxetine release due to the initial dissolution, hydration, perforation of the coating, etc., but would not be considered to be a significant deviation from the non-coated composition.

"Pharmaceutically acceptable salts" includes derivatives of the disclosed compounds, wherein the parent compound is modified by making non-toxic acid or base addition salts thereof, and further refers to pharmaceutically acceptable solvates, including hydrates, of such compounds and such salts. Examples of pharmaceutically acceptable salts include, but are not limited to, mineral or organic acid addition salts of basic residues such as amines; alkali or organic addition salts of acidic residues such as carboxylic acids; and the like, and combinations comprising one or more of the foregoing salts. The pharmaceutically acceptable salts include non-toxic salts and the quaternary ammonium salts of the parent compound formed, for example, from non-toxic inorganic or organic acids. For example, non-toxic acid salts include those derived from inorganic acids such as hydrochloric, hydrobromic, sulfuric, sulfamic, phosphoric, nitric and the like; other acceptable inorganic salts include metal salts such as sodium salt, potassium salt, cesium salt, and the like; and alkaline earth metal salts, such as calcium salt, magnesium salt, and the like, and combinations comprising one or more of the foregoing salts. Pharmaceutically acceptable organic salts includes salts prepared from organic acids such as acetic, trifluoroacetic, propionic, succinic, glycolic, stearic, lactic, malic, tartaric, citric, ascorbic, pamoic, maleic, hydroxymaleic, phenylacetic, glutamic, benzoic, salicylic, mesylic, esylic, besylic, sulfanilic, 2-acetoxybenzoic, fumaric, toluenesulfonic, methanesulfonic, ethane disulfonic, oxalic, isethionic, HOOC-(CH₂)ₙ-COOH where n is 0-4, and the like; organic amine salts such as triethylamine salt, pyridine salt, picoline salt, ethanolamine salt, triethanolamine salt, dicyclohexylamine salt, N,N'-dibenzylethylenediamine salt, and the like; and amino acid salts such as arginate, asparginate, glutamate, and the like, and combinations comprising one or more of the foregoing salts.

By "oral dosage form" is meant to include a unit dosage form prescribed or intended for oral administration. An oral dosage form may or may not comprise a plurality of subunits such as, for example, microcapsules or microtablets, packaged for administration in a single dose.

By "releasable form" is meant to include immediate-release, controlled-release, and sustained-release forms. Certain release forms can be characterized by their dissolution profile. "Dissolution profile" as used herein, means a plot of the cumulative amount of active ingredient released as a function of time. The dissolution profile can be measured utilizing the Drug Release Test <724>, which incorporates standard test USP 26 (Test <711>). A profile is characterized by the test conditions selected. Thus the dissolution profile can be generated at a preselected apparatus type, shaft speed, temperature, volume, and pH of the dissolution media.

A first dissolution profile can be measured at a pH level approximating that of the stomach. A second dissolution profile can be measured at a pH level approximating that of one point in the intestine or several pH levels approximating multiple points in the intestine.

A highly acidic pH may simulate the stomach and a less acidic to basic pH can simulate the intestine. By the term "highly acidic pH" it is meant a pH of about 1 to about 4. By the term "less acidic to basic pH" is meant a pH of greater than about 4 to about 7.5, preferably about 6 to about 7.5. A pH of about 1.2 can be used to simulate the pH of the stomach. A pH of about 6 to about 7.5, preferably about 6.8 can be used to simulate the pH of the intestine.

Release forms may also be characterized by their pharmacokinetic parameters. "Pharmacokinetic parameters" are parameters, which describe the *in vivo* characteristics of atomoxetine over time, including for example the *in vivo* dissolution characteristics and plasma concentration of atomoxetine. By "C*ₘₐₓ*" is meant the measured concentration of atomoxetine in the plasma at the highest observed concentration. By "C*₂₄*" is meant the concentration of atomoxetine in the plasma at about 24 hours. The term "T*ₘₐₓ*" refers to the time at which the concentration of atomoxetine in the plasma is the highest. "AUC" is the area under the curve of a graph of the concentration of atomoxetine (typically plasma concentration) vs. time, measured from one time to another.

By "sequestered form" is meant an ingredient that is not released or substantially not released at one hour after the intact dosage form comprising atomoxetine is orally administered. The term "substantially not released" is meant to include the ingredient that might be released in a small amount, as long as the amount released does not affect or does not significantly affect efficacy when the dosage form is orally administered to mammals, for example, humans, as intended.

By "instant-release" is meant a dosage form designed to ensure rapid dissolution of the active agent by modifying the normal crystal form of the active agent to obtain a more rapid dissolution.

By "immediate-release", it is meant a conventional or non-modified release form in which greater then or equal to about 50% or more preferably about 75% of atomoxetine is released within two hours of administration, preferably within one hour of administration.

By "controlled-release" it is meant a dosage form in which the atomoxetine release is controlled or modified over a period of time. Controlled can mean, for example, sustained, delayed or pulsed-release at a particular time. Alternatively, controlled can mean that the atomoxetine release is extended for longer than it would be in an immediate-release dosage for, i.e., at least over several hours.

By "delayed-release", it is meant that there is a time-delay before significant plasma levels of atomoxetine are achieved. A delayed-release atomoxetine avoids an initial burst of atomoxetine, or can be formulated so that atomoxetine release in the stomach is avoided.

A "pulsed-release" formulation can contain a combination of immediate-release, sustained-release, and/or delayed-release formulations in the same dosage form. A "semi-delayed-release" formulation is a pulsed-released formulation in which a moderate dosage is provided immediately after administration and a further dosage some hours after administration.

By "sustained-release" or "extended-release" is meant to include the release of atomoxetine at such a rate that blood (e.g., plasma) levels are maintained within a therapeutic range but below toxic levels for at least about 8 hours, preferably at least about 12 hours after administration at steady-state. The term "steady-state" means that a plasma level for a given active agent, such as atomoxetine, has been achieved and which is maintained with subsequent doses of the drug at a level which is at or above the minimum effective therapeutic level and is below the minimum toxic plasma level for a given active agent.

By "subunit" is meant to include a composition, mixture, particle, etc., that can provide an oral dosage form alone or when combined with other subunits. By "part of the same subunit" is meant to refer to a subunit comprising certain ingredients. For example, a subunit comprising atomoxetine and an additional active ingredient may be placed together with additional subunits in a capsule to provide an oral dosage form.

The term "thermo-responsive" as used herein includes thermoplastic compositions capable of softening, or becoming dispensable in response to heat and hardening again when cooled. The term also includes thermotropic compositions capable of undergoing change in response to the application of energy in a gradient manner. These compositions are temperature sensitive in their response to the application or withdrawal of energy. In certain embodiments thermo-responsive compositions possess the physical property of exhibiting solid, or solid-like properties at temperatures up to about 32 °C, and become fluid, semisolid, or viscous when at temperatures above about 32 °C, usually in the range of about 32 °C to about 40 °C. Thermo-responsive compositions, including thermo-responsive carriers, have the property of melting, dissolving, undergoing dissolution, softening, or liquefying and thereby forming a dispensable composition at the elevated temperatures. The thermo-responsive carrier can be lipophilic, hydrophilic, or hydrophobic. Another property of a thermo-responsive carrier is its ability to maintain the stability of the agent contained therein during storage and during delivery of the agent. A thermo-responsive composition can be easily excreted, metabolized, or assimilated, upon being dispensed into a biological environment.

By "water-soluble" active agent is meant an active agent, including atomoxetine hydrochloride, and other active agents that may be used in combination with atomoxetine that are at least slightly water-soluble (for example, about 1 to about 10 mg/ml at 25°C). Preferably, all active agents are moderately water-soluble (for example, less than about 100 mg/ml at 25°C), or highly water-soluble (for example, greater than about 100 mg/ml at 25°C).

By "water-insoluble" or "poorly soluble" active agent, it is meant an agent having a water solubility of less than 1 mg/ml, and in some cases even less than 0.1 mg/ml.

By "STRATTERA" is meant the trademarked form of atomoxetine hydrochloride marketed by Eli Lilly, Indianapolis, IN described in the FDA Package Insert, NDA 21-411, for this compound.

### DOSAGE FORMS: RELEASE PROPERTIES

The dosage forms comprising atomoxetine can be characterized by the release properties of the formulation. Certain dosage form can be targeted-release formulations wherein release occurs in a particular segment of the gastrointestinal tract, for example in the small intestine. Alternatively, the dosage forms can be immediate or modified release dosage forms in which the rate of atomoxetine release in the blood stream is regulated.

### IMMEDIATE-RELEASE DOSAGE FORMS

An immediate-release dosage form is one in which the release properties of the drug from the dosage form are essentially unmodified. Immediate-release dosage forms generally also have dissolution profiles in any given media that are unmodified from those of atomoxetine alone in the same media. An immediate-release dosage form preferably results in delivery of greater than or equal to about 50% or more preferably greater than or equal to about 75% atomoxetine within about 2 hours of administration, preferably within 1 hour of administration. An immediate-release dosage form may contain optional excipients so long as the excipients do not significantly extend the release time of the drug.

### SUSTAINED-RELEASE DOSAGE FORMS

A sustained-release form is a form suitable for providing controlled-release of atomoxetine over a period of time (e.g., 8 hours, 12 hours, 24 hours). Sustained-release dosage forms of atomoxetine may release atomoxetine at a rate independent of pH, for example, about pH 1.2 to about 7.5. Alternatively, sustained-release dosage forms may release the active agent at a rate dependent upon pH, for example, a lower rate of release at pH 1.2 and a higher rate of release at pH 7.5. Preferably, the sustained-release form avoids "dose dumping," the production of a rapid rise and in the blood or plasma concentration of atomoxetine, upon oral administration. The sustained-release oral dosage form can be formulated to provide for an increased duration of therapeutic action permitting effective once-daily dosing. Generally in a sustained-release dosage form the atomoxetine release extends longer e.g, by several hours, than atomoxetine release from the immediate-release dosage form.

A sustained-release dosage form generally comprises a release-retarding material. The release-retarding material can be, for example, in the form of a matrix or a coating. Atomoxetine in sustained-release form may be, for example, a particle of atomoxetine that is combined with a release-retarding material. The release-retarding material is a material that permits release of atomoxetine at a sustained rate in an aqueous medium. The release-retarding material can be selectively chosen so as to achieve, in combination with the other stated properties, a desired *in vitro* release rate.

Release-retarding materials can be hydrophilic and/ or hydrophobic polymers. Release-retarding materials include, for example acrylic polymers, alkylcelluloses, shellac, zein, hydrogenated vegetable oil, hydrogenated castor oil, and combinations comprising one or more of the foregoing materials. The oral dosage form can contain between about 1% and about 80% (by weight) of the release-retarding material. Suitable acrylic polymers include, for example, acrylic acid and methacrylic acid copolymers, methyl methacrylate copolymers, ethoxyethyl methacrylates, cyanoethyl methacrylate, aminoalkyl methacrylate copolymer, poly(acrylic acid), poly(methacrylic acid), methacrylic acid alkylamide copolymer, poly(methyl methacrylate), poly(methacrylic acid anhydride), methyl methacrylate, polymethacrylate, poly(methyl methacrylate) copolymer, polyacrylamide, aminoalkyl methacrylate copolymer, glycidyl methacrylate copolymers, and combinations comprising one or more of the foregoing polymers. The acrylic polymer may comprise a methacrylate copolymers described in NF XXIV as fully polymerized copolymers of acrylic and methacrylic acid esters with a low content of quaternary ammonium groups.

Suitable alkylcelluloses include, for example, ethylcellulose. Those skilled in the art will appreciate that other cellulosic polymers, including other alkyl cellulosic polymers, can be substituted for part or all of the ethylcellulose.

Other suitable hydrophobic materials are water-insoluble with more or less pronounced hydrophilic trends. The hydrophobic material may have a melting point of about 30 °C to about 200 °C, more preferably about 45 °C to about 90 °C. The hydrophobic material can include neutral or synthetic waxes, fatty alcohols (such as lauryl, myristyl, stearyl, cetyl or preferably cetostearyl alcohol), fatty acids, including fatty acid esters, fatty acid glycerides (mono-, di-, and tri-glycerides), hydrogenated fats, hydrocarbons, normal waxes, stearic acid, stearyl alcohol, hydrophobic and hydrophilic materials having hydrocarbon backbones, and combinations comprising one or more of the foregoing materials. Suitable waxes include beeswax, glycowax, castor wax, carnauba wax and wax-like substances, e.g., material normally solid at room temperature and having a melting point of from about 30 °C to about 100 °C, and combinations comprising one or more of the foregoing waxes.

In other instances, the release-retarding material may comprise digestible, long chain (e.g., C₈ - C₅₀, preferably C₁₂ -C₄₀), substituted or unsubstituted hydrocarbons, such as fatty acids, fatty alcohols, glyceryl esters of fatty acids, mineral and vegetable oils, waxes, and combinations comprising one or more of the foregoing materials. Hydrocarbons having a melting point of between about 25 °C and about 90 °C may be used. Of these long chain hydrocarbon materials, fatty (aliphatic) alcohols are preferred. The oral dosage form can contain up to about 60% by weight of at least one digestible, long chain hydrocarbon.

Further, the sustained-release matrix can contain up to 60% by weight of at least one polyalkylene glycol.

Alternatively, the release-retarding material may comprise polylactic acid, polyglycolic acid, or a co-polymer of lactic and glycolic acid.

Release-modifying agents, which affect the release properties of the release-retarding material, may optionally be used. The release-modifying agent may, for example, function as a pore-former. The pore former can be organic or inorganic, and include materials that can be dissolved, extracted or leached from the coating in the environment of use. The pore-former can comprise one or more hydrophilic polymers, such as hydroxypropylmethylcellulose, hydroxypropylcellulose, polycarbonates comprised of linear polyesters of carbonic acid in which carbonate groups reoccur in the polymer chain, and combinations comprising one or more of the foregoing release-modifying agents. Alternatively, the pore former may be a small molecule such as lactose, or metal stearates, and combinations comprising one or more of the foregoing release-modifying agents.

The release-retarding material can also optionally include other additives such as an erosion-promoting agent (e.g., starch and gums); and/or a semi-permeable polymer. In addition to the above ingredients, a sustained-release dosage form may also contain suitable quantities of other materials, e.g., diluents, lubricants, binders, granulating aids, colorants, flavorants and glidants that are conventional in the pharmaceutical art. The release-retarding material can also include an exit means comprising at least one passageway, orifice, or the like. The passageway can have any shape, such as round, triangular, square, elliptical, irregular, etc.

The sustained-release dosage form comprising atomoxetine and a release-retarding material may be prepared by a suitable technique for preparing atomoxetine dosage forms as described in detail below. The atomoxetine and release-retarding material may, for example, be prepared by wet granulation techniques, melt extrusion techniques, etc. To obtain a sustained-release dosage form, it may be advantageous to incorporate an additional hydrophobic material.

Atomoxetine in sustained-release form can include a plurality of substrates comprising the active ingredient, which substrates are coated with a sustained-release coating comprising a release-retarding material. The sustained-release preparations may thus be made in conjunction with a multiparticulate system, such as beads, ion-exchange resin beads, spheroids, microspheres, seeds, pellets, granules, and other multiparticulate systems in order to obtain a desired sustained-release of atomoxetine. The multiparticulate system can be presented in a capsule or other suitable unit dosage form.

In certain cases, more than one multiparticulate system can be used, each exhibiting different characteristics, such as pH dependence of release, time for release in various media (e.g., acid, base, simulated intestinal fluid), release *in vivo,* size, and composition.

In some cases, a spheronizing agent, together with the active ingredient can be spheronized to form spheroids. Microcrystalline cellulose and hydrous lactose impalpable are examples of such agents. Additionally (or alternatively), the spheroids can contain a water insoluble polymer, preferably an acrylic polymer, an acrylic copolymer, such as a methacrylic acid-ethyl acrylate copolymer, or ethyl cellulose. In this formulation, the sustained-release coating will generally include a water insoluble material such as a wax, either alone or in admixture with a fatty alcohol, or shellac or zein.

Spheroids or beads, coated with an active ingredient can be prepared, for example, by dissolving or dispersing the active ingredient in a solvent and then spraying the solution onto a substrate, for example, sugar spheres NF21, 18/20 mesh, using a Wurster insert. Optionally, additional ingredients are also added prior to coating the beads in order to assist the active ingredient binding to the substrates, and/or to color the resulting beads, etc. The resulting substrate-active material may optionally be overcoated with a barrier material, to separate the atomoxetine from the next coat of material, e.g., release-retarding material. Preferably, the barrier material is a material comprising hydroxypropyl methylcellulose. However, any film-former known in the art may be used. Preferably, the barrier material does not affect the dissolution rate of the final product.

To obtain a sustained-release of atomoxetine in a manner sufficient to provide an anti-psychotic effect for sustained durations, the substrate comprising atomoxetine can be coated with an amount of release-retarding material sufficient to obtain a weight gain level from about 2 to about 30%, although the coating can comprise a be greater or lesser weight percent depending upon the physical properties of atomoxetine or its particular salt and the desired release rate, among other things. Moreover, there can be more than one release-retarding material used in the coating, as well as various other pharmaceutical excipients.

The release-retarding material may thus be in the form of a film coating comprising a dispersion of a hydrophobic polymer. Solvents typically used for application of the release-retarding coating include pharmaceutically acceptable solvents, such as water, methanol, ethanol, methylene chloride, and combinations comprising one or more of the foregoing solvents.

In addition, the sustained-release profile of atomoxetine release in the formulations (either *in vivo* or *in vitro*) can be altered, for example, by using more than one release-retarding material, varying the thickness of the release-retarding material, changing the particular release-retarding material used, altering the relative amounts of release-retarding material, altering the manner in which the plasticizer is added (e.g., when the sustained-release coating is derived from an aqueous dispersion of hydrophobic polymer), by varying the amount of plasticizer relative to retardant material, by the inclusion of additional ingredients or excipients, by altering the method of manufacture, etc.

In addition to or instead of being present in a matrix, the release-retarding agent can be in the form of a coating. Optionally, the dosage forms can be coated, or a gelatin capsule can be further coated, with a sustained-release coating such as the sustained-release coatings described herein. Such coatings are particularly useful when the subunit comprises atomoxetine in releasable form, but not in sustained-release form. The coatings may include a sufficient amount of a hydrophobic material to obtain increase the weight of the dosage form about 2 to about 30 percent, although the coating can increase the weight of the dosage form by a larger percent depending on the physical properties atomoxetine or the particular atomoxetine salt utilized and the desired release rate, among other things.

The sustained-release formulations preferably release atomoxetine slowly, e.g., when ingested and exposed to gastric fluids, and then to intestinal fluids. The sustained-release profile of the formulations can be altered, for example, by varying the amount of retardant, e.g., hydrophobic material, by varying the amount of plasticizer relative to hydrophobic material, by the inclusion of additional ingredients or excipients, by altering the method of manufacture, etc.

### DELAYED-RELEASE DOSAGE FORMS

Delayed-release dosage forms provide a time-delay before significant plasma levels of atomoxetine are achieved. A delayed-release formulation of atomoxetine can avoid an initial burst of atomoxetine (i.e. "dose dumping"), or can be formulated so that release of atomoxetine in the stomach is avoided and absorption is effected in the small intestine.

Delayed-release tablets may comprise a core, a first coating and optionally a second coating. The core may include atomoxetine, and excipients, notably a lubricant, and a binder and/or a filler, and optionally a glidant as well as other excipients.

Examples of suitable lubricants include stearic acid, magnesium stearate, glyceryl behenate, talc, mineral oil (in PEG), etc. Examples of suitable binders include water-soluble polymer, such as modified starch, gelatin, polyvinylpyrrolidone, polyvinyl alcohol, etc. Examples of suitable fillers include lactose, microcrystalline cellulose, etc. An example of a glidant is silicon dioxide (AEROSIL, Degussa).

The core may contain, by dry weight from about 10 to about 98% atomoxetine or a salt thereof, from about 0.5 to about 10% lubricant, and from about 90 % to about 1.5% binder or filler.

In certain instances the core may contain, by dry weight, about 70 to about 98% atomoxetine or a salt thereof, about 0.5 to about 10% lubricant, and about 2 to about 20% binder or filler.

The first coating may be, for example, a semi-permeable coating to achieve delayed-release of atomoxetine. The first coating may comprise a water-insoluble film-forming polymer, together with a plasticizer and a water-soluble polymer. The water-insoluble film-forming polymer can be a cellulose ether, such as ethylcellulose, a cellulose ester, such as cellulose acetate, palyvinylalcohol, etc. A suitable film-forming polymer is ethylcellulose (available from Dow Chemical under the trade name ETHOCEL). Other excipients can optionally also be present in the first coating, as for example acrylic acid derivatives (such and EUDRAGIT, Rohm Pharma), pigments, etc.

The first coating may contain from about 20 to about 85% water-insoluble film-forming polymer (e.g. ethylcellulose), about 10 to about 75% water-soluble polymer (e.g. polyvinylpyrrolidone), and about 5 to about 30% plasticizer. The relative proportions of ingredients, notably the ratio of water-insoluble film-forming polymer to water-soluble polymer, can be varied depending on the release profile to be obtained (where a more delayed-release is generally obtained with a higher amount of water-insoluble film-forming polymer).

The weight ratio of first coating to tablet core can be about 1:30 to about 3:10, preferably about 1:10.

The optional second coating may be designed to protect the coated tablet core from coming into contact with gastric juice, thereby preventing a food effect. The second coating may comprise an enteric polymer of the methacrylic type and optionally a plasticizer. The second coating can contain, by weight, about 40 to about 95% enteric polymer (e.g. EUDRAGIT L30D-55, Degussa, Piscataway, NJ) and about 5 to about 60% plasticizer (e.g. triethyl citrate, polyethylene glycol). The relative proportions of ingredients, notably the ratio of methacrylic polymer to plasticizer can be varied according to methods known to those of skill in the art of pharmaceutical formulation.

A process for preparing a delayed-release dosage form of atomoxetine comprises manufacturing a core by, for example, wet or dry granulation techniques. Alternatively, atomoxetine and lubricant may be mixed in a granulator and heated to the melting point of the lubricant to form granules. This mixture can then be mixed with a suitable filler and compressed into tablets. Alternatively, atomoxetine and a lubricant (e.g. mineral oil in PEG) may be mixed in a granulator, e.g. a fluidized bed granulator and then into tablets. Tablets may be formed by standard techniques, e.g. on a (rotary) press (for example KILIAN) fitted with suitable punches. The resulting tablets are hereinafter referred as tablet cores.

The coating process can be as follows. Ethylcellulose and polyethylene glycol (e.g. PEG 1450) are dissolved in a solvent such as ethanol; polyvinylpyrrolidone is then added. The resulting solution is sprayed onto the tablet cores, using a coating pan or a fluidized bed apparatus.

The process for applying the second coating can be as follows. Triethyl citrate and polyethylene glycol (e.g. PEG 1450) are dissolved in a solvent such as water; methacrylic polymer dispersion is then added. If present, silicon dioxide can be added as a suspension. The resulting solution is sprayed onto the coated tablet cores, using a coating pan or a fluidized bed apparatus.

The weight ratio of the second coating to coated tablet core is about 1:30 to about 3:10, preferably about 1:10.

An exemplary delayed-release dosage form comprises a core containing atomoxetine, polyvinylalcohol and glyceryl behenate; a first coating of ethylcellulose, polyvinylpyrrolidone and polyethylene glycol, and a second coating of methacrylic acid co-polymer type C, triethyl citrate, polyethylene glycol, and optionally containing silicon dioxide.

### PULSED-RELEASE DOSAGE FORMS

A pulsed-released formulation can contain a combination of immediate-release, sustained-release, and/or delayed-release formulations in the same dosage form. For example, a pulsed-released dosage form of atomoxetine my provide an increase in plasma concentration shortly after AM administration following by a second maxima in plasma concentration several hours later, preferably in the late afternoon.

An exemplary pulsed-release dosage form may provide at least a part of the dose with a pulsed delayed-release of the drug and another part of the formulation with rapid or immediate-release. The immediate and pulsed delayed-release of the drug can be achieved according to different principles, such as by single dose layered pellets or tablets, by multiple dose layered pellets or tablets, or by two or more different fractions of single or multiple dose layered pellets or tablets, optionally in combination with pellets or tablets having instant-release. Multiple dose layered pellets may be filled into a capsule or together with tablet excipients compressed into a multiple unit tablet. Alternatively, a multiple dose layered tablet may be prepared.

Single dose layered pellets or tablets giving one single delayed-release pulse of the drug may be prepared. The single dose layered pellets or tablets may comprise a core material, optionally layered on a seed/sphere, the core material comprising atomoxetine together with a water swellable substance; a surrounding lag time controlling layer, and an outer coating layer positioned to cover the lag time controlling layer. Alternatively, the layered pellets or tablets may comprise a core material comprising atomoxetine; a surrounding layer comprising a water swellable substance; a surrounding lag time controlling layer; and an outer coating layer positioned to cover the lag time controlling layer.

Multiple dose layered pellets or tablets giving two or more delayed-release pulses of the drug may be prepared comprising a core material, optionally layered on a seed/sphere comprising atomoxetine and a water swellable substance, a surrounding lag time controlling layer, a layer comprising atomoxetine optionally together with a water swellable substance; optionally a separating layer which is water-soluble or in water rapidly disintegrating; and an outer coating layer. Alternatively, a multiple dose layered pellet or tablet may comprise a core material, optionally layered on a seed/sphere, comprising atomoxetine; a surrounding layer comprising a water swellable substance; a surrounding lag time controlling layer; a layer comprising atomoxetine; optionally a separating layer; and an outer coating layer.

The core material comprising atomoxetine can be prepared either by layering the drug onto a seed, such as for instance a sugar sphere seed, or by extrusion/spheronization of a mixture comprising the drug and pharmaceutically acceptable excipients. It is also possible to prepare the core material by using tablet technology, i.e. compression of drug granules and optionally pharmaceutically acceptable excipients into a tablet core. For pellets of the two types, i.e. single or multiple dose pellets, which have the drug deposited onto a seed/sphere by layering, it is also possible to have an optional layer comprising a water swellable substance beneath the drug containing layer in the core material. The seeds/spheres can be water insoluble and comprise different oxides, celluloses, organic polymers and other materials, alone or in mixtures, or be water soluble and comprise different inorganic salts, sugars and other materials, alone or in mixtures. Further, the seeds/spheres may comprise atomoxetine in the form of crystals, agglomerates, compacts etc. The size of the seeds may be about 0.1 to about 2 mm. Before the seeds are layered, the active substance may be mixed with further components to obtain preferred handling and processing properties and a suitable concentration of the active substance in the final mixture.

Optionally an osmotic agent is placed in the core material. Such an osmotic agent is water soluble and will provide an osmotic pressure in the tablet. Examples of osmotic agents are magnesium sulfate, sodium chloride, lithium chloride, potassium chloride, potassium sulfate, sodium carbonate, lithium sulfate, calcium bicarbonate, sodium sulfate, calcium lactate, urea, magnesium succinate, sucrose or mixtures thereof.

Water swellable substances suitable for the dosage forms are compounds, which are able to expand when they are exposed to an aqueous solution, such as gastro-intestinal fluid. One or more water swellable substances may be present in the core material together with atomoxetine and optionally pharmaceutically acceptable excipient(s). Alternatively, one or more water swellable substances are included in a swelling layer applied onto the core material. As a further alternative, swellable substances(s) they may also be present in an optional swelling layer situated beneath the drug containing layer, if a layered seed or sphere is used as the core material.

The amount of water swellable substance(s) in the swelling layer or in the core to material ratio is chosen in such a way that the core material or the swelling layer in contact with an aqueous solution, such as gastro-intestinal fluid, will expand to such a degree that the surrounding lag-time controlling membrane ruptures. A water swellable substance may also be included in the drug comprising layer of the multiple layered pellets or tablets to increase dissolution rate of the drug fraction.

Suitable substances which can be used as water swellable substances include, for example, low-substituted hydroxypropyl cellulose, e.g. L-HPC; cross-linked polyvinyl pyrrolidone (PVP-XL), e.g. KOLLIDON CL and POLYPLASDONE XL; cross-linked sodium carboxymethylcellulose, e.g. AC-DI- SOL, PRIMELLOSE; sodium starch glycolate, e.g. PRIMOJEL; sodium carboxymethylcellulose, e.g. NYMCEL. ZSB10; sodium carboxymethyl starch, e.g. EXPLOTAB; ion-exchange resins, e.g. DOWEX or AMBERLITE; microcrystalline cellulose, e.g. AVICEL; starches and pregelatinized starch, e.g. STARCH 1500, SEPSITAB ST200; formalin-casein, e.g. PLAS-VITA, and combinations comprising one or more of the foregoing water swellable substances.

The lag time controlling layer may be a semipermeable membrane comprising a water resistant polymer that is semipermeable for an aqueous solution, such as gastro-intestinal fluid. Suitable polymers are cellulose acetate, ethylcellulose, polyvinyl acetate, cellulose acetate butyrate, cellulose acetate propionate, acrylic acid copolymers, such as EUDRAGIT RS or RL, and combinations comprising one or more of the foregoing polymers. The polymer may optionally comprise pore forming agents, such as a water soluble substance, e.g. sucrose, salt; or a water soluble polymer e.g., polyethylene glycol. Also pharmaceutically acceptable excipients such as fillers and membrane strength influencing agents such as talc, aerosil, or sodium aluminium silicate may be included.

There is at least one lag time controlling layer present in the pulsed-release dosage form. The lag time controlling layer positioned nearest the inner core material is constructed in the form of a semipermeable membrane that will disrupt after a desired time after ingestion. A desired lag time may be adjusted by the composition and thickness of the layer. The amount of substances forming such a disrupting semipermeable membrane, i.e. a lag time controlling layer, may be about 0.5 to about 25 % of the weight of the core material including swelling substances or a swelling layer, preferably about 2 to about 20% by weight.

The lag time controlling layer may comprise a mixture of ethylcellulose and talc. In some embodiments the mixture contains about10% to 80% w/w of talc.

Before applying the outer coating layer onto the layered pellets or tablets, they may optionally be covered with one or more separating layers comprising excipients. This separating layer separates the composition of the layered pellets or tablets from the outer enteric coating layer. Suitable materials for the optional separating layer are pharmaceutically acceptable compounds such as, for instance, sugar, polyethylene glycol, polyvinyl pyrrolidone, polyvinyl alcohol, polyvinyl acetate, hydroxypropyl cellulose, methylcellulose, ethylcellulose, hydroxypropyl methylcellulose, carboxymethylcellulose sodium and others, and combinations comprising one or more of the foregoing materials substances and other additives may also be included into the separating layer.

When the optional separating layer is applied to the layered pellets or tablets it may constitute a variable thickness. The maximum thickness of the optional separating layer is normally only limited by processing conditions. The separating layer may serve as a diffusion barrier and may act as a pH-buffering zone. The optional separating layer may improve the chemical stability of the active substance and/or the physical properties of the dosage form.

Finally the layered pellets or tablets are covered by one or more outer coating layers by using a suitable coating technique. The outer coating layer material may be dispersed or dissolved in either water or in suitable organic solvents. Suitable methacrylic acid copolymers, cellulose acetate phthalate, hydroxypropyl methylcellulose phthalate, hydroxypropyl methylcellulose acetate succinate, polyvinyl acetate phthalate, cellulose acetate trimellitate, carboxymethyl ethylcellulose, shellac or other suitable coating layer polymer(s).

The applied polymer containing layers, and specially the outer coating layers may also contain pharmaceutically acceptable plasticizers to obtain desired mechanical properties.

### BIOEQUIVALENCE

In some instances, the formulations described herein preferably exhibit bioequivalence to the marketed drug product, for example STRATTERA. Bioequivalence is defined as "the absence of a significant difference in the rate and extent to which the active ingredient or active moiety in pharmaceutical equivalents or pharmaceutical alternatives becomes available at the site of drug action when administered at the same molar dose under similar conditions in an appropriately designed study" (21 CFR 320.1). As used herein, bioequivalence of a dosage form is determined according to the Federal Drug Administration's (FDA) guidelines and criteria, including "GUIDANCE FOR INDUSTRY BIOAVAILABILITY AND BIOEQUVALENCE STUDIES FOR ORALLY ADMINISTERED DRUG PRODUCTS-GENERAL CONSIDERATIONS" available from the U.S. Department of Health and Human Services (DHHS), Food and Drug Administration (FDA), Center for Drug Evaluation and Research (CDER) March 2003 Revision 1; and "GUIDANCE FOR INDUSTRY STATISTICAL APPROACHES TO ESTABLISHING BIOEQUIVALENCE" DHHS, FDA, CDER, January 2001; and "STATISTICAL PROCEDURES FOR BIOEQUIVALENCE STUDIES USING A STANDARD TWO-TREATMENT CROSSOVER DESIGN" DHHS, FDA, CDER, July 1992.

Particularly relevant sections of the guidelines include:
Pharmacolcinetic Analysis of Data: Calculation of area under the plasma concentration-time curve to the last quantifiable concentration (AUC₀₋ₜ,) and to infinity (AUCO_{0-∞}), Cₘₐₓ, and Tₘₐₓ should be performed according to standard techniques.

Statistical Analysis of Pharmacokinetic Data: The log transformed AUC and Cₘₐₓ data should be analyzed statistically using analysis of variance. These two parameters for the test product should be shown to be within 80-125% of the reference product using the 90% confidence interval. See also Division of Bioequivalence Guidance Statistical Procedures for Bioequivalence Studies Using a Standard Two-Treatment Crossover Design.

Multiple Dose Studies: At a minimum, the following pharmacokinetic parameters for the substance of interest should be measured in a multiple dose bioequivalence study:
a. Area under the plasma/blood concentration - time curve from time zero to time T over a dosing interval at steady state (AUC_{0-T}), wherein T is the dosing interval.
b. Peak drug concentration (Cₘₐₓ) and the time to peak drug concentration (Tₘₐₓ), obtained directly from the data without interpolation, after the last dose is administered.
c. Drug concentrations at the end of each dosing interval during steady state (Cₘᵢₙ).
d. Average drug concentration at steady state (Cₐᵥ), where Cₐᵥ - AUC_{0-T}/T.
e. Degree of fluctuation (DF) at steady state, where DF = 100% X (Cₘₐₓ - Cₘᵢₙ)/Cav. Evidence of attainment of steady state for the test and reference products should be submitted in the bioequivalence study report.

Statistical Analysis Parametric (normal-theory) general linear model procedures are recommended for the analysis of pharmacokinetic data derived from in vivo bioequivalence studies. An analysis of variance (ANOVA) should be performed on the pharmacokinetic parameters AUC and Cmax using General Linear Models (GLM) procedures of SAS (4) or an equivalent program. Appropriate statistical models pertaining to the design of the bioequivalence study should be employed. For example, for a conventional two-treatment, two-period, two-sequence (2 x 2) randomized crossover study design, the statistical model often includes factors accounting for the following sources of variation:
1. Sequence (sometimes called Group or Order)
2. Subjects, nested in sequences
3. Period (or Phase)
4. Treatment (sometimes called Drug or Formulation)

The sequence effect should be tested using the [subject (sequence)]mean square from the ANOVA as an error term. All other main effects should be tested against the residual error (error mean square) from the ANOVA. The LSMEANS statement should be used to calculate least squares means for treatments. The ESTIMATE statement in SAS should be used to obtain estimates for the adjusted differences between treatment means and the standard error associated with these differences.

The two one-sided hypotheses at the α = 0.05 level of significance should be tested for AUC and Cₘₐₓ by constructing the 90% confidence interval for the ratio between the test and reference averages.

Logarithmic Transformation of Pharmacokinetic Data:
Statistical Assumptions: The assumptions underlying the ANOVA are:
   1. Randomization of samples
   2. Homogeneity of variances
   3. Additivity (linearity) of the statistical model
   4. Independency and normality of residuals

In bioequivalence studies, these assumptions can be interpreted as follows:
1. The subjects chosen for the study should be randomly assigned to the sequences of the study.
2. The variances associated with the two treatments, as well as between the sequence groups, should be equal or at least comparable.
3. The main effects of the statistical model, such as 25 subject, sequence, period and treatment effect for a standard 2 x 2 crossover study, should be additive. There should be no interactions between these effects.
4. The residuals of the model should be independently and normally distributed. In other words, data from bioequivalence studies should have a normal distribution.

If these assumptions are not met, additional steps should be taken prior to the ANOVA including data transformation to improve the fit of the assumptions or use of a nonparametric statistical test in place of ANOVA. However, the normality and constant variance assumptions in the ANOVA model are known to be relatively robust, i.e., small or moderate departure from each (or both) of these assumptions will not have a significant effect on the final result.

### EXEMPLARY FORMULATIONS

The various release properties described above may be achieved in a variety of different ways. Suitable formulations include, for example, wax formulations, press coat formulations, easily administered formulations, osmotic pump dosage forms, etc.

### WAX FORMULATIONS

A wax formulation is a solid dosage form comprising the atomoxetine or a salt thereof, most preferably atomoxetine hydrochloride, in a waxy matrix. The waxy matrix may be prepared by hot melting a suitable wax material and using the melt to granulate atomoxetine material. The matrix material comprises the waxy material and atomoxetine.

The wax material can be, for example, an amorphous wax, an anionic wax, an anionic emulsifying wax, a bleached wax, a carnauba wax, a cetyl esters wax, a beeswax, a castor wax, a cationic emulsifying wax, a cetrimide emulsifying wax, an emulsifying wax, a glyceryl behenate, a microcrystalline wax, a nonionic wax, a nonionic emulsifying wax, a paraffin, a petroleum wax, a spermaceti wax, a white wax, a yellow wax, and combinations comprising one or more of the foregoing waxes. These and other suitable waxes are known to those of skill in the art. A cetyl esters wax, for example, preferably has a molecular weight of about 470 to about 490 and is a mixture containing primarily esters of saturated fatty alcohols and saturated fatty acids. The wax material can comprise a carnauba wax, glyceryl behenates, castor wax, and combinations comprising one or more of the foregoing waxes. When the waxy material contains only carnauba wax and no other waxy material is used, the matrix may be coated with a functional coating. When the waxy material includes glyceryl behenates and carnauba wax, the matrix can be used without a coating, but may have either a cosmetic coating or a functional coating depending on the precise release profile and appearance desired.

The wax material can be used at about 16% to about 35%, preferably about 20% to about 32%, more preferably about 24% to about 31%, and most preferably about 28% to about 29% of the total weight of the matrix material. When a combination of wax is used, e.g., carnauba wax and glyceryl behenate, the component waxes can be used in a suitable ratio. Certain formulations include the wax material component from 100 to about 85 parts carnauba wax and from 0 to about 15 parts glyceryl behenate. In formulations that have a combination of carnauba wax and castor wax, for example, the wax component may have about 100 to about 85 parts carnauba wax and 0 to about 15 parts castor wax. When carnauba wax, glyceryl behenate and castor wax are present, the carnauba wax can comprise at least about 85% of the waxy material and the balance of the waxy material is made up of a combination of glyceryl behenate and castor wax, in a suitable relative proportion.

Optionally, fatty acids and fatty acid soaps can be present in the waxy dosage form. In some cases, the fatty acids and/or fatty acid soaps can replace a portion of the wax or waxes. These optional fatty acids and fatty acid soaps can be those that are generally used in the pharmaceutical industry as tableting lubricants, such as, for example, solid fatty acids (for example fatty acids having from about 16 to about 22 carbon atoms), and the alkaline earth metal salts thereof, particularly the magnesium and calcium salts, and combinations comprising one or more of the foregoing fatty acids. The fatty acid can be, for example, stearic acid. The optional fatty acids and fatty acid soaps, when present, can be used in amounts of up to about 10% of the total weight of the matrix material, or about 2.5% to about 9%, or about 2.7% to about 8.6%, or from about 3% to about 6% of the total weight of the matrix material. An amount of up to about 2% of the total core formulation of the optional fatty acid materials may be used as a blend with the melt granulate. Amounts of at least about 1% may be used in this fashion with the remainder being added to the waxes for melting and granulating atomoxetine.

To prepare the dosage form, the waxes may be melted and used to granulate atomoxetine. The granulate may be allowed to cool and then be milled to a proper size. Advantageously, the granulate is milled to an average particle size of about 75 microns to about 850 microns, preferably about 150 microns to about 425 microns. The milled granulate may be mixed with optional processing aids. The processing aids include, for example, hydrophobic colloidal silicon dioxide (such as CAB-O-SIL M5). Hydrophobic silicon dioxide may be used in amounts of less than or equal to about 0.5%, but individual formulations can be varied as required. The blend of the waxy granulate and the processing aids, if any, may be compressed and then optionally coated.

The wax dosage form can include, for example, compressed coated or uncoated tablets, compressed pellets contained in capsules, or loose powder or powder filled capsules.

Thus in a first instance the disclosure pertains to a solid dosage formulation comprising a matrix, wherein the matrix comprises a pharmaceutically effective amount of atomoxetine or a pharmaceutically acceptable salt thereof; and a wax material. It is preferred that the matrix comprises a pharmaceutically effective amount of atomoxetine hydrochloride.

In certain instances the wax material includes carnauba wax, glyceryl behenate, castor wax, or any combination thereof.

In some instances the matrix in the wax formulation is coated with a coating composition. The coating composition maybe either a functional coating composition.

When the coating composition is a functional coating composition, the coating composition may comprise a water insoluble component; and a water-soluble component.

When the coating composition is a non-functional coating composition, the coating composition may comprise a water-soluble component in the substantial absence of a non-water-permeable component. The non-functional coating composition may additionally comprise pharmaceutically acceptable dyes, pigments, or mixtures thereof.

The wax formulation which comprises a matrix of atomoxetine and a wax material may also further comprise a processing aid. Examples of processing aids are given above.

The wax formulation may also include an additional active agent in the matrix, e.g. the formulation may be a combination of atomoxetine and another active agent used to treat attention deficit disorder.

In certain instances the wax formulation comprising a matrix comprising atomoxetine, and a wax material, is formulated as a sustained-release dosage formulation.

An instance of the disclosure is directed to an atomoxetine wax having a size which is substantially smaller than the size of a same strength dosage form of STRATTERA.

The disclosure also includes a method of making a wax formulation comprising a matrix, the method comprising: hot melting a waxy material to form a melt, granulating atomoxetine hydrochloride with the melt to form a granulate; milling the granulate; and compressing the granulate to form a matrix. This method may further comprise: blending the granulate with a processing aid; prior to compressing the granulate to form a matrix. The method may further comprise coating the matrix with a functional and/ or a non-functional coating.

The disclosure also pertains to the wax formulation made by the resulting from the method of the disclosure.

Examples of atomoxetine wax formulations, and methods of preparing atomoxetine wax formulations are given below, in Examples 1 to 4.

### PRESS COAT FORMULATIONS

A press coat oral dosage form of atomoxetine or a salt thereof comprises a core composition and a coating composition press-coated on the core. The core composition comprises a waxy material and atomoxetine or its salt and the coating composition comprises a hydrophilic polymer and optionally atomoxetine or its salt. Preferably the atomoxetine is in the form of atomoxetine hydrochloride.

The core composition of the press coat dosage from comprises a waxy material. The waxy material can be a hydrophobic waxy material to provide controlled-release of atomoxetine. In pharmaceutical products, for example, such waxy materials may be, for example, carnauba wax, tribehenin, fatty alcohols (particularly those having 12-24 carbon atoms, such as lauryl alcohol, myristyl alcohol, stearyl alcohol, palmityl alcohol, etc.), fatty acids (particularly those having 12-24 carbon atoms, such as lauric acid, myristic acid, stearic acid, palmitic acid, etc), polyethylenes, castor wax, C₁₆₋₃₀ fatty acid triglycerides, beeswax, and combinations comprising one or more of the foregoing waxes.

The coating composition comprises a hydrophilic polymer. The hydrophilic polymer can provide for controlled-release of atomoxetine. The hydrophilic polymer providing controlled-release may be a film forming polymer, such as a hydrophilic cellulose polymer. Such a hydrophilic cellulose polymer may be hydroxyalkyl cellulose polymer, for example hydroxyethylcellulose (HEC), hydroxypropyl cellulose (HPC), hydroxypropylmethylcellulose (HPMC), hydroxypropylethylcellulose (HPEC), hydroxypropylpropylcellulose (HPPC), hydroxypropylbutylcellulose (HPBC), and combinations comprising one or more of the foregoing polymers.

Both the core composition and the coating composition may further include a filler, such as a water insoluble filler, water soluble filler, and mixtures thereof. A water-insoluble filler can be talc or a calcium salt such as a calcium phosphate, e.g., a dicalcium phosphate. The filler in the coating composition can be the same or different as the filler in the core composition, if any. For example, the core composition can include a water-soluble filler while the coating composition can include a water-insoluble filler.

Optional excipients can also be present in the core composition and the coating composition, including lubricants (such as talc and magnesium stearate), glidants (such as fumed or colloidal silica), pH modifiers (such as acids, bases and buffer systems), pharmaceutically useful processing aids, and combinations comprising one or more of the foregoing excipients. Excipients in the coating compositon can be the same or different as those in the core composition.

In formation of the dosage form, the core composition can be press-coated with the press-coat composition coating formulation to form a tablet. The tablet can be further coated with optional additional coatings. The additional coatings can be pH-dependent or pH-independent, aesthetic or functional, and can include atomoxetine in immediate or controlled-release. The optional additional coating can include atomoxetine or salt thereof or a different active agent than is contained in the core composition and the coating composition. The additional coating may, for example, include an immediate-release dosage form of atomoxetine hydrochloride.

In forming the dosage form, the core composition components (atomoxetine, wax, and optional excipients) are blended together and compressed into suitable cores. The blending can take place in a suitable order of addition. The cores may be blended by starting with the smallest volume component and then successively adding the larger volume components. Another process is to melt the wax and to blend atomoxetine and optional excipients into the melted wax. Alternatively, atomoxetine, wax and optional excipients can be blended together and then subjected to a temperature at which the wax will melt. Once cooled, the solidified mass can be milled into granules for compaction into cores.

The press coat formulations may have substantially zero order, first order, and second order release rate profiles by adjusting the amount of atomoxetine in the core composition and the coating composition. The ratio of atomoxetine in the core compositon (Core_{AA}) to atomoxetine in the coating composition (Coat_{AA}) may be about 1:99 to about 99:1, more preferably about 95:5 to about 5:99, most preferably about 9:1 to about 1:9. For the highly soluble active agents, including atomoxetine hydrochloride and other highly soluble active agents that may be used in combination with atomoxetine hydrochloride, a CoreAA:Coat_{AA} of about 3:4 to about 5:3 is can provide a substantially zero order release rate, a Core_{AA}:Coat_{AA} of less than about 3:4 can provide a substantially first order release rate, and a Core_{AA}:Coat_{AA} of greater than about 5:3 can provide a substantially second order release rate.

The press coat formulations can be press-coated tablets containing 5 mg, 10 mg, 18 mg, 25 mg, 40 mg or 60 mg atomoxetine (particularly in the form of atomoxetine hydrochloride). One exemplary press coat atomoxetine hydrochloride formulation comprises 5 mg atomoxetine in an immediate-release coating composition and 55 mg atomoxetine between the core composition and the coating composition. In this example, the 0-4 hour cumulative release of atomoxetine in 0.1 N hydrochloric acid may be at least about 25% to about 50%, more preferably about 35% to about 40%, of the loaded dose, and the 0-12 hour cumulative release of atomoxetine in 0.1 N hydrochloric acid may be at least about 75%, more preferably at least about 85%, of the dosage form dose. In another example, a 60 mg atomoxetine hydrochloride formulation comprises a 3:2:1 (core:press coat:immediate-release coat) ratio, e.g., a core composition comprising 30 mg of atomoxetine hydrochloride, a coating composition comprising 20 mg of atomoxetine hydrochloride, and an immediate-release loading dose comprising 10 mg of atomoxetine hydrochloride.

Thus an embodiment of the invention pertains to a press-coat dosage form comprising a core composition comprising an active agent, which active agent is atomoxetine or a pharmaceutically acceptable salt thereof, a waxy material; and a coating composition comprising a hydrophilic polymer, wherein the coating composition is press-coated onto the core composition. Preferably the active agent is atomoxetine hydrochloride.

The invention also pertains to a press-coat dosage form comprising a core composition comprising an active agent, which is atomoxetine or a pharmaceutically acceptable salt thereof, a waxy material; and a coating composition comprising a hydrophilic polymer, wherein the coating composition which also contains atomoxetine or a pharmaceutically acceptable salt thereof, is press-coated onto the core. Preferably the atomoxetine in the coating composition is in the form of atomoxetine hydrochloride.

In some embodiments of the invention the ratio of atomoxetine in the core composition of the press-coat dosage form to atomoxetine in the coating composition is about 1:99 to about 99:1. Other embodiments of the invention pertain to an atomoxetine press-coat formulation in which the ratio of atomoxetine in the core composition to atomoxetine in the coating composition is greater than about 5:3.

In certain embodiments the waxy material of the press-coat dosage form core is carnauba wax, tribehenin, fatty alcohols, lauryl alcohol, myristyl alcohol, stearyl alcohol, palmityl alcohol, fatty acids, lauric acid, myristic acid, stearic acid, palmitic acid, polyethylenes, castor wax, C₁₆₋₃₀ fatty acid triglycerides, beeswax, or any combination thereof. In some embodiments of the invention the hydrophilic polymer in the coating composition of the atomoxetine press-coat dosage form comprises a hydrophilic cellulose polymer.

An embodiment of the invention pertains to an atomoxetine press-coat dosage comprising a core composition comprising an active agent, which is atomoxetine hydrochloride and wherein the hydrophilic cellulose polymer is hydroxypropylmethyl cellulose (HPMC).

Another embodiment of the invention pertains to an atomoxetine press-coat dosage form comprising a core composition comprising an active agent which is atomoxetine or atomoxetine hydrochloride, carnauba wax; and a coating composition comprising atomoxetine or atomoxetine hydrochloride and hydroxypropylmethyl cellulose (HPMC), wherein the coating composition is press-coated onto the core.

Yet another embodiment of the invention pertains to an atomoxetine press-coat dosage form comprising a core composition comprising atomoxetine hydrochloride and carnauba wax, a coating composition comprising atomoxetine hydrochloride and hydroxypropylmethyl cellulose (HPMC), wherein the coating composition is press-coated onto the core, and an additional coating composition comprising atomoxetine hydrochloride. In some embodiments of the invention the additional coating composition is an immediate-release coating composition.

In some embodiments of the invention the press-coat dosage form contains atomoxetine in the core composition and atomoxetine in the coating composition present in amounts effective to provide a substantially zero order release profile.

In other embodiments of the invention the atomoxetine in the core composition and atomoxetine in the coating composition are present in amounts effective to provide a substantially first order release profile.

In still other embodiments of the invention the atomoxetine in the core composition and atomoxetine in the coating composition are present in amounts effective to provide a substantially second order release profile.

The invention also provides a method for preparing a press-coat dosage form, the method comprising providing a core composition comprising atomoxetine or a pharmaceutically acceptable salt thereof and a waxy material, providing a coating composition comprising atomoxetine or a pharmaceutically acceptable salt thereof and a hydrophilic polymer, and press-coating the coating composition onto the core composition to provide the press-coat dosage form.

Examples of atomoxetine press-coated formulations, and methods of preparing atomoxetine press formulations are given below, in Examples 5 to 9.

### EASILY ADMINISTERED DOSAGE FORMS

The disclosure provides easily administerable dosage forms for administration to patients who have difficulty swallowing, to reduce the risk of choking upon administration, and to improve patient compliance. Such dosage forms are particularly useful for administration to elderly and juvenile patients. The disclosure provides, for example, sprinlde dosage forms, taste-masked liquid dosage forms and fast-dissolve dosage forms.

### CHEWABLE TABLETS

Another solid dosage form is a chewable tablet containing atomoxetine. A chewable tablet comprises a chewable base and optionally a sweetener. The chewable base comprises an excipient such as, for example, mannitol, sorbitol, lactose, or a combination comprising one or more of the foregoing excipients. The optional sweetener used in the chewable dosage form may be, for example, digestible sugars, sucrose, liquid glucose, sorbitol, dextrose, isomalt, liquid maltitol, aspartame, lactose, and combinations comprising one ore more of the foregoing sweeteners. In certain cases, the chewable base and the sweetener may be the same component. The chewable base and optional sweetener may comprise about 50 % to about 90 % by weight of the total weight of the dosage form.

The chewable dosage form may additionally contain preservatives, agents that prevent adhesion to oral cavity and crystallization of sugars, flavoring agents, souring agents, coloring agents, and combinations comprising one or more of the foregoing agents. Glycerin, lecithin, hydrogenated palm oil or glyceryl monostearate may be used as a protecting agent of crystallization of the sugars in an amount of about 0.04 to about 2.0 weight % of the total weight of the ingredients, to prevent adhesion to oral cavity and improve the soft property of the products. Additionally, isomalt or liquid maltitol may be used to enhance the chewing properties of the chewable dosage form.

A method of making a chewable dosage form of atomoxetine is similar to the method used to make soft confectionary. The method generally involves the formation of a boiled sugar-digestible sugar blend to which is added a frappe mixture. The boiled sugar-digestible sugar blend may be prepared from sugar and digestible sugar blended in parts by weight ratio of 90: 10 to 10: 90. This blend may be heated to temperatures above 250 °F to remove water and to form a molten mass. The frappe mixture may be prepared from gelatin, egg albumen, milk proteins such as casein, and vegetable proteins such as soy protein, and the like which are added to a gelatin solution and rapidly mixed at ambient temperature to form an aerated sponge like mass. The frappe mixture is then added to the molten candy base and mixed until homogenous at temperatures between 150 °F to about 250 °F. A wax matrix containing atomoxetine may then be added as the temperature of the mix is lowered to about 120 °F to about 194 °F, whereupon additional ingredients such as flavors, colorants, and preservatives may be added. The formulation is further cooled and formed to pieces of desired dimensions.

### FAST DISSOLVING DOSAGE FORMS

Another oral dosage form is a non-chewable, fast dissolving dosage form of atomoxetine. These dosage forms can be made by methods known to those of ordinary skill in the art of pharmaceutical formulations. For example, Cima Labs has produced oral dosage forms including microparticles and effervescents which rapidly disintegrate in the mouth and provide adequate taste-masking. Cima Labs has also produced a rapidly dissolving dosage form containing atomoxetine or salt thereof and a matrix that includes a nondirect compression filler and a lubricant. Zydis (ZYPREXA) is produced by Eli Lilly as a rapidly dissolvable, freeze-dried, sugar matrix formulated as a rapidly dissolving tablet. Fast-dissolving dosage forms are disclosed in U.S. Pat. No. 5,178,878 and U.S. Pat. No. 6,221,392, which are hereby incorporated by reference for their teachings regarding fast-dissolve dosage forms.

An exemplary fast dissolve dosage form includes a mixture incorporating a water and/or saliva activated effervescent, disintegration agent, and microparticles. The microparticles incorporate atomoxetine or its salt together with a protective material substantially encompassing atomoxetine. The term "substantially encompassing" as used in this context means that the protective material substantially shields atomoxetine from contact with the environment outside of the microparticle. Thus, each microparticle may incorporate a discrete mass of atomoxetine covered by a coating of the protective material, in which case the microparticle can be referred to as a "microcapsule". Alternatively or additionally, each microparticle may have atomoxetine dispersed or dissolved in a matrix of the protective material. The mixture including the microparticles and effervescent agent may be present as a tablet of a size and shape adapted for direct oral administration to a patient. The tablet is substantially completely disintegrable upon exposure to water and/or saliva. The effervescent disintegration agent is present in an amount effective to aid in disintegration of the tablet, and to provide a distinct sensation of effervescence when the tablet is placed in the mouth of a patient.

The effervescent sensation is not only pleasant to the patient but also tends to stimulate saliva production, thereby providing additional water to aid in further effervescent action. Thus, once the tablet is placed in the patient's mouth, it will disintegrate rapidly and substantially completely without any voluntary action by the patient. Even if the patient does not chew the tablet, disintegration will proceed rapidly. Upon disintegration of the tablet, the microparticles are released and can be swallowed as a slurry or suspension of the microparticles. The microparticles are thus transferred to the patient's stomach for dissolution in the digestive tract and systemic distribution of the pharmaceutical ingredient.

The term effervescent disintegration agent(s) includes compounds that evolve gas. Effervescent agents may evolve gas by means of chemical reactions that take place upon exposure of the effervescent disintegration agent to water and/or to saliva in the mouth. The bubble or gas generating reaction is most often the result of the reaction of a soluble acid source and an alkali metal carbonate or carbonate source. The reaction of these two general classes of compounds produces carbon dioxide gas upon contact with water included in saliva.

Such water activated materials should be kept in a generally anhydrous state with little or no absorbed moisture or in a stable hydrated form since exposure to water will prematurely disintegrate the tablet. The acid sources or acid may be any which are safe for human consumption and may generally include food acids, acid anhydrides and acid salts. Food acids include citric acid, tartaric acid, malic acid, fumaric acid, adipic acid, and succinic acids, etc. Because these acids are directly ingested, their overall solubility in water is less important than it would be if the effervescent tablet formulations of the present invention were intended to be dissolved in a glass of water. Acid anhydrides and acid of the above-described acids may also be used. Acid salts may include sodium, dihydrogen phosphate, disodium dihydrogen pyrophosphate, acid citrate salts and sodium acid sulfite.

Carbonate sources include dry solid carbonate and bicarbonate salts such as sodium bicarbonate, sodium carbonate, potassium bicarbonate and potassium carbonate, magnesium carbonate and sodium sesquicarbonate, sodium glycine carbonate, L-lysine carbonate, arginine carbonate, amorphous calcium carbonate, and combinations comprising one or more of the foregoing carbonates.

The effervescent disintegration agent is not always based upon a reaction which forms carbon dioxide. Reactants which evolve oxygen or other gasses which are safe for human patients, including pediatric patients, are also within the scope of the invention. Where the effervescent agent includes two mutually reactive components, such as an acid source and a carbonate source, it is preferred that both components react substantially completely. Therefore, an equivalent ratio of components which provides for equal equivalents is preferred. For example, if the acid used is diprotic, then either twice the amount of a mono-reactive carbonate base, or an equal amount of a di-reactive base should be used for complete neutralization to be realized. However, the amount of either acid.or carbonate source may exceed the amount of the other component. This may be useful to enhance taste and/or performance of a tablet containing an overage of either component. In this case, it is acceptable that the additional amount of either component may remain unreacted.

In general, the amount of effervescent disintegration agent useful for the formation of tablets is about 5% to about 50% by weight of the final composition, preferably about 15% and about 30% by weight thereof, and most preferably about 20 and about 25% by weight of the total composition.

More specifically, tablets according to the present disclosure should contain an amount of effervescent disintegration agent effective to aid in the rapid and complete disintegration of the tablet when orally administered. By "rapid", it is understood that the tablets should disintegrate in the mouth of a patient in less than 10 minutes, and or in certain instances between about 30 seconds and about 7 minutes, preferably tablets should dissolve in the mouth between about 30 seconds and about 5 minutes. Disintegration time in the mouth can be measured by observing the disintegration time of the tablet in water at about 37 °C. The tablet is immersed in the water without forcible agitation. The disintegration time is the time from immersion for substantially complete dispersion of the tablet as determined by visual observation. As used herein, the term "complete disintegration" of the tablet does not require dissolution or disintegration of the microcapsules or other discrete inclusions.

In certain fast-dissolving dosage forms described herein Atomoxetine (atomoxetine or its salt) is present in microparticles. Each microparticle incorporates atomoxetine in conjunction with a protective material. The microparticle may be provided as a microcapsule or as a matrix-type microparticle. Microcapsules may incorporate a discrete mass of atomoxetine surrounded by a discrete, separately observable coating of the protective material. Conversely, in a matrix-type particle, atomoxetine is dissolved, suspended or otherwise dispersed throughout the protective material. Certain microparticles may include attributes of both microcapsules and matrix-type particle. For example, a microparticle may incorporate a core incorporating a dispersion of atomoxetine in a first protective material and a coating of a second protective material, which may be the same as or different from the first protective material surrounding the core. Alternatively, a microparticle may incorporate a core consisting essentially of atomoxetine and a coating incorporating the protective material, the coating itself having some of the pharmaceutical ingredient dispersed within it.

The microparticles may be about 75 and 600 microns mean outside diameter, and more preferably between about 150 and about 500 microns. Microparticles above about 200 microns may be used. Thus, the microparticles may be between about 200 mesh and about 30 mesh U.S. standard size, and more preferably between about 100 mesh and about 35 mesh.

Tablets can be manufactured by well-known tableting procedures. In common tableting processes, the material to be tableted is deposited into a cavity, and one or more punch members are then advanced into the cavity and brought into intimate contact with the material to be pressed, whereupon compressive force is applied. The material is thus forced into conformity with the shape of the punches and the cavity. Hundreds, and even thousands, of tablets per minute can be produced in this fashion.

Another exemplary fast-dissolve dosage form is a hard, compressed, rapidly dissolvable dosage form adapted for direct oral dosing. The dosage form includes atomoxetine, often in the form of a protected particle, and a matrix. The matrix includes a nondirect compression filler and a lubricant, although, it may include other ingredients as well. The dosage form is adapted to rapidly dissolve in the mouth of a patient, yet it has a friability of about 2% or less when tested according to the U.S.P. Generally, the dosage form will also have a hardness of at least about 1.5- 2.0 kP. Not only does the dosage form dissolve quickly, it does so in a way that provides a positive organoleptic sensation to the patient. In particular, the dosage form dissolves with a minimum of unpleasant grit, which is tactilely inconsistent with a positive organoleptic sensation to the patient.

The protective materials may include a polymers conventionally utilized in the formation of microparticles, matrix-type microparticles and microcapsules. Among these are cellulosic materials such as naturally occurring cellulose and synthetic cellulose derivatives; acrylic polymers and vinyl polymers. Other simple polymers include proteinaceous materials such as gelatin, polypeptides and natural and synthetic shellacs and waxes. Protective polymers may also include ethylcellulose, methylcellulose, carboxymethyl cellulose and acrylic resin material sold under the registered trademark EUDRAGIT by Rohm Pharma GmbH of Darmstadt, Germany.

Generally, when a coating is used, the coating may be used at greater than or equal to about 5 percent based on the weight of the resulting particles. More preferable, the coating should constitute at least about 10 percent by weight of the particle. The upper limit of protective coating material used is generally less critical, except that where a rapid release of the active ingredient is desired, the amount of coating material should not be so great that the coating material impedes the,release profile of atomoxetine or pharmaceutical ingredient when ingested. In certain instances it is possible to use a coating which is greater than 100 percent of the weight of the core, thereby providing a relatively thick coating.

The filler comprises nondirect compression fillers. Exemplary fillers include, for example, nondirect compression sugars and sugar alcohols, which meet the specifications discussed above. Such sugars and sugar alcohols include, without limitation, dextrose, mannitol, sorbitol, lactose and sucrose. Of course, dextrose, for example, can exist as either a direct compression sugar, i.e., a sugar which has been modified to increase its compressibility, or a nondirect compression sugar.

Generally, the balance of the formulation can be matrix. Thus the percentage of filler can approach 100% by weight. However, generally, the amount of nondirect compression filler is about 25 to about 95%, preferably about 50 and about 95% and more preferably about 60 to about 95%.

In the fast-dissolve dosage form, a relatively high proportion of lubricant should be used. Lubricants, and in particular, hydrophobic lubricants such as magnesium stearate, are generally used in an amount of about 0.25 to about 5%, according to the Handbook of Pharmaceutical Excipients. It has been found that the amount of lubricant used can be double, triple or even quadruple that proposed previously. Specifically, the amount of lubricant used can be about 1 to about 2.5% by weight, and more preferably about 1.5 to about 2% by weight. Despite the use of this relatively high weight % of lubricant, the formulations exhibit a superior compressibility, hardness, and rapid dissolution within the mouth.

Hydrophobic lubricants include, for example, alkaline stearates, stearic acid, mineral and vegetable oils, glyceryl behenate, sodium stearyl fumarate, and combinations comprising one or more of the foregoing lubricants. Hydrophilic lubricants can also be used.

The fast-dissolving dosage forms may have a hardness of at least about 1.5 kP and are designed to dissolve spontaneously and rapidly in the mouth of a patient in less than about 90 seconds to thereby liberate the particles. Preferably the dosage form will dissolve in less than about 60 seconds and even more preferably about 45 seconds. This measure of hardness is based on the use of small tablets of less than about 0.25 inches in diameter. A hardness of at least about 2.0 kP is preferred for larger tablets. Direct compression techniques are preferred for the formation of the tablets.

### SPRINKLE DOSAGE FORMS

Sprinkle dosage forms include particulate or pelletized forms of atomoxetine, optionally having functional or non-functional coatings, with which a patient or a caregiver can sprinkle the particulate/pelletized dose into drink or onto soft food. A sprinkle dosage form may comprise particles of about 10 to about 100 micrometers in their major dimension. Sprinkle dosage forms may be in the form of optionally coated granules or as microcapsules. Sprinkle dosage forms may be immediate or controlled-release formulations such as sustained-release formulations. See U.S. Pat. No. 5,084,278, which is hereby incorporated by reference for its teachings regarding microcapsule formulations, which may be administered as sprinkle dosage forms.

Thus an aspect of the invention provides a dosage form of atomoxetine comprising an easily openable capsule enclosing a plurality of micropellets, where each of the micropellets comprises a seed coated with a first coating mixture of atomoxetine and polyvinylpyrrolidone and coated thereon with a second coating mixture of about 90% to about 70% by weight of a non-hydrophilic polymer and about 10% to about 30% by weight of a hydrophilic polymer.

In certain instances the non-liydrophilic polymer is ethyl cellulose. The hydrophilic polymer may be hydroxypropyl methyl cellulose.

The weight of the second coating mixture is about 5-10% of the weight of the micropellets before the second coating is applied. In certain instances of the invention the second coating mixture comprises about 3 parts ethylcellulose to 1 about part hydroxypropylcellulose.

The disclosure includes an atomoxetine sprinkle dosage form wherein the polyvinylpyrrolidone used in the first coating has a molecular weight of about 30,000 to about 50,000; preferably the polyvinylpyrrolidone has a molecular weight of about 40,000.

The seed of the sprinkle dosage form may be a sugar seed and have a mesh size of 60/80.

In certain instances the micropellets of the atomoxetine sprinkle dosage form have a mean diameter of about 0.5 to about 0.7 mm.

The dislcosure also pertains to an atomoxetine sprinkle dosage form formulated as a sustained-release dosage form.

### TASTE MASKED SOLID DOSAGE FORMS

A solid oral dosage form may comprise a taste-masked dosage form. The taste-masked dosage forms may be liquid dosage forms such as those disclosed in U.S. Pat No. 6,197,348, assigned to F.H. Faulding, Inc., which is hereby incorporated by reference for its teachings regarding liquid taste-maslced dosage forms.

A solid taste masked dosage form comprises a core element comprising atomoxetine and a coating surrounding the core element. The core element comprising atomoxetine may be in the form of a capsule or be encapsulated by micro-encapsulation techniques, where a polymeric coating is applied to the formulation. The core element includes atomoxetine and may also include carriers or excipients, fillers, flavoring agents, stabilizing agents and/or colorants.

The taste masked dosage form may include about 77 weight% to about 100 weight%, preferably about 80 weight% to about 90 weight%, based on the total weight of the composition of the core element including atomoxetine; and about 20 weight % to about 70 weight %, of a substantially continuous coating on the core element formed from a coating material including a polymer. The core element includes about 52 %to about 85% by weight of atomoxetine; and approximately 5% to about 25% by weight of a supplementary component selected from waxes, water insoluble polymers, enteric polymers, and partially water soluble polymers, other suitable pharmaceutical excipients, and combinations comprising one or more of the foregoing components.

The core element optionally include carriers or excipients, fillers, flavoring agents, stabilizing agents, colorants, and combinations comprising one or more of the foregoing additives. Suitable fillers include, for example, insoluble materials such as silicon dioxide, titanium dioxide, talc, alumina, starch, kaolin, polacrilin potassium, powdered cellulose, and microcrystalline cellulose, and combinations comprising one or more of the foregoing fillers. Soluble fillers include, for example, mannitol, sucrose, lactose, dextrose, sodium chloride, sorbitol, and combinations comprising one or more of the foregoing fillers. The filler may be present in amounts of up to about 75 weight % based on the total weight of the composition.

The core element may be in the form of a powder, for example, having a particle size range of about 35 µm to about 125 µm. The small particle size facilitates a substantially non-gritty feel in the mouth. Small particle size also minimizes break-up of the particles in the mouth, e.g. by the teeth. When in the form of a powder, the taste masked dosage form may be administered directly into the mouth or mixed with a carrier such as water, or semi-liquid compositions such as yogurt and the like. However, the taste masked atomoxetine may be provided in any suitable unit dosage form.

The coating material of the taste-masked formulation may take a form which provides a substantially continuous coating and still provides taste masking. In some cases, the coating also provides controlled-release of atomoxetine. The polymer used in taste masked dosage form coating may be a water insoluble polymer such as, for example, ethyl cellulose. The coating material of the taste masked dosage form may further include a plasticizer.

A method of preparing taste-masked pharmaceutical formulations such as powdered formulations includes mixing a core element and a coating material in a diluent and spray drying the mixture to form a taste-masked formulation. Spray drying of the pharmaceutically active ingredient and polymer in the solvent involves spraying a stream of air into an atomized suspension so that solvent is caused to evaporate leaving atomoxetine coated with the polymer coating material.

For a solvent such as methylene chloride, the solvent concentration in the drying chamber may be maintained above about 40,000 parts, or about 40,000 to about 100,000 parts per million of organic solvent. The spray-drying process for such solvents may be conducted at a process temperature of about 5°C to about 35°C. Spray drying of the dosage forms may be undertaken utilizing either rotary, pneumatic or pressure atomizers located in either a co-current, counter-current or mixed-flow spray dryer or variations thereof. The drying gas may be heated or cooled to control the rate of drying. A temperature below the boiling point of the solvent may be used. Inlet temperatures may be about 40 °C to about 120 °C and outlet temperatures about 5 °C to about 35 °C.

The coat formation may be optimized to meet the needs of the material or application. Controlling the process parameters including temperature, solvent concentration, spray dryer capacity, atomizing air pressure, droplet size, viscosity, total air pressure in the system and solvent system, allows the formation of a range of coats, ranging from dense, continuous, non-porous coats through to more porous microcapsule/polymer matrices.

A post-treatment step may be used to remove residual solvent. The post treatment may include a post drying step including drying the final product on a tray and drying the product at a bed temperature sufficient to remove excess solvent, but not degrade atomoxetine. Preferably the drying temperature is in the range of about 35 °C to about 4 °C. Once completed, the product may be collected by a suitable method, such as collection by sock filters or cyclone collection.

Thus, in one instance the diclosure includes a chewable taste-maslced dosage form, comprising: a microcapsule of about 10 microns to about 1.5 mm in diameter having a core comprising a pharmaceutically active agent, which is atomoxetine or a pharmaceutically acceptable salt thereof, and a polymer mixture coating having sufficient elasticity to withstand chewing; the polymeric mixture coating comprising: about 50% by weight of a polymer that forms a polymeric film at temperatures of at least about 30 °C; and about 50% by weight of a low temperature film forming copolymer that forms a polymeric film at temperatures less than about 25 °C; and the polymeric mixture coating is adapted to release the pharmaceutically active agent in the stomach. Preferably the active agent is atomoxetine hydrochloride.

The disclosure includes a taste-masked atomoxetine dosage form in which the polymer that forms a polymeric film at temperatures of at least about 30 °C is an ethyl cellulose.

The disclosure also includes a taste-masked atomoxetine dosage form in which the low temperature film forming copolymer is a methacrylic acid ester copolymer or a styrene acrylate copolymer. In other instances the taste-masked dosage the low temperature film forming polymer comprises a polymethacrylic acid ester copolymer having a weight average molecular weight of about 800,000.

In certain instances the core of the taste-masked atomoxetine dosage form described above further comprises a diluent.

The polymer coating of the chewable taste-masked atomoxetine dosage form may further comprise a plasticizer. Suitable plasticizers, include, but are not limited to polyethylene glycol, triacetin, vinylpyrrolidone, diethyl phthallate, dibutylsebacate, or a citric acid ester and combinations thereof.

The disclosure also includes a taste-masked atomoxetine solid, preferably chewable, dosage form, wherein the dosage form is a sustained-release dosage form.

### TASTE MASKED LIQUID DOSAGE FORMS

Liquid dosage forms of atomoxetine that provide adequate taste masking may also be formulated. A taste masked liquid dosage form can comprise a suspension of microcapsules taste masked as a function of the pH of a suspending medium and a polymer coating. Many active agents are less soluble at higher or lower pH than at the pH value of the mouth, which is around 5.9. In these cases, atomoxetine can be insufficiently solubilized to be tasted if the equilibrium concentration is below the taste threshold. However, problems can arise if all of the suspended particles are not swallowed because atomoxetine which remains in the mouth is then able to dissolve at the pH of the mouth. The use of polymeric coatings on atomoxetine particles, which inhibit or retard the rate of dissolution and solubilization of atomoxetine is one means of overcoming the taste problems with delivery of atomoxetine in suspension. The polymeric coating allows time for all of the particles to be swallowed before the taste threshold concentration is reached in the mouth.

Optimal taste masked liquid formulations may be obtained when consideration is given to: (i) the pH of maximum insolubility of atomoxetine; (ii) the threshold concentration for taste of atomoxetine; (iii) the minimum buffer strength required in the medium to avoid delayed or after taste; (iv) the pH limit beyond which further increase or decrease of pH leads to unacceptable instability of atomoxetine; and (v) the compatibility and chemical, physical and microbial stability of the other ingredients to the pH values of the medium.

Thus the disclosure provides a taste masked liquid dosage form that comprises particles of an active agent which is atomoxetine or a pharmaceutically acceptable salt thereof, a polymer encapsulating the particles, wherein the polymer has quaternary ammonium groups on the polymer backbone; and a liquid suspending medium for suspending the encapsulated particles, wherein the liquid suspending medium comprises a water-based medium adjusted to a predetermined pH at which the active agent remains substantially insoluble. Atomoxetine is taste masked by the combination of the polymer and suspending medium. Preferably atomoxetine is present in the form of atomoxetine hydrochloride.

Atomoxetine, in the taste masked dosage form, may be in the form of its neutral or salt form and may be in the form of particles, crystals, microcapsules, granules, microgranules, powders, pellets, amorphous solids or precipitates. The particles may further include other functional components. Atomoxetine may have a defined particle size distribution, preferably in the region of about 0.1 to about 500 µm, more preferably about 1 to about 250 µm, and most preferably about 10 to about 150 µm, where there is acceptable mouth feel and little chance of chewing on the residual particles and releasing atomoxetine to taste.

The taste masked liquid dosage form may include, along with atomoxetine, other functional components present for the purpose of modifying the physical, chemical, or taste properties of atomoxetine. For example atomoxetine may be in the form of ion-exchange or cyclodextrin complexes or atomoxetine may be included as a mixture or dispersion with various additives such as waxes, lipids, dissolution inhibitors, taste-masking or -suppressing agents, carriers or excipients, fillers, and combinations comprising one or more of the foregoing components.

The polymer used to encapsulate the pharmaceutically active ingredient or the pharmaceutical unit is preferably a polymer having a quaternary ammonium functionality, i.e., a polymer having quaternary ammonium groups on the polymer backbone. These polymers are more effective in preventing the taste perception of atomoxetine when the resulting microcapsules are formulated as suspensions that may be stored for long periods despite their widely recognized properties of being permeable to water and dissolved atomoxetine. A suitable polymer is a copolymer of acrylic and methacrylic acid esters with quaternary ammonium groups. The polymer may be a copolymer of methyl methacrylate and triethylammonium methacrylate. Specific examples of suitable polymer include EUDRAGIT RS or EUDRAGIT RL, available from Röhm America, LLC, Piscataway, NJ used individually or in combination to change the permeability of the coat. A polymer coat having a blend of the RS or RL polymer along with other pharmaceutically acceptable polymers may also be used. These other polymers may be cellulose ethers such as ethyl cellulose, cellulose esters such as cellulose acetate and cellulose propionate, polymers that dissolve at acidic or alkaline pH, such as EUDRAGIT E, cellulose acetate phthalate, and hydroxypropylmethyl cellulose phthalate.

The quantity of polymer used in relation to atomoxetine is about 0.01-10:1, preferably about 0.02-1:1, more preferably about 0.03-0.5:1 and most preferably about 0.05-0.3:1 by weight.

The atomoxetine particle may be suspended, dispersed or emulsified in the suspending medium after encapsulation with the polymer. The suspending medium may be a water-based medium, but may be a non-aqueous carrier as well, constituted at an optimum pH for atomoxetine or pharmaceutical unit, such that atomoxetine remains substantially insoluble. The pH and ionic strength of the medium may be selected on the basis of stability, solubility and taste threshold to provide the optimum taste masking effect, and which is compatible with the stability of atomoxetine the polymer coat and the coating excipients.

Buffering agents may be included in the suspending medium for maintaining the desired pH. The buffering agents may include dihydrogen phosphate, hydrogen phosphate, amino acids, citrate, acetate, phthalate, tartrate salts of the alkali or alkaline earth metal cations such as sodium, potassium, magnesium, calcium, and combinations comprising one or more of the foregoing buffering agents. The buffering agents may be used in a suitable combination for achieving the required pH and may be of a buffer strength of about 0.01 to about 1 moles/liter of the final formulation, preferably about 0.01 to about 0.1 moles/liter, and most preferably about 0.02 to about 0.05 moles/liter.

The taste masked liquid dosage form may further include other optional dissolved or suspended agents to provide stability to the suspension. These include suspending agents or stabilizers such as, for example, methyl cellulose, sodium alginate, xanthan gum, (poly)vinyl alcohol, microcrystalline cellulose, colloidal silicas, bentonite clay, and combinations comprising one or more of the foregoing agents. Other agents used include preservatives such as methyl, ethyl, propyl and butyl parabens, sweeteners such as sucrose, saccharin sodium, aspartame, mannitol, flavorings such as grape, cherry, peppermint, menthol and vanilla flavors, and antioxidants or other stabilizers, and combinations comprising one or more of the foregoing agents.

A method of preparing a taste masked dosage form for oral delivery, comprises encapsulating atomoxetine with a polymer having a quaternary ammonium functionality; and adding a suspending medium adjusted to a pH at which atomoxetine is substantially insoluble, for suspending the encapsulated atomoxetine; wherein atomoxetine is taste masked by the combination of the polymer and the medium. In the process, the polymer for encapsulation of atomoxetine or the atomoxetine-containing particle is dissolved in a solution or solvent chosen for its poor solubility for atomoxetine and good solubility for the polymer. Examples of appropriate solvents include but are not limited to methanol, ethanol, isopropanol, chlorofom, methylene chloride, cyclohexane, and toluene, either used in combination or used alone. Aqueous dispersions of polymers may also be used for forming atomoxetine microparticles.

Encapsulation of atomoxetine or pharmaceutical unit by the polymer may be performed by a method such as suspending, dissolving, or dispersing the pharmaceutically active ingredient in a solution or dispersion of polymer coating material and spray drying, fluid-bed coating, simple or complex coacervation, coevaporation, co-grinding, melt dispersion and emulsion-solvent evaporation techniques, and the like.

Polymer coated atomoxetine powder can also as an alternative be applied for the preparation of reconstitutable powders, ie; dry powder atomoxetine products that are reconstituted as suspensions in a liquid vehicle such as water before usage. The reconstitutable powders have a long shelf life and the suspensions, once reconstituted, have adequate taste masking.

One aspect of the disclosure pertains to a taste-masked liquid dosage form, comprising: particles of an active agent, wherein the active agent is atomoxetine or a pharmaceutically acceptable salt thereof; and a polymer encapsulating the particles, wherein the polymer has quaternary ammonium groups on the polymer backbone; and a liquid suspending medium for suspending the encapsulated particles, wherein the liquid suspending medium comprises a water-based medium adjusted to a predetermined pH at which atomoxetine remains substantially insoluble. Preferably the active agent is atomoxetine hydrochloride.

The polymer encapsulating the particles may be a copolymer of acrylic and methacrylic acid esters with quaternary ammonium groups, or a copolymer of methyl methacrylate and triethylammonium methacrylate.

In certain instances of the invention the ratio of polymer to active agent in the taste masked atomoxetine liquid dosage form is about 0.01:1 1 to about 10:1.

The disclosure also provides a taste-masked atomoxetine liquid dosage form, in which atomoxetine or its salt is in the form of ion-exchange complex, a cyclodextrin complex, or as a mixture with a wax, a lipid, a dissolution inhibitor, a taste-masking agent, a taste-suppressing agent, a carrier, an excipient, a filler, or a combination comprising at least one of the foregoing forms.

The disclosure includes taste-masked atomoxetine liquid dosage forms as described above further comprising an additional polymer, wherein the additional polymer is a cellulose ether, a cellulose ester, and polymers that dissolve at acidic or alkaline pH.

The disclosure includes taste-masked atomoxetine liquid dosage forms as described above, wherein the suspending medium further comprises a buffering agent.

The disclosure also includes instances in which the buffering agent of the taste-masked atomoxetine liquid dosage form has a buffer strength of 0.1 to 1 moles/liter.

The disclosure includes taste-masked liquid dosage forms as described above, which further comprise a stabilizer, wherein the stabilizer is methyl cellulose, sodium alginate, xanthan gum, (poly)vinyl alcohol, microcrystalline cellulose, colloidal silicas, bentonite clay, or a combination of any of the foregoing stabilizers.

In certain instances the invention includes taste-masked atomoxetine, liquid dosage forms as described above wherein the particle size is about 0.1 to about 500 micrometers.

In certain instances the taste-masked atomoxetine liquid dosage form is a fast-dissolve form.

### OSMOTIC PUMP DOSAGE FORMS

Another dosage form of atomoxetine is one formulated with "osmotic pump" technology, such as OROS technology (Alza Corporation, Mountain View, CA). Such dosage forms have a fluid-permeable (semipermeable) membrane wall, an osmotically active expandable driving member (the osmotic push layer), and a density element for delivering atomoxetine. In an osmotic pump dosage form, the active material may be dispensed through an exit means comprising a passageway, orifice, or the like, by the action of the osmotically active driving member, Atomoxetine, of the osmotic pump dosage form may be formulated as a thermo-responsive formulation in which atomoxetine is dispersed in a thermo-responsive composition. Alternatively, the osmotic pump dosage form may contain a thermo-responsive element comprising a thermo-responsive composition at the interface of the osmotic push layer and atomoxetine composition.

The osmotic pump dosage form comprises a semipermeable membrane. The capsule or other dispenser of the osmotic pump dosage form can be provided with an outer wall comprising the selectively semipermeable material. A selectively permeable material is one that does not adversely affect a host or animal, is permeable to the passage of an external aqueous fluid, such as water or biological fluids, while remaining essentially impermeable to the passage of atomoxetine, and maintains its integrity in the presence of a thermotropic thermo-responsive composition, that is it does not melt or erode in its presence. The selectively semipermeable material forming the outer wall is substantially insoluble in body fluids, nontoxic, and non-erodible.

Representative materials for fonning the selectively semipermeable wall include semipermeable homopolymers, semipermeable copolymers, and the like. Suitable materials include, for example, cellulose esters, cellulose monoesters, cellulose diesters, cellulose triesters, cellulose ethers, cellulose ester-ethers, and combinations comprising one or more of the foregoing materials. These cellulosic polymers have a degree of substitution, D.S., on their anhydroglucose unit from greater than 0 up to 3 inclusive. By degree of substitution is meant the average number of hydroxyl groups originally present on the anhydroglucose unit that are replaced by a substituting group, or converted into another group. The anhydroglucose unit can be partially or completely substituted with groups such as acyl, alkanoyl, aroyl, alkyl, alkenyl, alkoxy, halogen, carboalkyl, alkylcarbamate, alkylcarbonate, alkylsulfonate, alkylsulfamate, and like semipermeable polymer forming groups.

Other selectively semipermeable materials include, for example, cellulose acylate, cellulose diacylate, cellulose triacylate, cellulose acetate, cellulose diacetate, cellulose triacetate, mono-, di- and tri-cellulose allcanylates, mono-, di- and tri-alkenylates, mono-, di- and tri-aroylates, and the like, and combinations comprising one or more of the foregoing materials. Exemplary polymers including cellulose acetate having a D.S. of 1.8 to 2.3 and an acetyl content of about 32 to about 39.9%; cellulose diacetate having a D.S. of 1 to 2 and an acetyl content of about 21 to about 35%; cellulose triacetate having a D.S of 2 to 3 and an acetyl content of about 34 to about 44.8%, and the like. More specific cellulosic polymers include cellulose propionate having a D.S. of 1.8 and a propionyl content of about 38.5%; cellulose acetate propionate having an acetyl content of about 1.5 to about 7% and an propionyl content of about 39 to about 42%; cellulose acetate propionate having an acetyl content of about 2.5 to about 3%, an average propionyl content of about 39.2 to about 45% and a hydroxyl content of about 2.8 to about 5.4%; cellulose acetate butyrate having a D.S. of 1.8, an acetyl content of about 13 to about 15%, and a butyryl content of about 34 to about 39%; cellulose acetate butyrate having an acetyl content of about 2 to about 29.5%, a butyryl content of about 17 to about 53%, and a hydroxyl content of about 0.5 to about 4.7%; cellulose triacylates having a D.S. of 2.9 to 3 such as cellulose trivalerate, cellulose trilaurate, cellulose tripalmitate, cellulose trioctanoate, and cellulose tripropionate; cellulose diesters having a D.S. of 2.2 to 2.6 such as cellulose disuccinate, cellulose dipalmitate, cellulose dioctanoate, cellulose dicarpylate and the like; mixed cellulose esters such as cellulose acetate valerate, cellulose acetate succinate, cellulose propionate succinate, cellulose acetate octanoate, cellulose valerate palmitate, cellulose acetate heptonate, and the like, and combinations comprising one or more of the foregoing polymers.

Additional selectively semipermeable polymers include, for example, acetaldehyde dimethyl cellulose acetate, cellulose acetate ethylcarbamate, cellulose acetate methylcarbamate, cellulose dimethylaminoacetate, semi-permeable polyamides, semipermeable polyurethanes, semi-permeable polysulfanes, semipermeable sulfonated polystyrenes, cross-linked, selectively semipermeable polymers formed by the coprecipitation of a polyanion and a polycation, selectively semipermeable silicon rubbers, semipermeable polystyrene derivates, semipermeable poly(sodium styrenesulfonate), semipermeable poly(vinylbenzyltrimethyl) ammonium chloride polymers, and combinations comprising one or more of the foregoing polymers.

The osmotically expandable driving member, or osmotic push layer, of the soft capsule osmotic pump dosage form is swellable and expandable inner layer. The materials used for forming the osmotic push layer, are neat polymeric materials, and/or polymeric materials blended with osmotic agents that interact with water or a biological fluid, absorb the fluid, and swell or expand to an equilibrium state. The polymer should exhibit the ability to retain a significant fraction of imbibed fluid in the polymer molecular structure. Such polymers may be, for example, gel polymers that can swell or expand to a very high degree, usually exhibiting about a 2 to 50-fold volume increase. Swellable, hydrophilic polymers, also known as osmopolymers, can be non-cross-linked or lightly cross-linked. The cross-links can be covalent or ionic bonds with the polymer possessing the ability to swell but not dissolve in the presence of fluid. The polymer can be of plant, animal or synthetic origin. Polymeric materials useful for the present purpose include poly(hydroxyalkyl methacrylate) having a molecular weight of about 5,000 to about 5,000,000, poly(vinylpyrrolidone) having a molecular weight of about 10,000 to about 360,000, anionic and cationic hydrogels, poly(electrolyte) complexes, poly(vinyl alcohol) having a low acetate residual, a swellable mixture of agar and carboxymethyl cellulose, a swellable composition comprising methyl cellulose mixed with a sparingly crosslinked agar, a water-swellable copolymer produced by a dispersion of finely divided copolymer of maleic anhydride with styrene, ethylene, propylene, or isobutylene, water swellable polymer of N-vinyl lactams, and the like, and combinations comprising one or more of the foregoing polymers. Other gelable, fluid imbibing and retaining polymers useful for forming the osmotic push layer include pectin having a molecular weight ranging from about 30,000 to about 300,000, polysaccharides such as agar, acacia, karaya, tragacanth, algins and guar, acidic carboxy polymer and its salt derivatives, polyacrylamides, water-swellable indene maleic anhydride polymers; polyacrylic acid having a molecular weight of about 80,000 to about 200,000, polyethylene oxide polymers having a molecular weight of about 100,000 to about 5,000,000, and greater, starch graft copolymers, polyanions and polycations exchange polymers, starch-polyacrylonitrile copolymers, acrylate polymers with water absorbability of about 400 times its original weight, diesters of polyglucan, a mixture of cross-linked polyvinyl alcohol and poly(N-vinyl-2-pyrrolidone), zein available as prolamine, Polyethylene glycol) having a molecular weight of about 4,000 to about 100,000, and the like, and combinations comprising one or more of the foregoing polymers.

The osmotically expandable driving layer of the osmotic pump dosage form may further contain an osmotically effective compound (osmagent) that can be used neat or blended homogeneously or heterogeneously with the swellable polymer, to form the osmotically expandable driving layer. Such osmagents include osmotically effective solutes that are soluble in fluid imbibed into the swellable polymer, and exhibit an osmotic pressure gradient across the semipermeable wall against an exterior fluid. Suitable osmagents include, for example, solid compounds such as magnesium sulfate, magnesium chloride, sodium chloride, lithium chloride, potassium sulfate, sodium sulfate, mannitol, urea, sorbitol, inositol, sucrose, glucose, and the like, and combinations comprising one or more of the foregoing osmagents. The osmotic pressure in atmospheres, atm, of the osmagent may be greater than about zero atm, and generally about zero atm to about 500 atm, or higher.

The swellable, expandable polymer of the osmotically expandable driving layer, in addition to providing a driving source for delivering atomoxetine from the dosage form, may also function as a supporting matrix for an osmotically effective compound. The osmotic compound can be homogeneously or heterogeneously blended with the polymer to yield the desired expandable wall or expandable pocket. The compositon in a presently preferred instance comprises (a) at least one polymer and at least one osmotic compound, or (b) at least one solid osmotic compound. Generally, a composition will comprise about 20% to about 90% by weight of polymer and about 80% to about 10% by weight of osmotic compound, with a presently preferred composition comprising about 35% to about 75% by weight of polymer and about 65% to about 25% by weight of osmotic compound.

Atomoxetine of the osmotic pump dosage form may be formulated as a thermo-responsive formulation in which atomoxetine is dispersed in a thermo-responsive composition. Alternatively, the osmotic pump dosage form may contain a thermo-responsive element comprising a thermo-responsive composition at the interface of the osmotic push layer and atomoxetine composition. Representative thermo-responsive compositions and their melting points are as follows: Cocoa butter (32 °C-34 °C), cocoa butter plus 2% beeswax (35 °C-37 °C), propylene glycol monostearate and distearate (32 °C-35 °C), hydrogenated oils such as hydrogenated vegetable oil (36 °C-37.5 °C), 80% hydrogenated vegetable oil and 20% sorbitan monopalmitate (39°C-39.5 °C), 80% hydrogenated vegetable oil and 20% polysorbate 60, (36 °C-37 °C), 77.5% hydrogenated vegetable oil, 20% sorbitan trioleate, 2.5% beeswax and 5.0% distilled water, (37 °C-38 °C), mono-, di-, and triglycerides of acids having from 8-22 carbon atoms including saturated and unsaturated acids such as palmitic, stearic, oleic, lineolic, linolenic and archidonic; triglycerides of saturated fatty acids with mono- and diglycerides (34 °C-35.5 °C), propylene glycol mono- and distearates (33 °C-34 °C), partially hydrogenated cottonseed oil (35 °C-39 °C), a block polymer of polyoxy-alkylene and propylene glycol; block polymers comprising 1,2-butylene oxide to which is added ethylene oxide; block copolymers of propylene oxide and ethylene oxide, hardened fatty alcohols and fats (33 °C-36 °C), hexadienol and hydrous lanolin triethanolamine glyceryl monostearate (38 °C), eutectic mixtures of mono-, di-, and triglycerides (35 °C-39 °C), WITEPSOL#15, triglyceride of saturated vegetable fatty acid with monoglycerides (33.5 °C-35.5 °C), WITEPSOL H32 free of hydroxyl groups (31 °C-33 °C), WITEPSOL W25 having a saponification value of 225-240 and a melting point of (33.5 °C-35.5 °C), WITEPSOL E75 having a saponification value of 220-230 and a melting point of (37 °C-39 °C), a polyalkylene glycol such as polyethylene glycol 1000, a linear polymer of ethylene oxide (38 °C-41 °C), polyethylene glycol 1500 (38 °C-41 °C), polyethylene glycol monostearate (39 °C-42.5 °C), 33% polyethylene glycol 1500, 47% polyethylene glycol 6000 and 20% distilled water (39 °C-41 °C), 30% polyethylene glycol 1500, 40% polyethylene glycol 4000 and 30% polyethylene glycol 400, (33 °C-38 °C), mixture of mono-, di-, and triglycerides of saturated fatty acids having 11 to 17 carbon atoms, (33 °C-35 °C), and the like. The thermo-responsive compositions; including thermo-responsive carriers are useful for storing atomoxetine in a solid composition at a temperature of about 20 °C to about 33 °C, maintaining an immiscible boundary at the swelling composition interface, and for dispensing the agent in a flowable composition at a temperature greater than about 33 °C and preferably between about 33 °C and about 40 °C.

The amount of atomoxetine present in the osmotic pump dosage form is about 25 mg to about 2 g or more. The osmotic dosage form may be formulated for once daily or less frequent administration.

Atomoxetine in the osmotic pump dosage form may be formulated by a number of techniques known in the art for formulating solid and liquid oral dosage forms. Atomoxetine formulations of the osmotic pump dosage form may be formulated by wet granulation. In an exemplary wet granulation method, atomoxetine and the ingredients comprising the atomoxetine layer are blended using an organic solvent, such as isopropyl alcohol-ethylene dichloride 80:20 v:v (volume:volume) as the granulation fluid. Other granulating fluid such as denatured alcohol 100% may be used for this purpose. The ingredients forming the atomoxetine layer are individually passed through a screen such as a 40-mesh screen and then thoroughly blended in a mixer. Next, other ingredients comprising the atomoxetine layer are dissolved in a portion of the granulation fluid, such as the cosolvent described above. Then the latter prepared wet blend is slowly added to the atomoxetine blend with continual mixing in the blender. The granulating fluid is added until a wet blend is produced, which wet mass then is forced through a screen such as a 20-mesh screen onto oven trays. The blend is dried for about 18 to about 24 hours at about 30 °C to about 50 °C. The dry granules are sized then with a screen such as a 20-mesh screen. Next, a lubricant is passed through a screen such as an 80-mesh screen and added to the dry screen granule blend. The granulation is put into milling jars and mixed on a jar mill for about 1 to about 15 minutes. The push layer may also be made by the same wet granulation techniques. The compositions are pressed into their individual layers in a KILIAN press-layer press.

Another manufacturing process that can be used for providing the atomoxetine layer and osmotically expandable driving layer comprises blending the powered ingredients for each layer independently in a fluid bed granulator. After the powered ingredients are dry blended in the granulator, a granulating fluid, for example, poly(vinyl-pyrrolidone) in water, or in denatured alcohol, or in 95:5 ethyl alcohol/water, or in blends of ethanol and water is sprayed onto the powders. Optionally, the ingredients can be dissolved or suspended in the granulating fluid. The coated powders are then dried in a granulator. This process granulates the ingredients present therein while adding the granulating fluid. After the granules are dried, a lubricant such as stearic acid or magnesium stearate is added to the granulator. The granules for each separate layer are pressed then in the manner described above.

The osmotic push atomoxetine formulation and osmotic push layer of the osmotic push dosage form may also be manufactured by mixing atomoxetine with composition forming ingredients and pressing the composition into a solid lamina possessing dimensions that correspond to the internal dimensions of the compartment. In another manufacture, atomoxetine, other atomoxetine composition-forming ingredients, and a solvent are mixed into a solid, or a semisolid, by methods such as ballmilling, calendaring, stirring or rollmilling, and then pressed into a preselected layer forming shape. Next, a layer of a composition comprising an osmopolymer and an optional osmagent are placed in contact with the layer comprising atomoxetine. The layering of the first layer comprising atomoxetine and the second layer comprising the osmopolymer and optional osmagent composition can be accomplished by using a conventional layer press technique. The semipermeable wall can be applied by molding, spraying or dipping the pressed bilayer's shapes into wall forming materials. An air suspension coating procedure which includes suspending and tumbling the two layers in current of air until the wall forming composition surrounds the layers is also used to form the semi-permeable wall of the osmotic dosage forms.

The dispenser of the osmotic pump dosage form may be in the form of a capsule. The capsule may comprise an osmotic hard capsule and/or an osmotic soft capsule. The osmotic hard capsule may be composed of two parts, a cap and a body, which are fitted together after the larger body is filled with atomoxetine. The osmotic hard capsule may be fitted together by slipping or telescoping the cap section over the body section, thus completely surrounding and encapsulating atomoxetine. Hard capsules may be made by techniques known in the art.

The soft capsule of the osmotic pump dosage form may be a one-piece osmotic soft capsule. Generally, the osmotic soft capsule is of sealed construction encapsulating atomoxetine. The soft capsule may be made by various processes, such as the plate process, the rotary die process, the reciprocating die process, and the continuous process.

Materials useful for forming the capsule of the osmotic pump dosage form are commercially available materials including gelatin, gelatin having a viscosity of about 5 to about 30 millipoises and a bloom strength up to about 150 grams; gelatin having a bloom value of about 160 to about 250; a composition comprising gelatin, glycerine, water and titanium dioxide; a composition comprising gelatin, erythrosin, iron oxide and titanium dioxide; a composition comprising gelatin, glycerine, sorbitol, potassium sorbate and titanium dioxide; a composition comprising gelatin, acacia, glycerin, and water; and the like, and combinations comprising one or more of the foregoing materials.

The semipermeable wall forming composition can be applied to the exterior surface of the capsule in laminar arrangement by molding, forming, air spraying, dipping or brushing with a semipermeable wall forming composition. Other techniques that can be used for applying the semipermeable wall are the air suspension procedure and the pan coating procedures. The air suspension procedure includes suspending and tumbling the capsule arrangement in a current of air and a semipermeable wall forming composition until the wall surrounds and coats the capsule. The procedure can be repeated with a different semipermeable wall forming composition to form a semipermeable laminated wall.

Exemplary solvents suitable for manufacturing the semipermeable wall include inert inorganic and organic solvents that do not adversely harm the materials, the capsule wall, atomoxetine, the thermo-responsive composition, the expandable member, or the final dispenser. Solvents for manufacturing the semipermeable wall may be aqueous solvents, alcohols, ketones, esters, ethers, aliphatic hydrocarbons, halogenated solvents, cycloaliphatics, aromatics, heterocyclic solvents, and combinations comprising one or more of the foregoing solvents. Particular solvents include acetone, diacetone alcohol, methanol, ethanol, isopropyl alcohol, butyl alcohol, methyl acetate, ethyl acetate, isopropyl acetate, n-butyl acetate, methyl isobutyl ketone, methyl propyl ketone, n-hexane, n-heptane, ethylene glycol monoethyl ether, ethylene glycol monoethyl acetate, methylene dichloride, ethylene dichloride, propylene dichloride, carbon tetrachloride, nitroethane, nitropropane, tetrachloroethane, ethyl ether, isopropyl ether, cyclohexane, cyclooctane, benzene, toluene, naphtha, 1,4-dioxane, tetrahydrofuran, water, and mixtures thereof such as acetone and water, acetone and methanol, acetone and ethyl alcohol, methylene dichloride and methanol, and ethylene dichloride,methanol, and combinations comprising one or more of the foregoing solvents. The semipermeable wall may be applied at a temperature a few degrees less than the melting point of the thermo-responsive composition. Alternatively, the thermo-responsive composition can be loaded into the dispenser after applying the semipermeable wall.

The exit means or hole in the osmotic pump dosage form, for releasing atomoxetine, can be formed by mechanical or laser drilling, or by eroding an erodible element in the wall, such as a gelatin plug. The orifice can be a polymer inserted into the semipermeable wall, which polymer is a porous polymer and has at least one pore, or which polymer is a microporous polymer and has at least one micro-pore.

### CONTROLLED-RELEASE FORMULATION FOR RELEASE INTO THE STOMACH AND UPPER GASTROINTESTINAL TRACT

An exemplary controlled-release formulation for release of atomoxetine in the stomach and gastrointestinal tract is one in which atomoxetine is dispersed in a polymeric matrix that is water-swellable rather than merely hydrophilic, that has an erosion rate that is substantially slower than its swelling rate, and that releases atomoxetine primarily by diffusion. The rate of diffusion of atomoxetine out of the matrix can be slowed by increasing atomoxetine particle size, by the choice of polymer used in the matrix, and/or by the choice of molecular weight of the polymer. The matrix is a relatively high molecular weight polymer that swells upon ingestion, preferably to a size that is at least about twice its unswelled volume, and that promotes gastric retention during the fed mode. Upon swelling, the matrix may also convert over a prolonged period of time from a glassy polymer to a polymer that is rubbery in consistency, or from a crystalline polymer to a rubbery one. The penetrating fluid then causes release of atomoxetine in a gradual and prolonged manner by the process of solution diffusion, i.e., dissolution of atomoxetine in the penetrating fluid and diffusion of the dissolved drug back out of the matrix. The matrix itself is solid prior to administration and, once administered, remains undissolved in (i.e., is not eroded by) the gastric fluid for a period of time sufficient to permit substantially all of atomoxetine to be released by the solution diffusion process during the fed mode. By substantially all, it is meant greater than or equal to about 90 wt%, preferably greater than or equal to about 95 wt% of atomoxetine is released. The rate-limiting factor in the release of atomoxetine may be therefore controlled diffusion of atomoxetine from the matrix rather than erosion, dissolving or chemical decomposition of the matrix.

For soluble active agents such as atomoxetine, the swelling of the polymeric matrix thus achieves two objectives: (i) causing the tablet to swell to a size large enough to cause it to be retained in the stomach during the fed mode, and (ii) retarding the rate of diffusion of the soluble active agent long enough to provide multi-hour, controlled delivery of the active agent into the stomach.

The water-swellable polymer forming the matrix is a polymer that is non-toxic, that swells in a dimensionally unrestricted manner upon imbibition of water, and that provides for sustained-release of incorporated atomoxetine. Examples of suitable polymers include, for example, cellulose polymers and their derivatives (such as for example, hydroxyethylcellulose, hydroxypropylcellulose, carboxymethylcellulose, and microcrystalline cellulose, polysaccharides and their derivatives, polyalkylene oxides, polyethylene glycols, chitosan, poly(vinyl alcohol), xanthan gum, maleic anhydride copolymers, poly(vinyl pyrrolidone), starch and starch-based polymers, poly (2-ethyl-2-oxazoline), poly(ethyleneimine), polyurethane hydrogels, and crosslinked polyacrylic acids and their derivatives. Further examples are copolymers of the polymers listed in the preceding sentence, including block copolymers and grafted polymers. Specific examples of copolymers are PLURONIC and TECTONIC, which are polyethylene oxide-polypropylene oxide block copolymers available from BASF Corporation, Chemicals Div., Wyandotte, Mich., USA.

The terms "cellulose" and "cellulosic" denote a linear polymer of anhydroglucose. Cellulosic polymers include, for example, alkyl- substituted cellulosic polymers that ultimately dissolve in the gastrointestinal (GI) tract in a predictably delayed manner. Alkyl-substituted cellulose derivatives may be those substituted with alkyl groups of 1 to 3 carbon atoms each. Specific examples are methylcellulose, hydroxymethylcellulose, hydroxyethylcellulose, hydroxypropylcellulose, hydroxypropylmethylcellulose, and carboxymethylcellulose. In terms of their viscosities, one class of suitable alkyl-substituted celluloses includes those whose viscosity is about 100 to about 110,000 centipoise as a 2% aqueous solution at 20°C. Another class includes those whose viscosity is about 1,000 to about 4,000 centipoise as a 1% aqueous solution at 20°C. Exemplary alkyl-substituted celluloses are hydroxyetliylcellulose and hydroxypropylmethylcellulose. A specific example of a hydroxyethylcellulose is NATRASOL 250HX NF (National Formulary), available from Aqualon Company, Wilmington, Del., USA.

Suitable polyalkylene oxides are those having the properties described above for alkyl-substituted cellulose polymers. An example of a polyalkylene oxide is poly(ethylene oxide), which term is used herein to denote a linear polymer of unsubstituted ethylene oxide. Poly(ethylene oxide) polymers having molecular weights of about 4,000,000 and higher are preferred. More preferred are those with molecular weights of about 4,500,000 to about 10,000,000, and even more preferred are polymers with molecular weights of about 5,000,000 to about 8,000,000. Preferred polyethylene oxides are those with a weight-average molecular weight within the range of about 1×10⁵ to about 1×10⁷, and preferably within the range of about 9×10⁵ to about 8×10⁶. Poly(ethylene oxide)s are often characterized by their viscosity in solution and may have a viscosity of about 50 to about 2,000,000 centipoise for a 2% aqueous solution at 20°C. Two specific example of poly(ethylene oxide)s are POLYOX NF, grade WSR Coagulant, molecular weight 5 million, and grade WSR 303, molecular weight 7 million, both available from Dow.

Polysaccharide gums, both natural and modified (semi-synthetic) can be used. Examples are dextran, xanthan gum, gellan gum, welan gum and rhamsan gum.

Crosslinked polyacrylic acids of greatest utility are those whose properties are the same as those described above for alkyl-substituted cellulose and polyalkylene oxide polymers. Certain crosslinked polyacrylic acids useful in the disclosure are those with a viscosity of about 4,000 to about 40,000 centipoise for a 1% aqueous solution at 25°C. Three specific examples are CARBOPOL NF grades 971P, 974P and 934P (BPGoodrich Co., Specialty Polymers and Chemicals Div., Cleveland, Ohio, USA). Further examples include polymers known as WATER LOCK, which are starch/acrylates/acrylamide copolymers available from Grain Processing Corporation, Muscatine, Iowa, USA.

The hydrophilicity and water swellability of these polymers cause atomoxetine-containing matrices to swell in size in the gastric cavity due to ingress of water in order to achieve a size that will be retained in the stomach when introduced during the fed mode. These qualities also cause the matrices to become slippery, which provides resistance to peristalsis and further promotes their retention in the stomach. The release rate of atomoxetine from the matrix is primarily dependent upon the rate of water imbibition and the rate at which atomoxetine dissolves and diffuses from the swollen polymer, which in turn is related to the solubility and dissolution rate of atomoxetine, atomoxetine particle size and atomoxetine concentration in the matrix. Also, because these polymers dissolve very slowly in gastric fluid, the matrix maintains its physical integrity over at least a substantial period of time, in many cases at least 90% and preferably over 100% of the dosing period. The particles will then slowly dissolve or decompose. Complete dissolution or decomposition may not occur until 24 hours or more after the intended dosing period ceases, although in most cases, complete dissolution or decomposition will occur within 10 to 24 hours after the dosing period.

The dosage forms may include additives that impart a small degree of hydrophobic character, to further retard the release rate of atomoxetine into the gastric fluid. One example of such a release rate retardant is glyceryl monostearate. Other examples are fatty acids and salts of fatty acids, one example of which is sodium myristate. The quantities of these additives when present can vary; and in most cases, the weight ratio of additive to atomoxetine will be about 1:20 to about 1:1, and preferably about 1:8 to about 1:2.

The amount of polymer relative to atomoxetine can vary, depending on atomoxetine release rate desired and on the polymer, its molecular weight, and excipients that may be present in the formulation. The amount of polymer will be sufficient however to retain at least about 40% of atomoxetine within the matrix one hour after ingestion (or immersion in the gastric fluid). Preferably, the amount of polymer is such that at least 50% of atomoxetine remains in the matrix one hour after ingestion. More preferably, at least 60%, and most preferably at least 80%, of atomoxetine remains in the matrix one hour after ingestion. In all cases, however, atomoxetine will be substantially all released from the matrix within about ten hours, and preferably within about eight hours, after ingestion, and the polymeric matrix will remain substantially intact until all of atomoxetine is released. The term "substantially intact" is used herein to denote a polymeric matrix in which the polymer portion substantially retains its size and shape without deterioration due to becoming solubilized in the gastric fluid or due to breakage into fragments or small particles.

The water-swellable polymers can be used individually or in combination. Certain combinations will often provide a more controlled-release of atomoxetine than their components when used individually. An examplary combination is cellulose-based polymers combined with gums, such as hydroxyethyl cellulose or hydroxypropyl cellulose combined with xanthan gum. Another example is poly(ethylene oxide) combined with xanthan gum.

The benefits of this dosage form will be achieved over a wide range of atomoxetine loadings, with the weight ratio of atomoxetine to polymer of 0.01:99.99 to about 80:20. Preferred loadings (expressed in terms of the weight percent of atomoxetine relative to total atomoxetine and polymer) are about 15% to about 80%, more preferably about 30% to about 80%, and most preferably in certain cases about 30% to about 70%. For certain applications, however, the benefits will be obtained with atomoxetine loadings of 0.01% to 80%, and preferably 15% to 80%.

As indicated above, the dosage forms find their greatest utility when administered to a subject who is in the digestive state (also referred to as the postprandial or "fed" mode). The postprandial mode is distinguishable from the interdigestive (or "fasting") mode by their distinct patterns of gastroduodenal motor activity, which determine the gastric retention or gastric transit time of the stomach contents.

### COMBINATIONS

In addition to the instances where atomoxetine is the only active agent, the disclosure includes combination dosage forms that also contain other active agents useful in the treatment of conditions such as ADD and/ or ADHD. Other active agents useful in the treatment of these disorders include, but are not limited to stimulants, mood stabilizers, tricyclic antidepressants, bupropion, alpha-adrenergic antagonists, and selective serotonin reuptake inhibitors.

Thus the disclosure includes combination dosage forms that contain atomoxetine and at least one stimulant, such as methylphenidate, dextroamphetamine, amphetamine, or pemoline. Thus the disclosure includes combination dosage forms that contain atomoxetine and at least one tricyclic antidepressant such as desipramine, imipramine, nortryptiline, amitriptyline, doxepin, or clomipramine. The disclosure included combination dosage forms containing atomoxetine and bupropion, an antidepressent. The disclosure also includes combination dosage forms that contain atomoxetine and at least one alpha-adrenergic antagonist, such as clonidine or guanfacine. The disclosure includes combination dosage forms that contain atomoxetine and at least one mood stabilizers such as lithium, valproate, or carbamazepine. The disclosure includes combination dosage forms that contain atomoxetine and at least one selective serotonin reuptake inhibitor, such as paroxetine, sertaline, and fluvoxamine.

Patients presenting with attention deficit disorder are frequently afflicted with other neuropsychiatric disorders, including depression, anxiety, oppositional/ defiant disorder, bipolar disorder, pervasive developmental disorder, and Tourette's syndrome. Thus the disclosure includes combination dosage forms containing atomoxetine and at least one active agent useful the treatment of these disorders.

Instances of the disclosure are directed to compounds containing atomoxetine and a neuroleptic agent such as trifluoperazine, pimozide, flupenthixol, clozepine, chlorpromazine, flupenthixol, fluphenazine decanoate, pipotiazine, risperidone, or haloperidol decanoate, as an additional active agent.

The disclosure also pertains to combination dosage forms containing atomoxetine and an an anxiolytic as the additional active agent Examples of frequently used anxiolytics include benzodiazepines such as lorazepam, chlordiazepoxide, oxazepam, clorazepate, diazepam, and alprazolam.

The disclosure provides dosage forms as described herein, e.g. wax, press-coat, taste-masked, and sprinkle dosage forms, and well as other dosage forms described herein, which contain atomoxetine and at least one additional active agent.

The disclosure also pertains to methods of treating patients suffering from ADD or ADHD which comprise administering and effective amount of a dosage form containing atomoxetine and at least one additional active agent to a patient in need of such treatment.

### DISSOLUTION PROFILES FOR ATOMOXETINE DOSAGE FORMS

The disclosure provides the atomoxetine dosage forms and dosage forms comprising atomoxetine and one or more other active agent described herein formulated so that particular dissolution profiles are achieved.

The dissolution, or release, profile, of the atomoxetine dosage form is obtained by immersing the dosage from in 750 ml of 0.1 N HCl for 2 hours at 37 °C at a speed of 100 rpm and then adding 250 ml of 0.2 M sodium phosphate buffer to the dissolution media to afford at pH of 6.2. Alternatively the atomoxetine release rate data is obtained for a dosage form in USP Apparatus 2 at 50 rpm using 900 ml of either water or 0.1 N HCl as the dissolution media. The dissolution profile can also be measured utilizing the Drug Release Test (724), which incorporates standard test USP (2002) (Test (711)).

In one instance the disclosure provides a dosage form that exhibits a dissolution profile that is substantially identical to that of STRATTERA in the same dissolution media.

The disclosure includes a controlled-release dosage form comprising a pharmaceutically effective amount of atomoxetine or a pharmaceutically acceptable salt thereof and at least one excipient, exhibiting a dissolution profile in a dissolution media, e.g. 0.1 N HCl, such that at 4 hours after combining with the dissolution media 50 to 95% of the atomoxetine or atomoxetine salt is released.

The disclosure also includes a controlled-release atomoxetine dosage form dissolution profile such that at 1 hour after combining the dosage form with a dissolution media, e.g. 0.1 N HCl, 30 to 80% of the atomoxetine or atomoxetine salt is released, at 2 hours after combining the dosage form with the dissolution media 40 to 85% of the atomoxetine or atomoxetine salt is released, at 3 hours after combining the dosage form with the dissolution media 45 to 90% of the atomoxetine or atomoxetine salt is released, and at 4 hours after combining the dosage form with the dissolution media 50 to 95% of the atomoxetine or atomoxetine salt is released.

The disclosure includes a controlled-release atomoxetine dosage form comprising a pharmaceutically effective amount of atomoxetine or a pharmaceutically acceptable salt thereof and at least one excipient, exhibiting a dissolution profile in 0.1 N HCl such that at 16 hours after combining the dosage form with a dissolution media less that 90 % of the atomoxetine or the pharmaceutically acceptable salt thereof is released.

The disclosure also provides a controlled-release dosage form comprising a pharmaceutically effective amount of atomoxetine or a pharmaceutically acceptable salt thereof and at least one excipient, exhibiting a dissolution profile in 0.1 N HCl such that at 1 hour after combining the dosage form with a dissolution media 5 to 15% of the atomoxetine or atomoxetine salt is released, at 2 hours after combining the dosage form with the dissolution media 10 to 25% of the atomoxetine or atomoxetine salt is released, at 4 hours after combining the dosage form with the dissolution media 15 to 35% of the atomoxetine or atomoxetine salt is released, at 8 hours after combining the dosage form with the dissolution media 25 to 50% of the atomoxetine or atomoxetine is released.

Preferably the atomoxetine in the controlled-release dosage form is in the form of atomoxetine hydrochloride.

The disclosure also provides the novel atomoxetine dosage forms, including the atomoxetine wax formulations, press-coat dosage forms, and sprinkle dosage forms having a dissolution profile which substantially mimics that of STRATTERA

### PHARMACOKINETIC PROPERTIES OF A TOMOXETINE DOSAGE FORMS

The disclosure provides the atomoxetine dosage forms and dosage forms comprising atomoxetine and one or more other active agent (combinations) described herein formulated so that particular plasma levels, Cₘₐₓ, Tₘₐₓ, and AUC values are achieved.

In one instance the disclosure provides a dosage form that is bioequivalent dosage to a dosage form of STRATTERA (Astra Zeneca) containing the same weight of atomoxetine. Such a dosage form exhibits Cₘₐₓ and AUC values from time of administration to 24 hours after administration that are from 80 % to 120 % of the Cₘₐₓ and AUC values from time of administration to 24 hours after administration exhibited by a dosage form STRATTERA containing the same weight of atomoxetine under the same conditions.

The plasma concentration of atomoxetine may be determined as follows: Blood samples are taken from patients immediately before dosing and at specified time intervals after administration. The concentration of atomoxetine in the blood sample is quantified using liquid-liquid extraction and HPLC with UV detection. An average of the maximum observed plasma concentration of atomoxetine in a sampling of patients administered with the atomoxetine; usually at least 12 patients would be included in a sampling. The average Cₘₐₓ is of all sampled patients. Alternatively the averaged Cₘₐₓ may be obtained from logarithmically transformed Cₘₐₓ data. In this method Cₘₐₓ values from sampled patients are log-transformed and the analysis is done on the transformed data. For a two-period crossover study, the ANOVA model used to calculate estimates of the error variance and the least square means are identical for both transformed and untransfonned data. The procedural difference comes after the lower and upper 90% confidence intervals are found by formulas based on Student's t-distribution. The lower and upper confidence bounds of the log-transformed data are then back-transformed in order to be expressed on the original scale of the measurement, as the geometric mean.

### Sustained-release dosage forms

Described herein is an oral dosage form comprising atomoxetine or a pharmaceutically acceptable salt thereof in controlled-release form which provides a maximum atomextine plasma concentration (C*ₘₐₓ*) and an atomoxetine plasma concentration at about 48 hours (C*₄₈*) after administration to a patient, for example a human patient, wherein the ratio of C*ₘₐₓ* to C*₄₈* is less than about 4:1.

The disclosure also provides oral dosage form comprising atomoxetine or a pharmaceutically acceptable salt thereof in controlled-release form which provides a maximum atomoxetine plasma concentration (C*ₘₐₓ*) and an atomoxetine plasma concentration at about 24 hours (C*₂₄*), after administration to a patient, for example a human patient, wherein the ratio of C*ₘₐₓ* to C*₂₄* is less than about 4:1.

It is contemplated that the human patient administered with such a sustained-release atomoxetine dosage form is an extensive metabolize of atomoxetine.

The atomoxetine sustained-release dosage form of any one of Claims 1, 20, or 37 which may when administered to a human patient, provide a maximum atomextine plasma concentration (C*ₘₐₓ*) and an atomoxetine plasma concentration at about 48 hours after administration (C*₄₈*), wherein the ratio of C*ₘₐₓ* to C*₄₈* is less than about 4:1.

Also described herein are atomoxetine wax, press-coat, and sprinkle dosage forms, as described above which, when administered to a human patient provides a maximum atomextine plasma concentration (C*ₘₐₓ*) and an atomoxetine plasma concentration at about 24 hours after administration (C*₂₄*), wherein the ratio of C*ₘₐₓ* to C*₂₄* is less than about 4:1.

Preferably the atomoxetine in the sustained-release dosage forms describes herein is in the form of atomoxetine hydrochloride.

The disclosure provides sustained-release dosage forms as described above wherein the the ratio (e.g. the ratio of C*ₘₐₓ* to C*₂₄* or C*ₘₐₓ* to C*₄₈*) is achieved at steady-state.

The disclosure also provides atomoxetine sustained-release oral dosage forms comprising atomoxetine or a pharmaceutically acceptable salt thereof in controlled-release form, which, at steady-state, provides a maximum atomoxetine plasma concentration (C*ₘₐₓ*), an atomoxetine plasma concentration at about 12 hours after administration (C*₁₂*), and an atomoxetine plasma concentration at about 24 hours after administration (C*₂₄*), wherein the average atomoxetine plasma concentration between C*ₘₐₓ* and C*₁₂* is substantially equal to the average atomoxetine plasma concentration between C*₁₂* and C_{*2*4}.

Certain of these sustained-release dosage forms when administered to a human patient, at steady-state, provide a maximum atomoxetine plasma concentration (C*ₘₐₓ*), an atomoxetine plasma concentration at about 12 hours after administration (C*₁₂*), and an atomoxetine plasma concentration at about 24 hours after administration (C*₂₄*), wherein the average atomoxetine plasma concentration between C*ₘₐₓ* and C*₁₂* is substantially equal to the average atomoxetine plasma concentration between C*₁₂* and C*₂₄*.

The disclosure also provides atomoxetine wax, press-coat, and sprinkle dosage forms as described above which when administered to a human patient provide a maximum atomoxetine plasma concentration (C*ₘₐₓ*), an atomoxetine plasma concentration at about 12 hours after administration (C*₁₂*), and an atomoxetine plasma concentration at about 24 hours after administration (C*₂₄*), wherein the average atomoxetine plasma concentration between C*ₘₐₓ* and C*₁₂* is substantially equal to the average atomoxetine plasma concentration between C*₁₂* and C*₂₄*. Preferably the atomoxetine in these sustained-release atomoxetine wax, press-coat, and sprinkle dosage forms is in the form of atomoxetine hydrochloride.

In certain of these atomoxetine sustained-release wax, press-coat, and sprinkle dosage forms provide a C*ₘₐₓ* at between 5.5 and 12 hours after administration. Certain other of these atomoxetine wax, press-coat, and sprinkle dosage forms provide a C*ₘₐₓ* at between 2 and 3.5 hours after administration.

### Pulsed-release dosage forms

Also described herein are pulsed-release atomoxetine dosage forms, which exhibit characteristic plasma concentration profiles following administration. It is an object of the disclosure to provide an atomoxetine pulsed-released dosage forms that provides an atomoxetine plasma concetration maxima or "pulse" in the morning and another in the afternoon or evening. In certain instances the disclosure provides an atomoxetine dosage form that provides a morning pulse followed by an afternoon pulse, about 5 to 9 hours later, thus providing a second plasma concentration maxima at the end school day without the need for administration at school.

Thus the disclosure provides an atomoxetine pulsed-release oral dosage form comprising atomoxetine or a pharmaceutically acceptable salt thereof in sustained-release form, which, at steady-state, provides a first maximum atomoxetine plasma concentration (*Cₘₐₓ₁*) between 0 hours and about 12 hours after administration, and a second maximum atomoxetine plasma concentration (C*ₘₐₓ₂*) between about 12 hours and about 24 hours after administration, wherein the ratio of C*ₘₐₓ₁* and C*ₘₐₓ₂*.is between about 1:4 and about 4:1.

The disclosure also provides an atomoxetine pulsed-release oral dosage form comprising atomoxetine or a pharmaceutically acceptable salt thereof in pulsed-release form, which, at steady-state, provides a first maximum atomoxetine plasma concentration (C*ₘₐₓ₁*) between 0 hours and about 3 hours after administration, and a second maximum atomoxetine plasma concentration (C*ₘₐₓ₂*) between about 5 hours and about 9 hours after administration, wherein the ratio of C*ₘₐₓ₁* and C*ₘₐₓ₂*.is between about 1:4 and about 4:1.

Preferably the atomoxetine in these dosage forms is in the form of atomoxetine hydrochloride.

The disclosure also provides an atomoxetine pulsed-released oral dosage form as described above which, at steady-state, provides a first maximum atomoxetine plasma concentration (C*ₘₐₓ₁*) between 0 hours and about 12 hours after administration, a second maximum atomoxetine plasma concentration (C*ₘₐₓ₂*) between about 12 hours and about 24 hours after administration, and an atomoxetine plasma concentration at about 24 hours after administration (C*₂₄*), wherein the average atomoxetine plasma concentration between about C*ₘₐₓ₁* and about C*ₘₐₓ₂* is substantially equal to the average atomoxetine plasma concentration between about C*ₘₐₓ₂* and about C*₂₄*. In other embodiments the invention include atomoxetine dosage form wherein the ratio of C*ₘₐₓ₁* to C*ₘᵢₙ₁* is less than about 4:1 1 or the ratio of C*ₘₐₓ₂* to C*₂₄* is less than about 4:1.

The disclosure further provides an atomoxetine pulsed-release oral dosage form as described above, which, at steady-state, provides a first maximum atomoxetine, plasma concentration (C*ₘₐₓ₁*) and a first minimum atomaxetine plasma concentration (C*ₘᵢₙ₁*) between 0 hours and about 5 hours after administration, a second maximum atomoxetine plasma concentration (C*ₘₐₓ₂*) between about 5 hours and about 9 hours after administration, and an atomoxetine plasma concentration at about 24 hours after administration (C*₂₄*), wherein the ratio of C*ₘₐₓ₁* to C*ₘᵢₙ₁* is less than about 4:1 1 or the ratio of C*ₘₐₓ₂* to C*₂₄* is less than about 4: 1.

In certain instances the disclosure pertains to atomoxetine pulsed-release dosage forms, wherein C*ₘₐₓ₂* occurs about 12 to about 14 hours after administration.

In other instances the disclosure pertains to atomoxetine pulsed-release dosage forms, wherein C*ₘₐₓ₁* occurs about 6 to about 8 hours after administration.

The disclosure also includes atomoxetine pulsed-release dosage forms, as described herein, wherein, at steady-state, the difference between the ratio of C*ₘₐₓ₁* to C*ₘᵢₙ₁* and the ratio of C*ₘₐₓ₂* to C₂₄ is less than about 30%, or in certain instances less than about 20%.

The disclosure further provides a sustained-release oral dosage form comprising a first subunit and a second subunit, wherein the first subunit comprises atomoxetine and a first release-retarding material and the second subunit comprises atomoxetine and a second release-retarding material, wherein the first and second release-retarding material can be the same or different, and wherein the dosage form, at steady-state, provides a maximum atomoxetine plasma concentration (C*ₘₐₓ*) and an atomoxetine plasma concentration at about 24 hours after administration (C*₂₄*), wherein the ratio of C*ₘₐₓ* to C*₂₄* is less than about 4: 1.

### Semi-Delayed-release Dosage Forms

Also described herein are semi-delay release dosage forms, in which the peak atomoxetine plasma concentration is attained significantly later after administration than the peak atomoxetine plasma concentration is attained following administration of an immediate-release form of atomoxetine. The disclosure also provides semi-delayed-release dosage from which provide a moderate atomoxetine concentration upon administration, followed by a larger "pulse" atomoxetine plasma concentration some hours after administration. The purpose of such dosage forms is to provide a moderate atomoxetine dosage following A.M. administration, followed by a larger dosage at night.

Thus the disclosure provides an atomoxetine oral dosage form which, at steady-state, provides a first maximum atomoxetine plasma concentration (C*ₘₐₓ₁*) between 0 hours and about 12 hours after administration, and a second maximum atomoxetine plasma concentration (C*ₘₐₓ₂*) between about 12 hours and about 24 hours after administration, wherein the ratio of C*ₘₐₓ₁* to C*ₘₐₓ₂* is greater than 1:1.5 and less than about 1:4, or in certain instances wherein the ratio of C*ₘₐₓ₁* to C*ₘₐₓ₂* is greater than about 1:3 and less than about 1:4.

### Dosage Forms Characterized by AUC

Atomoxetine dosage forms of the disclosure exhibit characteristic plasma concentrations over time. When integrated the graph of plasma concentration over time provides a characteristic "area under the curve" or AUC.

In addition to providing an oral dosage form that provides an AUC between 0 and 24 hours after administration that is more than 80 percent and less than 120 percent of the AUC provided by an equivalent weight of STRATTERA between 0 and 24 hours after administration. The disclosure also provides the following sustained-release atomoxetine dosage forms having a characteristic AUC.

A dosage form of comprising atomoxetine which provides an AUC between 0 and 24 hours after administration that is more than 80 percent and less than 120 percent of the AUC provided by 2 times the equivalent weight of STRATTERA between 0 and 24 hours after administration.

An oral dosage form comprising atomoxetine or a pharmaceutically acceptable salt thereof in sustained-release form, which, at steady-state, provides a first AUC (AUC₁) between 0 and about 12 hours and a second AUC (AUC₂) between about 12 hours and about 24 hours, wherein difference between AUC₂ and AUC₁ is less than about 50%.

In one instance, the oral dosage form has an AUC₁ and AUC₂ that are about equal.

The disclosure provides the particular dosage forms described herein, e.g. atomoxetine wax formulations, press-coat dosage formulation, taste masked formulations, and the like, formulated to provide the atomoxetine plasma concentration profiles and characteristic AUC ratios described above.

### MANUFACTURE OF ATOMOXETINE DOSAGE FORMS

### AMORPHOUS TECHNOLOGY

Amorphous solids consist of disordered arrangements of molecules and do not possess a distinguishable crystal lattice. Atomoxetine may be prepared in such a way that substantially all of atomoxetine is present in amorphous form.

A process for preparing solid, amorphous atomoxetine comprises mixing atomoxetine free base or a salt thereof with a solvent such as water and a pharmaceutically acceptable polymeric carrier; and drying to form a composition comprising amorphous atomoxetine and polymeric carrier.

In another aspect, a pharmaceutical composition comprises atomoxetine salt in amorphous, solid form, and polymeric carrier, prepared by the aforementioned process.

Suitable pharmaceutically acceptable polymeric carriers include, for example, hydroxypropyl cellulose, methyl cellulose, carboxymethyl cellulose, sodium carboxymethyl cellulose, cellulose acetate phthalate, cellulose acetate butyrate, hydroxyethyl cellulose, ethyl cellulose, polyvinyl alcohol, polypropylene, dextrans, dextrins, hydroxypropyl-beta-cyclodextrin, chitosan, co(lactic/glycolid) copolymers, poly(orthoester), poly(anhydrate), polyvinyl chloride, polyvinyl acetate, ethylene vinyl acetate, lectins, carbopols, silicon elastomers, polyacrylic polymers, maltodextrins, polyvinylpyrrolidone (PVP), polyethylene glycol (PEG), and alpha-, beta-, and gamma-cyclodextrins, crospovidone, and combinations comprising one or more of the foregoing carriers.

Preferred polymeric carriers are one or more of polyvinylpyrrolidone, hydroxypropylmethyl cellulose, hydroxypropyl cellulose, methyl cellulose, block copolymers of ethylene oxide and propylene oxide, and polyethylene glycol, wherein a more preferred polymeric carrier is polyvinylpyrrolidone (PVP) having an average molecular weight of about 2,500 to about 3,000,000. A most preferred polymeric carrier is polyvinylpyrrolidone having an average molecular weight of from about 10,000 to about 450,000.

The polymeric carrier is preferably miscible with both atomoxetine free base and the salt, capable of keeping the salt in a homogeneous noncrystalline solid state dispersion after the solvent has been removed by evaporation and chemically inert with respect to the free base of the active ingredient, the salt of the free base, and the acid solution.

Atomoxetine may be added in either free base or salt form. When atomoxetine is added in free base form, the process comprises adding an acid corresponding to a salt of atomoxetine to the mixture or solution of the free base. The free base is then converted to a salt *in situ,* for example by addition of an inorganic or an organic acid. The acid may be added either as a gas, a liquid or as a solid dissolved into a solvent. A preferred acid is fumaric acid and the molar quantity of acid added to the solution of atomoxetine free base and carrier may either be in stoichiometric proportion to atomoxetine free base or be in excess of the molar quantity of atomoxetine free base, especially when added as a gas.

The preferred range of acid added is about 0.5 to about 1.8 times the molar quantity of atomoxetine free base. Preferred molar ratios of atomoxetine to fumaric acid are about 2:1. It is understood that upon addition of the acid, the formed free base salt remains dissolved in solution with the polymeric carrier.

Atomoxetine, polymeric carrier, and solvent may be combined in any order. It is preferred that they be combined in a manner so as to form a solution of atomoxetine salt and the polymeric carrier.

In forming a solution of polymeric carrier and solvent, heating of the solution is not necessary at lower concentrations but is strongly preferred at higher concentrations, provided that the temperature does not result in decomposition or degradation of any materials. It is preferred to add atomoxetine free base or salt after dissolving the polymeric carrier in the solvent, suitably at about 25° to about 100°C, preferably at about 45° to about 80°C. When atomoxetine is added as a free base, it is preferred to form a salt at a temperature at which the final solution is clear. For the most preferred instances, a temperature of at least about 60°C may result in a clear solution of atomoxetine salt being formed, although for other concentrations and instances, clear solutions are formed at other temperatures. It is preferred to only add enough heat to form a clear solution.

The ratio of atomoxetine to the polymeric carrier can be varied over a wide range and depends on the concentration of atomoxetine required in the pharmaceutical dosage form ultimately administered. The ratio by weight of polymeric carrier to atomoxetine salt is about 20:1 to about 0.5:1; preferably about 4:1 to about 1:1; more preferably about 3:1 to about 1.5:1; most preferably about 2:1.

Preferably a clear solution is formed. Upon formation of the clear solution, the process proceeds by recovering the solvent to form a solid state dispersion of the free base salt in the polymeric carrier. Any method of removal of the solvent which renders a homogeneous solid state dispersion is intended, although preferred are methods of evaporation under vacuum or spray drying. Methods of evaporation under vacuum include rotary evaporation, static vacuum drying and the combination thereof. It is understood that one skilled in the art of pharmaceutical formulations can determine a reasonable temperature at which the solvent can be removed, provided the temperature is not so high as to cause degradation or decomposition of the materials; however, it is preferred that evaporation occurs at about 25 °C to about 100 °C. Evaporation of the solvent should render a solid state dispersion which is homogeneous and substantially free of water. By substantially free it is meant that the solid state dispersion contains less than 20% by weight of residual solvent, preferably less than 10%, more preferably less than 5%, most preferably less than 1%.

The ratio of atomoxetine free base to the polymeric carrier can be varied over a wide range and depends on the atomoxetine concentration required in the pharmaceutical dosage form ultimately administered. However, the preferred range of atomoxetine in the solid dispersion is about 10% to about 50% of the total solid dispersion weight, more preferable is about 20% to about 50%, even more preferable is about 25% to about 40%, most preferable is about 33% of the total dispersion weight. In terms of weight ratio of polymeric carrier to atomoxetine, a preferred range is about 0.4:1 to 20:1.

Suitable pharmaceutically acceptable excipients can be added in the process. Examples of pharmaceutically acceptable excipients include diluents, binders, disintegrants, coloring agents, flavoring agents, lubricants and/or preservatives. The pharmaceutical composition may be formulated by conventional methods of admixture such as blending, filling, granulation and compressing. These agents may be utilized in conventional manner.

### OPTIONAL ADDITIONAL ADDITIVE FOR ATOMOXETINE FORMULATIONS

### EXCIPIENTS

Excipients are components added to active agent pharmaceutical formulation other than atomoxetine, and include inert substances used as a diluent or vehicle for atomoxetine. Excipients may be added to facilitate manufacture, enhance stability, control release, enhance product characteristics, enhance bioavailability, enhance patient acceptability, etc. Pharmaceutical excipients include binders, disintegrants, lubricants, glidants, compression aids, colors, sweeteners, preservatives, suspending agents, dispersing agents, film formers, flavors, printing inks, etc. Binders hold the ingredients in the dosage form together. Exemplary binders include, for example, polyvinyl pyrrolidone, hydroxypropyl cellulose, hydroxypropyl methylcellulose, methylcellulose and hydroxyethyl cellulose, sugars, and combinations comprising one or more of the foregoing binders. Disintegrants expand when wet causing a tablet to break apart. Exemplary disintegrants include water swellable substances, for example, low-substituted hydroxypropyl cellulose, e.g. L-HPC; cross-linked polyvinyl pyrrolidone (PVP-XL), e.g. Kollidon® CL and Polyplasdone® XL; cross-linked sodium carboxymethylcellulose (sodium croscarmellose), e.g. Ac-di-sol®, Primellose®; sodium starch glycolate, e.g. Primojel®; sodium carboxymethylcellulose, e.g. Nymcel ZSB10®; sodium carboxymethyl starch, e.g. Explotab®; ion-exchange resins, e.g. Dower® or Amberlite®; microcrystalline cellulose, e.g. Avicel®; starches and pregelatinized starch, e.g. Starch 1500®, Sepistab ST200 ®; formalin-casein, e.g. Plas-Vita®, and combinations comprising one or more of the foregoing water swellable substances. Lubricants, for example, aid in the processing of powder materials. Exemplary lubricants include calcium stearate, glycerol behenate, magnesium stearate, mineral oil, polyethylene glycol, sodium stearyl fumarate, stearic acid, talc, vegetable oil, zinc stearate, and combinations comprising one or more of the foregoing lubricants. Glidants include, for example, silicon dioxide.

### FILLERS

Certain dosage forms described herein contain a filler, such as a water insoluble filler, water soluble filler, and combinations thereof. The filler may be a water insoluble filler, such as silicon dioxide, titanium dioxide, talc, alumina, starch, kaolin, polacrilin potassium, powdered cellulose, microcrystalline cellulose, and combinations comprising one or more of the foregoing fillers. Exemplary water-soluble fillers include water soluble sugars and sugar alcohols, preferably lactose, glucose, fructose, sucrose, mannose, dextrose, galactose, the corresponding sugar alcohols and other sugar alcohols, such as mannitol, sorbitol, xylitol, and combinations comprising one or more of the foregoing fillers.

### PREPARATION OF ATOMOXETINE-CONTAINING SUBUNITS

Atomoxetine and any optional additives may be prepared in many different ways, for example as subunits. Pellets comprising an active ingredient can be prepared, for example, by a melt pelletization technique. In this technique, the active ingredient in finely divided form is combined with a binder and other optional inert ingredients, and thereafter the mixture is pelletized, e.g., by mechanically working the mixture in a high shear mixer to form the pellets (e.g., pellets, granules, spheres, beads, etc., collectively referred to herein as "pellets"). Thereafter, the pellets can be sieved in order to obtain pellets of the requisite size. The binder material may also be in particulate form and has a melting point above about 40°C. Suitable binder substances include, for example, hydrogenated castor oil, hydrogenated vegetable oil, other hydrogenated fats, fatty alcohols, fatty acid esters, fatty acid glycerides, and the like, and combinations comprising one or more of the foregoing binders.

Oral dosage forms may be prepared to include an effective amount of melt-extruded subunits containing atomoxetine or other active agents in the form of multiparticles within a capsule. For example, a plurality of the melt-extruded muliparticulates can be placed in a gelatin capsule in an amount sufficient to provide an effective release dose when ingested and contacting by gastric fluid.

Subunits, e.g., in the form of multi particulates, can be compressed into an oral tablet using conventional tableting equipment using standard techniques. The tablet formulation may include excipients such as, for example, an inert diluent such as lactose, granulating and disintegrating agents such as cornstarch, binding agents such as starch, and lubricating agents such as magnesium stearate.

Alternatively, the subunits containing atomoxetine and optionally containing additional active agents are added during the extrusion process and the extrudate can be shaped into tablets by methods know in the art. The diameter of the extruder aperture or exit port can also be adjusted to vary the thickness of the extruded strands. Furthermore, the exit part of the extruder need not be round; it can be oblong, rectangular, etc. The exiting strands can be reduced to particles using a hot wire cutter, guillotine, etc.

A melt-extruded multiparticulate system can be, for example, in the form of granules, spheroids, pellets, or the like, depending upon the extruder exit orifice. The terms "melt-extruded multiparticulate(s)" and "melt-extruded multiparticulate system(s)" and "melt-extruded particles" are used interchangeably herein and include a plurality of subunits, preferably within a range of similar size and/or shape. The melt-extruded multiparticulates can be about 0.1 to about 12 mm in length and have a diameter of about 0.1 to about 5 mm. In addition, the melt-extruded multiparticulates can be any geometrical shape within this size range. Alternatively, the extrudate can simply be cut into desired lengths and divided into unit doses of atomoxetine without the need of a spheronization step.

The melt-extruded dosage forms can further include combinations of melt-extruded multiparticulates containing one or more of the therapeutically active agents before being encapsulated. Furthermore, the dosage forms can also include an amount of atomoxetine formulated for immediate-release for prompt therapeutic effect. Atomoxetine formulated for immediate-release can be incorporated or coated on the surface of the subunits after preparation of the dosage forms (e.g., controlled-release coating or matrix-based). The dosage forms can also contain a combination of controlled-release beads and matrix multiparticulates to achieve a desired effect.

A melt-extruded material may be prepared without the inclusion of subunits containing atomoxetine, which are added thereafter to the extrudate. Such formulations have the subunits and other active agents blended together with the extruded matrix material. The mixture is then tableted in order to provide release of atomoxetine or other active agents. Such formulations can be particularly advantageous, for example, when an active agent included in the formulation is sensitive to temperatures needed for softening the hydrophobic material and/or the retardant material.

The oral dosage form containing atomoxetine may be in the form of micro-tablets enclosed inside a capsule, e.g. a gelatin capsule. For this, a gelatin capsule as is employed in pharmaceutical formulations can be used, such as the hard gelatin capsule known as CAPSUGEL, available from Pfizer.

### ATOMOXETINE PARTICLES

Many of the oral dosage forms described herein contain atomoxetine and optionally additional active agents in the form of particles. Such particles may be compressed into a tablet, present in a core element of a coated dosage form, such as a taste masked dosage form, a press coated dosage form, or an enteric coated dosage form, or may be contained in a capsule, osmotic pump dosage form, or other dosage form.

For particles, such as powder particles, present in the core element of a coated dosage form, the core element may have a particle size distribution with a median of about 100 µm. The particles in the distribution may vary from about 1 µm to about 250 µm, more preferably from 25 µm to about 250 µm, most preferably about 35 µm to about 125 µm. If the median of the distribution is close to either extreme of the distribution, the taste masking or sustained-release characteristics may be affected. In a particle size range of about 25 µm to about 250 µm, no more than about 25% of particles can be less than about 25 µm, and no more than about 25% can be over about 250 µm.

Another parameter to consider is particle shape. Particle shape can influence the coverage and stability of the coat. Both the crystallinity of atomoxetine and the aspect ratio of the particles are related to particle shape. It is preferred that atomoxetine of the coated dosage forms has a crystalline morphology, however, sharp angles on a crystal can cause weaknesses in the coat. These sharp corners may lead to stress points on the coat and cause weaknesses in the structure possibly leading to premature release of atomoxetine from the dosage form. Furthermore, areas of thin coating are susceptible to breaking and cracking and hence ineffective for sustained-release and taste masking.

Regarding the aspect ratio, a low aspect ratio is preferred. The aspect ratio is a measure of the length to breadth. For example, a low aspect ratio of about 1 would be a box or sphere. Crystals with a high aspect ratio are more pointed with needle-like crystals. Crystals with a high aspect ratio may result in a relatively thin coat at the crystal needle tips leading to a more rapid release rate of atomoxetine than is preferred. A low aspect ratio spherical shape of the particle is advantageous for both solubility of the coat and high payload of atomoxetine. Therefore, it is most preferable that the aspect ratio is less than about 3, more preferably about 1 to about 2, and most preferably approximately 1 providing a substantially rounded shape.

Inconsistencies in size and shape can lead to inconsistent coating. Where the particles containing atomoxetine are of different size and shape, polymeric coating materials such as ethyl cellulose may deposit differently on each particle. It is therefore preferable for coated dosage forms that substantially all particles of the dosage form have substantially the same size and shape so that the coating process is better controlled and maintained.

### COATINGS

The formulations described herein may be coated with a functional or non-functional coating. The coating may comprise about 0 to about 40 weight percent of the composition. The coating material may include a polymer, preferably a film-forming polymer, for example, methyl cellulose, ethyl cellulose, hydroxypropyl cellulose, hydroxypropyl methyl cellulose, hydroxybutyl methyl cellulose, cellulose acetate, cellulose propionate (lower, medium or higher molecular weight), cellulose acetate propionate, cellulose acetate butyrate, cellulose acetate phthalate, carboxymethyl cellulose, cellulose triacetate, cellulose sulphate sodium salt, poly(methyl methacrylate), poly (ethyl methacrylate), poly (butyl methacrylate), poly (isobutyl methacrylate), poly (hexyl methacrylate), poly (phenyl methacrylate), poly (methyl acrylate), poly (isopropyl acrylate), poly (isobutyl acrylate), poly (octadecyl acrylate), poly (ethylene), poly (ethylene) low density, poly (ethylene)high density, (poly propylene), poly (ethylene glycol poly (ethylene oxide), poly (ethylene terephthalate), poly(vinyl alcohol), poly(vinyl isobutyl ether), poly(viny acetate), poly (vinyl chloride), polyvinyl pyrrolidone, and combinations comprising one or more of the foregoing polymers.

In applications such as taste-masking, the polymer can be a water-insoluble polymer. Water insoluble polymers include ethyl cellulose or dispersions of ethyl cellulose, acrylic and/or methacrylic ester polymers, cellulose acetates, butyrates or propionates or copolymers of acrylates or methacrylates having a low quaternary ammonium content, and the like, and combinations comprising one or more of the foregoing polymers.

In controlled-release applications, for example, the coating can be a hydrophobic polymer that modifies the release properties of atomoxetine from the formulation. Suitable hydrophobic or water insoluble polymers for controlled-release include, for example, methacrylic acid esters, ethyl cellulose, cellulose acetate, polyvinyl alcohol-maleic anhydride copolymers, β-pinene polymers, glyceryl esters of wood resins, and combinations comprising one or more of the foregoing polymers.

The inclusion of an effective amount of a plasticizer in the coating composition may improve the physical properties of the film. For example, because ethyl cellulose has a relatively high glass transition temperature and does not form flexible films under normal coating conditions, it may be advantageous to add plasticizer to the ethyl cellulose before using the same as a coating material. Generally, the amount of plasticizer included in a coating solution is based on the concentration of the polymer, e.g., most often from about 1 to about 50 percent by weight of the polymer. Concentrations of the plasticizer, however, can be determined by routine experimentation.

Examples of plasticizers for ethyl cellulose and other celluloses include plasticizers such as dibutyl sebacate, diethyl phthalate, triethyl citrate, tributyl citrate, triacetin, and combinations comprising one or more of the foregoing plasticizers, although it is possible that other water-insoluble plasticizers (such as acetylated monoglycerides, phthalate esters, castor oil, etc.) can be used.

Examples of plasticizers for acrylic polymers include citric acid esters such as triethyl citrate 21, tributyl citrate, dibutyl phthalate, 1,2-propylene glycol, polyethylene glycols, propylene glycol, diethyl phthalate, castor oil, triacetin, and combinations comprising one or more of the foregoing plasticizers, although it is possible that other plasticizers (such as acetylated monoglycerides, phthalate esters, castor oil, etc.) can be used.

An example of a functional coating comprises a coating agent comprising a poorly water-permeable component (a) such as, an alkyl cellulose, for example an ethylcellulose, such as AQUACOAT (a 30% dispersion available from FMC, Philadelphia, PA) or SURELEASE (a 25% dispersion available from Colorcon, West Point, PA) and a water-soluble component (b), e.g., an agent that can form channels through the poorly water-permeable component upon the hydration or dissolution of the soluble component. Preferably, the water-soluble component is a low molecular weight, polymeric material, e.g., a hydroxyalkylcellulose, hydroxyalkyl(alkylcellulose), and carboxymethylcellulose, or salts thereof. Particular examples of these water soluble polymeric materials include hydroxyethylcellulose, hydroxypropylcellulose, hydroxyethylmethylocellulose, hydroxypropylmethylcellulose, carboxymethylcellulose, sodium carboxymethylcellulose, and combinations comprising one or more of the foregoing materials. The water-soluble component can comprise hydroxypropylmethylcellulose, such as METHOCEL. The water-soluble component is preferably of relatively low molecular weight, preferably less than or equal to about 25,000 molecular weight, or preferably less than or equal to about 21,000 molecular weight.

In the functional coating, the total of the water soluble portion (b) and poorly water permeable portion (a) are present in weight ratios (b):(a) of about 1:4 to bout 2:1, preferably about 1:2 to about 1:1, and more preferably in a ratio of about 2:3. While the ratios disclosed herein are preferred for duplicating target release rates of presently marketed dosage forms, other ratios can be used to modify the speed with which the coating permits release of atomoxetine. The functional coating may comprise about 1% to about 40%, preferably about 3% to about 30%, more preferably about 5% to about 25%, and yet more preferably about 6% to about 10% of the total formulation. For pellet formulations it is preferred that the coating comprise from about 1% to about 40%, or from about 5% to about 25%, or from about 10% to about 25% of the total pellet weight. For tablet formulations the coating may comprise from about 5% to about 40%, or from about 5% to about 10%, or about 6% of the total formulation.

In certain instances, particularly where the coating provides taste masking, it is preferred that the coating is substantially continuous coat and substantially hole-free. By "substantially continuous coating" is meant a coating, which retains a smooth and continuous appearance when magnified 1000 times under a scanning electron microscope and wherein no holes or breakage of the coating are evident.

Suitable methods can be used to apply the coating to atomoxetine. Processes such as simple or complex coacervation, interfacial polymerization, liquid drying, thermal and ionic gelation, spray drying, spray chilling, fluidized bed coating, pan coating, electrostatic deposition, may be used. A substantially continuous nature of the coating may be achieved, for example, by spray drying from a suspension or dispersion of atomoxetine in a solution of the coating composition including a polymer in a solvent in a drying gas having a low dew point.

When a solvent is used to apply the coating, the solvent is preferably an organic solvent that constitutes a good solvent for the coating material, but is substantially a non-solvent or poor solvent for of atomoxetine. While atomoxetine may partially dissolve in the solvent, it is preferred that the active ingredient will precipitate out of the solvent during the spray drying process more rapidly than the coating material. The solvent may be selected from alcohols such as methanol, ethanol, halogenated hydrocarbons such as dichloromethane (methylene chloride), hydrocarbons such as cyclohexane, and combinations comprising one or more of the foregoing solvents. Dichloromethane (methylene chloride) has been found to be particularly suitable.

The concentration of polymer in the solvent will normally be less than about 75% by weight, and typically about 10 to about 30% by weight. After coating, the coated dosage forms may be allowed to cure for at least about 1 to about 2 hours at a temperature of about 50 °C to about 60 °C, more preferably of about 55 °C.

The coatings may be about 0.005 micrometers to about 25 micrometers thick, preferably about 0.05 micrometers to about 5 micrometers.

### PREPARATION OF ATOMOXETINE DOSAGE FORMS

The term "dosage form" denotes a form of a formulation that contains an amount sufficient to achieve a therapeutic effect with a single administration. When the formulation is a tablet or capsule, the dosage form is usually one such tablet or capsule. The frequency of administration that will provide the most effective results in an efficient manner without overdosing will vary with the characteristics of the particular atomoxetine formulation, including both its pharmacological characteristics and its physical characteristics such as solubility, and with the characteristics of the swellable matrix such as its permeability, and the relative amounts of the drug and polymer. In most cases, the dosage form will be such that effective results will be achieved with administration no more frequently than once every eight hours or more, preferably once every twelve hours or more, and even more preferably once every twenty- four hours or more.

The dosage form can be prepared by various conventional mixing, comminution and fabrication techniques readily apparent to those skilled in the chemistry of drug formulations. Examples of such techniques are as follows:
(1) Direct compression, using appropriate punches and dies; the punches and dies are fitted to a suitable rotary tableting press;
(2) Injection or compression molding using suitable molds fitted to a compression unit
(3) Granulation followed by compression; and
(4) Extrusion in the form of a paste, into a mold or to an extrudate to be cut into lengths.

When particles are made by direct compression, the addition of lubricants may be helpful and sometimes important to promote powder flow and to prevent capping of the particle (breaking off of a portion of the particle) when the pressure is relieved. Useful lubricants are magnesium stearate (in a concentration of from 0.25% to 3% by weight, preferably less than 1% by weight, in the powder mix), and hydrogenated vegetable oil (preferably hydrogenated and refined triglycerides of stearic and palmitic acids at about 1% to 5% by weight, most preferably about 2% by weight. Additional excipients may be added to enhance powder flowability and reduce adherence.

### ATOMOXETINE-CONTAINING PELLETS IN CAPSULES

Oral dosage forms may be prepared to include an effective amount of melt-extruded subunits in the form of multiparticles within a capsule. For example, a plurality of the melt-extruded muliparticulates can be placed in a gelatin capsule in an amount sufficient to provide an effective release dose when ingested and contacted by gastric fluid.

### ATOMOXETINE-CONTAINING TABLETS IN CAPSULES

The composition may be in the form of micro-tablets enclosed inside a capsule, e.g. a gelatin capsule. For this, a gelatin capsule employed in the pharmaceutical formulation field can be used, such as the CAPSUGEL hard gelatin capsule, available from Pfizer.

### EXAMPLES

The following examples further illustrate the invention but, of course, should not be construed as in any way limiting its scope.

### EXAMPLE 1. ATOMOXETINE HYDROCHLORIDE WAX FORMULATION IN TABLET FORM

Atomoxetine hydrochloride tablet cores of the following formulation are prepared as follows (the Table I presents the formulas in % by weight, while Table II presents the same formulas in mg amounts per 500 mg atomoxetine hydrochloride dosage form):

| TABLE I | | | | | |
|---|---|---|---|---|---|
| Component | Formula 1 (wt. %) | Formula 2 (wt. %) | Formula 3 (wt. %) | Formula 4 (wt. %) | Formula 5 (wt. %) |
| Matrix | | | | | |
| Atomoxetine hydrochloride | 81.97 | 73.86 | 70.72 | 66.00 | 67.68 |
| Carnauba wax | 16.39 | 24.67 | 28.29 | 33.00 | 27.07 |
| Glyceryl Dibehenate | | | | | 4.06 |
| | | | | | |
| Processing aids | | | | | |
| Hydrophobic colloidal silicon dioxide (CAB-O-SIL M5) | | | | | 0.11 |
| Magnesium Stearate | 1.64 | 1.47 | 0.99 | 1.00 | 1.08 |
| Total Core | 100.00 | 100.00 | 100.00 | 100.00 | 100.00 |
| Matrix | | | | | |
| Atomoxetine hydrochloride | 45.7 | 45.7 | 45.7 | 45.7 | 45.7 |
| Carnauba wax | 12.5 | 21.25 | 25.0 | 31.25 | 25.0 |
| Processing aids | | | | | |
| Hydrophobic colloidal silicon dioxide (CAB-O-SIL M5) | | | | | 0.1 |
| Magnesium Stearate | 1.25 | 1.25 | 0.88 | 0.94 | 1.0 |
| Total Core | 59.5 | 68.2 | 71.6 | 77.9 | 71.8 |

Atomoxetine hydrochloride is mixed with carnauba wax and hot melt granulated. The granulate is then milled and the magnesium stearate and colloidal silicon dioxide processing aids are added and blended. The blend is then compressed.

### EXAMPLE 2. ATOMOXETINE HYDROCHLORIDE COATED WAX FORMULATION

Cores of Formula 3 in Example 1 above are coated at 35°C to 40°C with a functional coating solution comprising 5% OPADRY II Yellow (Colorcon), 20% SURELEASE (Colorcon), and 75% deionized water. The coating is applied so that the coating comprises 2%, 4%, or 6% of the total formulation.

### EXAMPLE 3. ATOMOXETINE HYDROCHLORIDE COATED WAX FORMULATION (CURED)

Cores of Formula 2 above (approximately 170 mg of carnauba wax/core) are coated with a functional coating solution comprising 5% OPADRY II Yellow (Colorcon, West Point, PA), 20% SURELEASE (Colorcon, West Point, PA), and 75% deionized water as in Example 2. The coating is applied so that the coating is 6% of the final formulation. A portion of the coated cores is cured for 1 hour at 55 °C.

### EXAMPLE 4. COATED ATOMOXETINE HYDROCHLORIDE WAX PREPARATION CONTAINING A PROCESSING AID

A further example of the invention includes the formulation set forth below.

| TABLE III | | |
|---|---|---|
| Component | | |
| Matrix | % total formulation | Weight (mg) |
| Atomoxetine Hydrochloride | 45.7 | 68.4 |
| Carnauba wax | 21.89 | 38.8 |
| Processing aids | | |
| Hydrophobic colloidal silicon dioxide (CAB-O-SIL M5) | 0.14 | 0.2 |
| Magnesium Stearate | 0.97 | 1.40 |
| | | |
| Coating | | |
| Opadry clear | 2.26 | 3.24 |
| Surelease Coat | 3.39 | 4.86 |
| Total coating | 5.65 | 8.10 |
| Total Tablet | 80.00 | 125.00 |

Atomoxetine hydrochloride is mixed with the carnauba wax and hot melt granulated. The granulate is then milled and magnesium stearate and colloidal silicon dioxide processing aids are added and blended. The mixture is compressed. The compressed (as yet uncoated) tablets are then coated with a blend of the coating ingredients at 35 °C to 40 °C and the tablets are cured for 1 to 2 hours at 55 °C.

Tablets of the invention are tested when freshly prepared and after storage at 40°C for 1, 2 or 3 months.

### EXAMPLE 5. PRESS-COATED TABLETS

Press-coated tablets are prepared from cores of the following formulation:

| Component (mg/ tablet) | |
|---|---|
| Atomoxetine hydrochloride | 22.8 |
| Dicalcium Phosphate | 6.9 |
| Carnauba Wax | 10.0 |
| CAB-O-SIL (silica) | 0.1 |
| Magnesium Stearate | 0.2 |
| Total Core | 40.0 |

The components are blended together and compressed to form a core composition. The core composition is then press-coated with the composition below:

| Component (mg/ tablet) | |
|---|---|
| Atomoxetine hydrochloride | 22.8 |
| Lactose monohydrate | 87.2 |
| METHOCEL K4M | 48.0 |
| CAB-O-SIL (silica) | 0.5 |
| Magnesium Stearate | 1.5 |
| Total Press Coat | 160.0 |

The press-coat composition components are blended together and about one half of the blend (about 100 mg) is placed in a die and lightly pressed. A core composition, as prepared above, is placed at approximately the center of the die and covered with the other half of the press-coat composition and pressed in the die to form a tablet. Some tablets are then given a further immediate-release "loading dose" of an additional 10 mg of atomoxetine hydrochloride per tablet.

Atomoxetine hydrochloride release rate data is obtained for these tablets in USP Apparatus 2 at 50 rpm using 900 ml of either water or 0.1 N HCl as the dissolution media.

### EXAMPLE 6. PRESS COATED FORMULATION CONTAINING HPMC

The cores of Example 5 are used with a press-coat composition containing (per tablet):

| TABLE IV | | | |
|---|---|---|---|
| | 15% HPMC | 20% HPMC | 40% HPMC |
| Component | mg per tablet | mg per tablet | mg per tablet |
| Atomoxetine hydrochloride | 45.7 | 45.7 | 45.7 |
| Lactose Monohydrate | 222.3 | 196.3 | 130.2 |
| METHOCEL K4M | 48 | 64.0 | 130.1 |
| CAB-O-SIL (silica) | 1 | 1.0 | 1 |
| Magnesium Stearate | 3 | 3.0 | 3 |
| Total Press-coat | 320 | 320.0 | 320 |

Tablets are prepared as in Example 5 (except that no loading dose was added) and dissolution profiles are measured as in Example 5.

### Example 7. Press Coated Tablets Having Fixed Core: Coat Ratios

The cores of Example 5 are used with a press-coat composition comprising 12 mg of atomoxetine hydrochloride to provide a Core_{AA}:Coat_{AA} ratio of 4:3 (Example 7A). Additional atomoxetine hydrochloride formulations are prepared as set forth below to provide Core_{AA}:Coat_{AA} ratio of 1:3 (Example 7B) and 3:1 (Example 7C). Atomoxetine dissolution is tested in 0.1 N HCl.

| TABLE V | | | |
|---|---|---|---|
| Core (mg/ tablet): | | | |
| Component | Example 7A (4:3) | Example 7B (1:3) | Example 7C (3:1) |
| Atomoxetine hydrochloride | 26.1 | 11.4 | 34.3 |
| Dicalcium Phosphate | 27.4 | 42.9 | 17.9 |
| Carnauba Wax | 25.9 | 25.1 | 27.2 |
| CAB-O-SIL (silica) | 0.2 | 0.2 | 0.2 |
| Magnesium Stearate | 0.4 | 0.4 | 0.4 |
| Total Core | 80.0 | 80.0 | 80.0 |

| Press-coating (mgs/ tablet): | | | |
|---|---|---|---|
| Atomoxetine hydrochloride | 19.6 | 34.3 | 11.4 |
| Lactose Monohydrate | 240.4 | 225.7 | 249.4 |
| METHOCEL K4M | 56.0 | 56.0 | 56.0 |
| CAB-O-SIL (silica) | 1.0 | 1.0 | 1.0 |
| Magnesium Stearate | 3.0 | 3.0 | 3.0 |
| Total Press-coat | 320.0 | 320.0 | 320.0 |

### EXAMPLE 8. PRESS COATED FORMULATION CONTAINING METHOCEL K100M

A press coated atomoxetine hydrochloride formulation is prepared utilizing METHOCEL K100M mannitol and a Core_{AA}: Coat_{AA} ratio of 4:1. Dissolution data is obtained using two rotation speeds.

| Core Component (mg/tablet) | |
|---|---|
| Atomoxetine hydrochloride | 36.6 |
| Dicalcium Phosphate | 17.2 |
| Carnauba Wax | 15.7 |
| Magnesium Stearate | 0.5 |
| Total Core | 70.0 |

| Press Coat Component (mg/ tablet) | |
|---|---|
| Atomoxetine hydrochloride | 9.1 |
| Mannitol granules (Pearlitol SD 200) | 234.4 |
| METHOCEL K100M | 82.5 |
| CAB-O-SIL (silica) | 1.0 |
| Magnesium Stearate | 3.0 |
| Total Press-coat | 330.0 |

### EXAMPLE 9. TASTE-MASKED ATOMOXETINE HYDROCHLORIDE SOLID DOSAGE FORM

A slurry is produced with the following composition: Atomoxetine hydrochloride (45.7 grams), EUDRAGIT (50 grams), ethanol (500 ml), and sodium lauryl sulfate (2 grams). The slurry is spray dried at a gas inlet temperature of 101°C to produce a free flowing fine powder which has satisfactory sustained-release properties and adequate taste masking of the atomoxetine hydrochloride.

### EXAMPLE 10. DELAYED-RELEASE FORM OF ATOMOXETINE HYDROCHLORIDE

### Core:

### Ingredients Amount (mg)

| Ingredients | Amount (mg) |
|---|---|
| Atomoxetine hydrochloride | 45.7 |
| Kollidon 90F (povidone USP) | 3.00 |
| Purified Water | 57.00 |
| Stearic Acid | 1.1 |
| Total (dry weight) | 49.8 |

Povidone is first dissolved in water. Atomoxetine hydrochloride is placed in the top spraying chamber of a Glatt GPCG1 fluidized bed apparatus. The solution of povidone is sprayed onto the active ingredient, using the following conditions:

| | |
|---|---|
| Air flow (m³/h) | 100-110 m³/hour |
| Liquid flow (g/min) | 6-7 g/minute |
| Inlet temperature | 65°C |
| Spraying pressure | 2.8 bar |

Once the granulation is complete, the granules are passed through a sieve (1 mm mesh). The stearic acid is weighed, added and blended in a V-blender. The resulting mixture is pressed into tablets (9/32 inch diameter and 9/32 inch curvature) with average hardness being between about 6.0 and about 120 kP.

The tablet cores are then coated with the following formulation.

| Ingredients | Amount (mg) |
|---|---|
| Tablet cores | 49.8 |
| ETHOCEL PR100 (ethylcellulose) | 2.35 |
| Kollidon 90F (povidone USP) | 2.35 |
| PEG 1450 | 0.70 |
| Denatured alcohol | 70.00 |
| Total (dry weight) | 55.20 |

Ethocel, povidone, and PEG 1450 are first dissolved in denatured alcohol. The coating solution is then sprayed onto the tablet cores in a coating pan (Vector LCDS), with the following spraying parameters:

| | |
|---|---|
| Air flow (m³/h) | 100-110 m³/h |
| Liquid flow (g/min) | 6-7 g/min |
| Inlet temperature | 65 °C. |
| Spraying pressure | 2.8 bar |

### EXAMPLE 11. ATOMOXETINE HYDROCHLORIDE DELAYED-RELEASE DOSAGE FORM WITH MODIFIED COATING

Tablet cores are prepared as in Example 11. The coating is modified by the addition of 0.20 mg of a red iron oxide pigment, to permit identification of the tablets. The total tablet weight is 55.4 mg. The coating and coating process are as in Example 11.

### EXAMPLE 12. COMBINED IMMEDIATE-RELEASE AND CONTROLLED-RELEASE ATOMOXETINE HYDROCHLORIDE TABLETS

In this formulation, one part of atomoxetine hydrochloride mixed with a binder is sprayed onto the coated tablet of Example 11. This produces immediate-release of atomoxetine from the coating, while maintaining controlled-release of atomoxetine from the core.

### Core:

| Ingredients | Amount (mg) |
|---|---|
| Atomoxetine Hydrochloride | 45.7 |
| Kollidon 90F (povidone USP) | 3.00 |
| Purified Water | 53.00 |
| Stearic Acid | 1.10 |
| Total (dry weight) | 49.80 |

The core is prepared by the procedure given in Example 11. These tablet cores are then coated with the following formulation.

### First Coating:

| Ingredients | Amount (mg) |
|---|---|
| Ethocel PR100 (ethylcellulose) | 2.35 |
| Kollidon 90F (povidone USP) | 2.35 |
| PEG 1450 | 0.70 |
| Denatured alcohol | 70.00 |

The first coating is applied by the spraying process given in Example 11. The coated tablet core is then sprayed with a second coating containing the remaining atomoxetine.

### Second Coating

### Ingredients Amount (mg)

| | |
|---|---|
| Coated Tablet Core | 55.20 |
| Atomoxetine Hydrochloride | 3.00 |
| ETHOCEL (ethylcellulose) | 1.66 |
| Denatured alcohol | 30.00 |
| Total (dry weight) | 59.86 |

The second coating is applied by the process used to apply the first coating.

The dissolution profile of this tablet is a combination of two profiles, the first one is an immediate-release profile and the second a controlled-release profile.

### EXAMPLE 13. SOLVENT FREE DELAYED-RELEASE ATOMOXETINE HYDROCHLORIDE FORMULATION

### Core:

| Ingredients | Amount (mg) |
|---|---|
| Atomoxetine Hydrochloride | 45.7 |
| Stearic Acid | 1.66 |
| AVICEL (Microcrystalline cellulose) | 6.66 |
| No solvent required | |
| Total (dry weight) | 54.02 |

Atomoxetine hydrochloride and stearic acid are placed in the chamber of a GLATT GPCG1 fluidized bed apparatus. The powders are fluidized with hot air. The powders are heated until the product temperature reaches 50-55 °C; at which point granulation occurs. The product is then cooled to room temperature. Avicel is sprayed onto the granules using the following parameters:

| | |
|---|---|
| Air flow (m³/h) | 100-110 m³/h |
| Inlet temperature | 60-65 °C |

Once the granulation is complete, the granules are passed through a sieve (1 mm) and microcrystalline cellulose is weighted, added and blended in a V-blender. The resulting mixture is pressed into tablets (9/32 inch diameter and 9/32 inch curvature) with average hardness being between about 5.0 and about 12.0 kP (kilopond). These tablet cores are then coated with the following formulation.

### Coating:

### Ingredients Amount (mg)

| | |
|---|---|
| Tablet cores | 54.02 |
| ETHOCEL (ethylcellulose) | 1.66 |
| KOLLIDON 90F (povidone USP) | 1.66 |
| PEG 1450 | .50 |
| Denatured alcohol | 70.00 |
| Total (dry weight) | 57.84 |

The coating process is as given in Example 11.

Alternatively the core is coated with the following coating; the same coating procedure is used.

### Example 13a. Alternate Coating

### Ingredients Amount (mg)

| | |
|---|---|
| Tablet cores | 54.02 |
| ETHOCEL (ethylcellulose) | 2.66 |
| KOLLIDON 90F (povidone USP) | 1.00 |
| PEG 1450 | 1.66 |
| Denatured alcohol | 100.00 |
| Total (dry weight) | 59.34 |

### EXAMPLE 14. DELAYED-RELEASE ATOMOXETINE HYDROCHLORIDE FORMULATION, ALTERNATE CORE FORMULATION

One of the two coatings set forth in Example 14 is applied to the following core formulation.

### Core:

### Ingredients Amount (mg)

| | |
|---|---|
| Atomoxetine Hydrochloride | 45.70 |
| Glyceryl behenate | 3.00 |
| Avicel (microcrystalline Cellulose) No solvent required | 6.00 |
| Total (dry weight) | 54.70 |

The core manufacturing process is identical to the one provided in Example 14, except that the powder mixture is heated to 65 °C.

### EXAMPLE 15. DELAYED-RELEASE ATOMOXETINE HYDROCHLORIDE FORMULATION

### Core

### Ingredients Amount (mg)

| | |
|---|---|
| Atomoxetine Hydrochloride | 45.70 |
| Polyethylene Glycol 8000 | 6.75 |
| Mineral oil | 0.9 |
| Purified Water | 36.00 |
| Total (dry weight) | 53.4 |

Polyethylene glycol 8000 is first dissolved in water. Mineral oil is then suspended in the PEG solution. Atomoxetine hydrochloride is placed in the top spraying chamber of Glatt GPCG1 fluidized bed apparatus. The solution of PEG and mineral oil is sprayed onto the active ingredient, with the following parameters:

| | |
|---|---|
| Air flow (m³/h) | 100-110 m³/h |
| Liquid flow (g/min) | 6-7 g/min |
| Inlet temperature | 65°C |
| Spraying pressure | 2.2 bar |

Once granulation is complete, the granules are passed through a sieve (1 mm mesh) and pressed into tablets (7 mm diameter and 7 mm curvature) with average hardness of about 5.0 to about 12.0 kP. These tablet cores are then coated with the following formulation.

### Coating:

| Ingredients | Amount (mg) |
|---|---|
| Tablet cores | 53.4 |
| ETHOCEL (ethylcellulose) | 1.50 |
| KOLLIDON 90F (povidone USP) | 1.50 |
| PEG 1450 | 0.45 |
| Denatured alcohol | 63.00 |
| Total (dry weight) | 56.85 |

The coating process is as provided in Example 11.

Alternatively, the Core formulation of this example can be coated with the following coating formulation.

### Coating

### Ingredients Amount (mg)

| | |
|---|---|
| Tablet cores | 53.4 |
| ETHOCEL (ethylcellulose) | 2.40 |
| KOLLIDON 90F (povidone USP) | 0.90 |
| PEG 1450 | 0.60 |
| Denatured alcohol | 90.0 |
| Total (dry weight) | 57.30 |

### EXAMPLE 16. DELAYED-RELEASE ATOMOXETINE HYDROCHLORIDE FORMULATION

### Core:

### Ingredients Amount (mg)

| | |
|---|---|
| Atomoxetine Hydrochloride | 45.7 |
| PVA (Polyvinyl Acetate USP) | 1.60 |
| Purified Water | 33.00 |
| Glyceryl behenate | 1.40 |
| Total (dry weight) | 48.70 |

The PVA is first dissolved in water. Atomoxetine hydrochloride is placed in the top spraying chamber of a GLATT GPCG1 fluidized bed apparatus. The solution of PVA is sprayed onto the active ingredient, with the following parameters:

| | |
|---|---|
| Air flow (m³/h) | 100-110 m³/h |
| Liquid flow (g/min) | 6-7 g/min |
| Inlet temperature | 65°C |
| Spraying pressure | 2.8 bar |

Once the granulation is completed, granules are passed through a sieve (1 mm mesh) and glyceryl behenate is weighed, added and blended in a V-blender. The resulting mixture is pressed into tablets (9/32 inch diameter and 9/32 inch curvature) with average hardness being between about 6.0 and 12.0 kP. These tablet cores are then coated with the following formulation.

### First coating

| Ingredients | Amount (mg) |
|---|---|
| Tablet cores | 48.70 |
| Ethocel PR100 (ethylcellulose) | 2.10 |
| Kollidon 90F (povidone USP) | 0.90 |
| PEG 1450 | 0.45 |
| Denatured alcohol | 66.00 |
| Total (dry weight) | 52.15 |

ETHOCEL, povidone, and PEG 1450 are first dissolved in denatured alcohol. The coating solution is then sprayed onto the tablet cores in a coating pan (Vector LCDS), with the following spraying parameters:

| | |
|---|---|
| Air flow (m³/h) | 100-110 m³/h |
| Liquid flow (g/min) | 6-7 g/min |
| Inlet temperature | 65°C |
| Spraying pressure | 2.8 bar |

### Second coating

### Ingredients Amount (mg)

| | |
|---|---|
| Coated tablets | 52.15 |
| EUDRAGIT L30 D-55 | 2.10 |
| Silicon dioxide | 0.63 |
| PEG 1450 | 0.42 |
| Triethyl citrate | 0.21 |
| Water | 12.00 |
| Total (dry weight) | 54.88 |

PEG and triethyl citrate 1450 are first dissolved in half the quantity of water. EUDRAGIT is then added and the solution is stirred for 45 minutes. Silicon dioxide is suspended in the remaining quantity of water and homogenized. The silicon dioxide suspension is then added to the EUDRAGIT dispersion. The tablets are coated in a coating pan (VECTOR LCDS), with the following spraying parameters:

| | |
|---|---|
| Air flow (m³/h) | 100-110 m³/h |
| Liquid flow (g/min) | 6-7 g/min |
| Inlet temperature | 55°C |
| Spraying pressure | 2.8 bar |

### EXAMPLE 17. POLYVINYLPYROLIDONE (PVP) 29/32K: ATOMOXETINE, 2:1 WEIGHT BASIS, OVEN DRYING (AMORPHOUS ATOMOXETINE FORMULATION)

Atomoxetine hydrochloride (4.62 g) and hot purified water (60°C, 48 mL) is added to a 125 mL Erlenmeyer flask is added PVP 29/32K (8.1210 g). The Erlenmeyer flask is immersed in water bath at 60 °C. Hot 1.0 N HCl (60°C., 13.6 mL) is added to the 125 mL Erlenmeyer flask and stirred for approximately 5 minutes. Approximately 5 mL of the hot solution is transferred using a pipette to a pre-heated crystallization dish (60 °C) and dried in a tray oven at 60 °C for 71 hours to yield a solid product containing amorphous atomoxetine hydrochloride.

### EXAMPLE 18. PVP 29/32K/ATOMOXETINE HYDROCHLORIDE, 2:1 WEIGHT BASIS, VACUUM DRYING

Approximately 5 mL of the hot solution prepared in Example 18 is transferred using a pipette to a pre-heated 50 mL round bottom flask (60°C). The sample is dried under static vacuum at 60°C for 29 hours to yield a solid product containing amorphous atomoxetine hydrochloride.

### EXAMPLE 19. PVP 29/32K/ ATOMOXETINE HYDROCHLORIDE, 1:1 WEIGHT BASIS, VACUUM DRYING

PVP having a molecular weight distribution corresponding to 29/32K (22.24 g), atomoxetine hydrochloride (22.21 g) and purified water (278 g) is added to a 250 mL flask (equipped with a magnetic stir bar). The contents of the flask are stirred and heated to a temperature of approximately 60 °C with a stirring hotplate to obtain a clear solution. The hot solution is spray dried onto dibasic calcium phosphate dihydrate (187.344 g) using a bench top fluid bed dryer.

## Claims

1. A press-coat dosage form comprising
a core composition comprising an active agent, which active agent is atomoxetine or a pharmaceutically acceptable salt thereof, a waxy material; and
a coating composition comprising a hydrophilic polymer, wherein the coating composition is press-coated onto the core composition.

2. The press-coat dosage form of Claim 1, wherein the active agent is atomoxetine hydrochloride.

3. The press-coat dosage form of Claim 2, wherein the coating composition further comprises the active agent.

4. The press-coat dosage form of Claim 3, wherein the active agent comprising the coating composition is atomoxetine hydrochloride.

5. The press-coat dosage form of Claim 3, wherein the ratio of the active agent in the core composition to the active agent in the coating composition is about 1:99 to about 99:1.

6. The press-coat dosage form of Claim 3, wherein the ratio of the active agent in the core composition to the active agent in the coating composition greater than about 5:3.

7. The press-coat dosage form of any one of Claims 1 to 6, wherein the waxy material is carnauba wax, tribehenin, fatty alcohols, lauryl alcohol, myristyl alcohol, stearyl alcohol, palmityl alcohol, fatty acids, lauric acid, myristic acid, stearic acid, palmitic acid, polyethylenes, castor wax, C₁₆₋₃₀ fatty acid triglycerides, beeswax, or a mixture of any combination thereof.

8. The press-coat dosage form of any one of Claims 1 to 7, wherein the hydrophilic polymer comprises a hydrophilic cellulose polymer.

9. The press-coat dosage form of Claim 8, comprising:
a core composition comprising an active agent which is atomoxetine hydrochloride and wherein the hydrophilic cellulose polymer is hydroxypropylmethyl cellulose (HPMC).

10. The press-coat dosage form of Claim 1, comprising:
the core composition comprising an active agent which is atomoxetine or atomoxetine hydrochloride,
carnauba wax; and
the coating composition comprising the active agent and hydroxypropylmethyl cellulose,
wherein the coating composition is press-coated onto the core.

11. The press-coat dosage form of Claim 10, comprising:
the core composition comprising atomoxetine hydrochloride and carnauba wax,
the coating composition comprising atomoxetine hydrochloride and hydroxypropylmethyl cellulose, wherein the coating composition is press-coated onto the core, and
comprising an additional coating composition comprising atomoxetine hydrochloride.

12. The press-coat dosage form of Claim 11, wherein the additional coating composition is an immediate-release coating composition.

13. The press-coat dosage form of any one of Claims 10 to 12, wherein the active agent in the core composition and the active agent in the coating composition are present in an amount to provide a substantially zero order release profile.

14. The press-coat dosage form of any one of Claims 10 to 12, wherein the active agent in the core composition and the active agent in the coating composition are present in an amount to provide a substantially first order release profile.

15. The press-coat dosage form of any one of Claims 10 to 12, wherein the active agent in the core composition and the active agent in the coating composition are present in an amount to provide a substantially second order release profile.

16. The press-coat dosage form of any one of Claims 1 to 15, wherein the press-coat dosage form is a tablet.

17. A method for preparing a press-coat dosage form, the method comprising
providing a core composition comprising atomoxetine or a pharmaceutically acceptable salt thereof and a waxy material,
providing a coating composition comprising atomoxetine or a pharmaceutically acceptable salt thereof and a hydrophilic polymer, and
press-coating the coating composition onto the core composition to provide the press-coat dosage form.

## Patentansprüche

1. Eine Pressschicht-Dosisform, welche umfasst:
eine Kernzusammensetzung, die ein aktives Mittel, wobei das aktive Mittel Atomoxetin oder ein pharmazeutisch verträgliches Salz von diesem ist, [und] ein wachsartiges Material umfasst; und
eine Beschichtungszusammensetzung, die ein hydrophiles Polymer umfasst, wobei die Beschichtungszusammensetzung auf die Kernzusammensetzung pressbeschichtet wird.

2. Die Pressschicht-Dosisform nach Anspruch 1, wobei das aktive Mittel Atomoxetinhydrochlorid ist.

3. Die Pressschicht-Dosisform nach Anspruch 2, wobei die Beschichtungszusammensetzung weiterhin das aktive Mittel umfasst.

4. Die Pressschicht-Dosisform nach Anspruch 3, wobei das aktive Mittel, welches die Beschichtungszusammensetzung umfasst, Atomoxetinhydrochlorid ist.

5. Die Pressschicht-Dosisform nach Anspruch 3, wobei das Verhältnis des aktiven Mittels in der Kernzusammensetzung zu dem aktiven Mittel in der Beschichtungszusammensetzung ca. 1:99 bis ca. 99:1 beträgt.

6. Die Pressschicht-Dosisform nach Anspruch 3, wobei das Verhältnis des aktiven Mittels in der Kernzusammensetzung zu dem aktiven Mittel in der Beschichtungszusammensetzung größer als ca. 5:3 ist.

7. Die Pressschicht-Dosisform nach irgendeinem der Ansprüche 1 bis 6, wobei das wachsartige Material Carnaubawachs, Tribehenin, ein Fettalkohol, Laurylalkohol, Myristylalkohol, Stearylalkohol, Palmitylalkohol, eine Fettsäure, Laurinsäure, Myristinsäure, Stearinsäure, Palmitinsäure, ein Polyethylen, Castorwachs, ein C₁₆₋₃₀-Fettsäuretriglycerid, Bienenwachs oder eine Mischung von irgendeiner Kombination von diesen ist.

8. Die Pressschicht-Dosisform nach irgendeinem der Ansprüche 1 bis 7, wobei das hydrophile Polymer ein hydrophiles Cellulosepolymer umfasst.

9. Die Pressschicht-Dosisform nach Anspruch 8, welche umfasst:
eine Kernzusammensetzung, die ein aktives Mittel umfasst, welches Atomoxetinhydrochlorid ist, und wobei das hydrophile Cellulosepolymer Hydroxypropylmethylcellulose (HPMC) ist.

10. Die Pressschicht-Dosisform nach Anspruch 1, welche umfasst:
die Kernzusammensetzung, die ein aktives Mittel umfasst, welches Atomoxetin oder Atomoxetinhydrochlorid ist,
Carnaubawachs; und
die Beschichtungszusammensetzung, die das aktive Mittel und Hydroxypropylmethylcellulose umfasst,
wobei die Beschichtungszusammensetzung auf den Kern pressbeschichtet wird.

11. Die Pressschicht-Dosisform nach Anspruch 10, welche umfasst:
die Kernzusammensetzung, die Atomoxetinhydrochlorid und Carnaubawachs umfasst,
die Beschichtungszusammensetzung, die Atomoxetinhydrochlorid und Hydroxypropylmethylcellulose umfasst, wobei die Beschichtungszusammensetzung auf den Kern pressbeschichtet wird, und
welche eine zusätzliche Beschichtungszusammensetzung umfasst, die Atomoxetinhydrochlorid umfasst.

12. Die Pressschicht-Dosisform nach Anspruch 11, wobei die zusätzliche Beschichtungszusammensetzung eine sofort freisetzende Beschichtungszusammensetzung ist.

13. Die Pressschicht-Dosisform nach irgendeinem der Ansprüche 10 bis 12, wobei das aktive Mittel in der Kernzusammensetzung und das aktive Mittel in der Beschichtungszusammensetzung in einer Menge vorliegen, um ein Profil einer Freisetzung von im Wesentlichen nullter Ordnung bereit zu stellen.

14. Die Pressschicht-Dosisform nach irgendeinem der Ansprüche 10 bis 12, wobei das aktive Mittel in der Kernzusammensetzung und das aktive Mittel in der Beschichtungszusammensetzung in einer Menge vorliegen, um ein Profil einer Freisetzung von im Wesentlichen erster Ordnung bereit zu stellen.

15. Die Pressschicht-Dosisform nach irgendeinem der Ansprüche 10 bis 12, wobei das aktive Mittel in der Kernzusammensetzung und das aktive Mittel in der Beschichtungszusammensetzung in einer Menge vorliegen, um ein Profil einer Freisetzung von im Wesentlichen zweiter Ordnung bereit zu stellen.

16. Die Pressschicht-Dosisform nach irgendeinem der Ansprüche 1 bis 15, wobei die Pressschicht-Dosisform eine Tablette ist.

17. Ein Verfahren zur Herstellung einer Pressschicht-Dosisform, wobei das Verfahren umfasst:
das Bereitstellen einer Kernzusammensetzung, die Atomoxetin oder ein pharmazeutische verträgliches Salz von diesem und ein wachsartiges Material umfasst,
das Bereitstellen einer Beschichtungszusammensetzung, die Atomoxetin oder ein pharmazeutisch verträgliches Salz von diesem und ein hydrophiles Polymer umfasst, und
das Pressbeschichten der Beschichtungszusammensetzung auf die Kernzusammensetzung, um die Pressschicht-Dosisform bereit zu stellen.

## Revendications

1. Forme pharmaceutique revêtue par compression comprenant :
une composition de noyau contenant un agent actif, lequel agent actif est l'atomoxétine ou un sel pharmaceutiquement acceptable de celle-ci, un matériau cireux ; et
une composition d'enrobage contenant un polymère hydrophile, la composition d'enrobage étant revêtue par compression sur la composition de noyau.

2. Forme pharmaceutique revêtue par compression selon la revendication 1, dans laquelle l'agent actif est le chlorhydrate d'atomoxétine;

3. Forme pharmaceutique revêtue par compression selon la revendication 2, dans laquelle la composition d'enrobage contient en outre l'agent actif.

4. Forme pharmaceutique revêtue par compression selon la revendication 3, dans laquelle l'agent actif comprenant la composition d'enrobage est le chlorhydrate d'atomoxétine.

5. Forme pharmaceutique revêtue par compression selon la revendication 3, dans laquelle le rapport de l'agent actif dans la composition de noyau par rapport à l'agent actif dans la composition d'enrobage est d'environ 1 : 99 à environ 99:1.

6. Forme pharmaceutique revêtue par compression selon la revendication 3, dans laquelle le rapport de l'agent actif dans la composition de noyau par rapport à l'agent actif dans la composition d'enrobage est supérieur à environ 5:3.

7. Forme pharmaceutique revêtue par compression selon l'une quelconque des revendications 1 à 6, dans laquelle le matériau cireux est de la cire de carnauba, de la tribéhénine, des alcools gras, de l'alcool laurylique, de l'alcool myristylique, de l'alcool stéarylique, de l'alcool palmitique, des acides gras, de l'acide laurique, de l'acide myristique, de l'acide stéarique, de l'acide palmitique, des polyéthylènes, de la cire de ricin, des triglycérides d'acides gras, de la cire d'abeille, ou un mélange d'une combinaison quelconque de ces derniers.

8. Forme pharmaceutique revêtue par compression selon l'une quelconque des revendications 1 à 7, dans laquelle le polymère hydrophile comprend un polymère de cellulose hydrophile.

9. Forme pharmaceutique revêtue par compression selon la revendication 8, comprenant :
une composition de noyau contenant un agent actif qui est le chlorhydrate d'atomoxétine et où polymère de cellulose hydrophile est l'hydroxypropylméthylecellulose (HPMC).

10. Forme pharmaceutique revêtue par compression selon la revendication 1, comprenant :
la composition de noyau contenant un agent actif qui est l'atomoxétine ou le chlorhydrate d'atomoxétine,
de la cire de carnauba ; et
la composition d'enrobage contenant l'agent actif et de l'hydroxypropylméthylecellulose,
où la composition d'enrobage est revêtue par compression sur le noyau.

11. Forme pharmaceutique revêtue par compression selon la revendication 10, comprenant :
la composition de noyau contenant du chlorhydrate d'atomoxétine et de la cire de carnauba,
la composition d'enrobage contenant du chlorhydrate d'atomoxétine et de l'hydroxypropylméthylecellulose, où la composition d'enrobage est revêtue par compression sur le noyau, et
comprenant une composition d'enrobage supplémentaire contenant du chlorhydrate d'atomoxétine.

12. Forme pharmaceutique revêtue par compression selon la revendication 11, dans laquelle la composition d'enrobage supplémentaire est une composition d'enrobage à libération immédiate.

13. Forme pharmaceutique revêtue par compression selon l'une quelconque des revendications 10 à 12, dans laquelle l'agent actif dans la composition de noyau et l'agent actif dans la composition d'enrobage sont présents dans une quantité permettant de fournir un profil de libération sensiblement d'ordre zéro.

14. Forme pharmaceutique revêtue par compression selon l'une quelconque des revendications 10 à 12, dans laquelle l'agent actif dans la composition de noyau et l'agent actif dans la composition d'enrobage sont présents dans une quantité permettant de fournir un profil de libération sensiblement du premier ordre.

15. Forme pharmaceutique revêtue par compression selon l'une quelconque des revendications 10 à 12, dans laquelle l'agent actif dans la composition de noyau et l'agent actif dans la composition d'enrobage sont présents dans une quantité permettant de fournir un profil de libération sensiblement du deuxième ordre.

16. Forme pharmaceutique revêtue par compression selon l'une quelconque des revendications 1 à 15, dans laquelle la forme pharmaceutique revêtue par compression est un comprimé.

17. Procédé de préparation d'une forme pharmaceutique revêtue par compression, le procédé consistant à :
fournir une composition de noyau contenant de l'atomoxétine ou un sel pharmaceutiquement acceptable de celle-ci et un matériau cireux,
fournir une composition d'enrobage contenant de l'atomoxétine ou un sel pharmaceutiquement acceptable de celle-ci et un polymère hydrophile, et
revêtir par compression la composition d'enrobage sur la composition de noyau afin de fournir la forme pharmaceutique revêtue par compression.
